# EUROPEAN PATENT SPECIFICATION

(11) **EP 4 308 096 B1**
(45) Date of publication and mention of the grant of the patent: **13.05.2026**
(21) Application number: 22771978.8
(22) Date of filing: 14.03.2022
(51) Int. Cl.: C07D 405/14, C07D 413/14, C07D 491/048, C07D 491/107, C07D 498/10, C07D 401/14, A61P 25/00, A61P 25/16

(54) **HETEROAROYL AMIDES AS LRRK2 INHIBITORS, PHARMACEUTICAL COMPOSITIONS, AND USES THEREOF**
HETEROAROYLAMIDE ALS LRRK2-INHIBITOREN, PHARMAZEUTISCHE ZUSAMMENSETZUNGEN UND VERWENDUNGEN DAVON
AMIDES D'HÉTÉROAROYLE EN TANT QU'INHIBITEURS DE LRRK2, COMPOSITIONS PHARMACEUTIQUES ET UTILISATIONS ASSOCIÉES

(30) Priority: 17.03.2021 US 202163162297 P
(43) Date of publication of application: 24.01.2024
(73) Proprietor: Merck Sharp & Dohme LLC, Rahway, New Jersey 07065 (US)
(72) Inventor: CHILDERS, Kaleen Konrad, Medford, Massachusetts 02052 (US); ELLIS, J. Michael, Milton, Massachusetts 02186 (US); FULLER, Peter, H., Boston, Massachusetts 02115-5727 (US); GULATI, Anmol, Boston, Massachusetts 02115-5727 (US); GUNAYDIN, Hakan, Belmont, Massachusetts 02478 (US); KATTAR, Solomon, D., Boston, Massachusetts 02115-5727 (US); KURUKULASURIYA, Ravi, Jamaica Plain, Massachusetts 02130 (US); METHOT, Joey, L., Boston, Massachusetts 02115-5727 (US); MORRIELLO, Gregori, J., Kenilworth, New Jersey 07033 (US); NEELAMKAVIL, Santhosh, Edison, New Jersey 08820 (US); LAPOINTE, Blair, T., Boston, Massachusetts 02115-5727 (US); SIMOV, Vladimir, Boston, Massachusetts 02115-5727 (US); YEUNG, Charles, S., Boston, Massachusetts 02115-5727 (US)
(74) Representative: Merck Sharp & Dohme LLC
(86) International application number: PCT/US2022/020108
(87) International publication number: WO 2022/197575

(56) References cited:
- WO-A1-2016/036586
- WO-A2-2020/247298
- US-A1- 2019 276 426
- DATABASE PubChem substance ANONYMOUS : "SID 244092243 ", XP055971865, retrieved from NCBI Database accession no. 244092243

## Description

### BACKGROUND OF THE INVENTION

Parkinson's disease (PD) is a common neurodegenerative disease caused by progressive loss of mid-brain dopaminergic neurons leading to abnormal motor symptoms such as bradykinesia, rigidity and resting tremor. Many PD patients also experience a variety of non-motor symptoms including cognitive dysfunction, autonomic dysfunction, emotional changes and sleep disruption. The combined motor and non-motor symptoms of Parkinson's disease severely impact patient quality of life.

While the majority of PD cases are idiopathic, there are several genetic determinants such as mutations in SNCA, Parkin, PINK1, DJ-1 and LRRK2. Linkage analysis studies have demonstrated that multiple missense mutations in the Leucine-Rich Repeat Kinase 2 (LRRK2) gene lead to an autosomal late onset form of PD. LRRK2 is a 286 kDa cytoplasmic protein containing kinase and GTPase domains as well as multiple protein-protein interaction domains. See for example, Aasly et al., Annals of Neurology, Vol. 57(5), May 2005, pp. 762-765; Adams et al., Brain, Vol. 128, 2005, pp. 2777-85; Gilks et al., Lancet, Vol. 365, Jan. 29, 2005, pp. 415-416, Nichols et al., Lancet, Vol. 365, Jan. 29, 2005, pp. 410-412, and U. Kumari and E. Tan, FEBS journal 276 (2009) pp. 6455-6463.

*In vitro* biochemical studies have demonstrated that LRRK2 proteins harboring the PD associated proteins generally confer increased kinase activity and decreased GTP hydrolysis compared to the wild type protein (Guo et al., Experimental Cell Research, Vol, 313, 2007, pp. 3658-3670) thereby suggesting that small molecule LRRK2 kinase inhibitors may be able to block aberrant LRRK2-dependent signaling in PD. In support of this notion, it has been reported that inhibitors of LRRK2 are protective in models of PD (Lee et al., Nature Medicine, Vol 16, 2010, pp. 998-1000).

LRRK2 expression is highest in the same brain regions that are affected by PD. LRRK2 is found in Lewy bodies, a pathological hallmark of PD as well as other neurodegenerative diseases such as Lewy body dementia (Zhu et al., Molecular Neurodegeneration, Vol 30, 2006, pp. 1-17).

Further, LRRK2 mRNA levels are increased in the striatum of MPTP-treated marmosets, an experimental model of Parkinson's disease, and the level of increased mRNA correlates with the level of L-Dopa induced dyskinesia suggesting that inhibition of LRRK2 kinase activity may have utility in ameliorating L-Dopa induced dyskinesias. These and other recent studies indicate that a potent, selective and brain penetrant LRRK2 kinase inhibitor could be a therapeutic treatment for PD. (Lee et al., Nat. Med. 2010 Sep;16(9):998-1000; Zhu, et al., Mol. Neurodegeneration 2006 Nov 30;1:17; Daher, et al., J Biol Chem. 2015 Aug 7; 290(32):19433-44; Volpicelli-Daley et al., J Neurosci. 2016 Jul 13; 36(28):7415-27).

LRRK2 mutations have been associated with Alzheimer's-like pathology (Zimprach et al., Neuron. 2004 Nov 18;44(4):601-7) and the LRRK2 R1628P variant has been associated with an increased risk of developing AD (Zhao et al., Neurobiol Aging. 2011 Nov; 32(11):1990-3). Mutations in LRRK2 have also been identified that are clinically associated with the transition from mild cognitive impairment to Alzheimer's disease (see WO2007149798). Together these data suggest that LRRK2 inhibitors may be useful in the treatment of Alzheimer's disease and other dementias and related neurodegenerative disorders.

LRRK2 has been reported to phosphorylate tubulin-associated tau and this phosphorylation is enhanced by the kinase activating LRRK2 mutation G2019S (Kawakami et al., PLoS One. 2012; 7(1):e30834; Bailey et al., Acta Neuropathol. 2013 Dec; 126(6):809-27.). Additionally, over expression of LRRK2 in a tau transgenic mouse model resulted in the aggregation of insoluble tau and its phosphorylation at multiple epitopes (Bailey et al., 2013). Hyperphosphorylation of tau has also been observed in LRRK2 R1441G overexpressing transgenic mice (Li et al., Nat Neurosci. 2009 Jul; 12(7):826-8.). Inhibition of LRRK2 kinase activity may therefore be useful in the treatment of tauopathy disorders characterized by hyperphosphorylated of tau such as argyrophilic grain disease, Picks disease, corticobasal degeneration, progressive supranuclear palsy, inherited frontotemporal dementia and parkinson's linked to chromosome 17 (Goedert and Jakes Biochim Biophys Acta. 2005 Jan 3.).

A growing body of evidence suggests a role for LRRK2 in immune cell function in the brain with LRRK2 inhibitors demonstrated to attenuate microglial inflammatory responses (Moehle et al., J Neurosci. 2012 Feb 1;32(5):1602-11.). As neuroinflammation is a hallmark of a number of neurodegenerative diseases such PD, AD, MS, HIV-induced dementia, ALS, ischemic stroke, MS, traumatic brain injury and spinal cord injury, LRRK2 kinases inhibitors may have utility in the treatment of neuroinflammation in these disorders. Significantly elevated levels of LRRK2 mRNA have been observed in muscle biopsy samples taken from patients with ALS (Shtilbans et al., Amyotroph Lateral Scler. 2011 Jul;12(4):250-6.). LRRK2 inhibitors have been disclosed in the art, e.g., WO2016036586, US2019276426, WO2020247298 and US20150031672.
LRRK2 is also expressed in cells of the immune system and recent reports suggest that LRRK2 may play a role in the regulation of the immune system and modulation of inflammatory responses. LRRK2 kinase inhibitors may therefore be of utility in a number of diseases of the immune system such as lymphomas, leukemias, multiple sclerosis rheumatoid arthritis, systemic lupus erythematosus autoimmune hemolytic anemia, pure red cell aplasia, idiopathic thrombocytopenic pupura (ITP), Evans Syndrome, vasculitis, bullous skin disorder, type I diabetes mellitus, Sjorgen's syndrome, Delvic's disease, inflammatory myopathies (Engel at al., Pharmacol Rev. 2011 Mar;63(1):127-56; Homam et al., Homam et al., Clin Neuromuscluar disease, 2010) and ankylosing spondylitis (Danoy et al., PLoS Genet. 2010 Dec 2; 6(12).).Increased incidence of certain types of non-skin cancers such as renal, breast, lung, prostate, and acute myelogenous leukemia (AML) have been reported in patients with the LRRK2 G2019S mutation (Agalliu et al., JAMA Neurol. 2015 Jan;72(1); Saunders-Pullman et al., Mov Disord. 2010 Nov 15;25(15):2536-41.). LRRK2 has amplification and overexpression has been reported in papillary renal and thyroid carcinomas. Inhibiting LRRK2 kinase activity may therefore be useful in the treatment of cancer (Looyenga et al., Proc Natl Acad Sci U S A. 2011 Jan 25;108(4):1439-44).

Genome-wide association studies also highlight LRRK2 in the modification of susceptibility to the chronic autoimmune Crohn's disease and leprosy (Zhang et al., The New England Jopuranl of Medicine, Vol 361, 2009, pp. 2609-2618; Umeno et al., Inflammatory Bowel Disease Vol 17, 2011, pp. 2407-2415).

### SUMMARY OF THE INVENTION

The present invention is directed to certain heteroaryl amide derivatives, which are collectively or individually referred to herein as "compound(s) of the invention" or "compounds of Formula (I)", as described herein. Applicant has found, surprisingly and advantageously, that the compounds of Formula (I), each of which possess four linked nitrogen containing cyclic groups, one of which is linked through an amide moiety, which exhibit excellent LRRK2 inhibitory activity. In some embodiments, the compounds of the invention exhibit unexpectedly superior potency as inhibitors of LRRK2 kinase, as evidenced by the data reported herein. The compounds of the invention may be useful in the treatment or prevention of diseases (or one or more symptoms associated with such diseases) in which the LRRK2 kinase is involved, including Parkinson's disease and other indications, diseases and disorders as described herein. The invention is also directed to pharmaceutical compositions comprising a compound of the invention and such compounds and compositions for use in methods for the treatments described herein.

### DETAILED DESCRIPTION OF THE INVENTION

For each of the following embodiments, any variable not explicitly defined in the embodiment is as defined in Formula (I). In each of the embodiments described herein, each variable is selected independently of the other unless otherwise noted.

Any references to methods of treatment in this description are to be interpreted as references to the compounds, pharmaceutical compositions and medicaments for use in a method of treatment of the human (or animal) body by therapy (or for diagnosis).

In one embodiment, the compounds of the invention have the structural Formula (I): 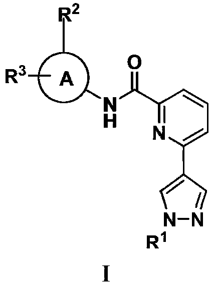 or a pharmaceutically acceptable salt thereof, wherein:
A is a nitrogen containing heteroaryl selected from pyridyl, pyrimidinyl and pyridazinyl;
R¹ is selected from hydrogen, C₁₋₆ alkyl, C₁₋₆ haloalkyl, and -(CH₂)ₙC₃₋₆ cycloalkyl, said alkyl and cycloalkyl optionally substituted with 1 to 3 groups selected from CF₃ and CN;
R² is an N-linked heterocycloalkyl selected from a group consisting of pyrrolidinyl, piperidinyl, piperazinyl, azetidinyl, morpholinyl, tetrahydrofuronyl, azabicylohexanyl, azabicyloheptanyl, azaspiroheptanyl, azaspirononanyl, diazaspirononanyl, and azaspirooctanyl, said pyrrolidinyl, piperidinyl, piperazinyl, azetidinyl, morpholinyl, tetrahydrofuronyl, azabicylohexanyl, azabicyloheptanyl, azaspiroheptanyl, azaspirononanyl, diazaspirononanyl, and azaspirooctanyl, unsubstituted or substituted with 1 to 4 groups independently selected from R⁴;
R³ is selected from hydrogen, CH₃, CF₃, and cyclopropyl;
each R⁴ is independently selected from the group consisting of C₁₋₆ alkyl, C₁₋₆ alkylOH, C₁₋₆ alkylCN, OC₁₋₆ alkyl, C₁₋₆ haloalkyl, C₃₋₆ cycloalkyl, oxetanyl, oxo, OH, halogen, said oxetanyl optionally substituted with 1 to 3 of CH₃ groups; and
n is selected from 0 to 3.

An embodiment of this invention is realized when A pyridyl.

Another embodiment of this invention is realized when A is pyrimidinyl.

Another embodiment of this invention is realized when A is pyridazinyl.

Another embodiment of this invention is realized when R¹ is hydrogen.

Another embodiment of this invention is realized when R¹ is C₁₋₆ alkyl said alkyl optionally substituted with 1 to 3 groups selected from CF₃ and CN. A subembodiment of this aspect of the invention is realized when the alkyl is selected from CH₃ and CH₂CH₃. Another subembodiment of this aspect of the invention is realized when the alkyl is CH₃. Yet another subembodiment of the invention is realized when the alkyl is CH₂CH₃.

Another embodiment of this invention is realized when R¹ is C₁₋₆ haloalkyl. A subembodiment of this aspect of the invention is realized when the haloalkyl is selected from CH₂CF₃, and CH(CH₃)CF₃. Another subembodiment of this aspect of the invention is realized when the haloalkyl is CH₂CF₃. Still another subembodiment of this aspect of the invention is realized when the haloalkyl is CH(CH₃)CF₃.

Another embodiment of this invention is realized when R¹ is -(CH₂)ₙC₃₋₆ cycloalkyl, said cycloalkyl optionally substituted with 1 to 3 groups selected from CF₃ and CN. A subembodiment of this aspect of the invention is realized when the cycloalkyl is selected from optional substituted -(CH₂)ₙcyclopropyl and -(CH₂)ₙcyclobutyl. Still another subembodiment of this aspect of the invention is realize when the cycloalkyl is optionally substituted - (CH₂)ₙcylopropyl. Yet another subembodiment of this aspect of the invention is realize when the optionally substituted cycloalkyl is -(CH₂)ₙcyclobutyl. Still another embodiment of this aspect of the invention is realized when the substituent is CN. Still another embodiment of this aspect of the invention is realized when the substituent is CF₃.

Still another embodiment of the invention is realized when R¹ is selected from the group consisting of hydrogen, CH₃, CH₂CH₃, CH₂CF₃, CH(CH₃)CF₃, -(CH₂)ₙcyclopropyl, and - (CH₂)ₙcyclobutyl, said group optionally substituted with 1 to 3 groups selected from CN and CF3.

Another embodiment of this invention is realized when R² is pyrrolidinyl, unsubstituted or substituted with 1 to 4 groups independently selected from R⁴. Another embodiment of this invention is realized when R² is piperidinyl, unsubstituted or substituted with 1 to 4 groups independently selected from R⁴. Another embodiment of this invention is realized when R² is piperazinyl, unsubstituted or substituted with 1 to 4 groups independently selected from R⁴. Another embodiment of this invention is realized when R² is azetidinyl, unsubstituted or substituted with 1 to 4 groups independently selected from R⁴. Another embodiment of this invention is realized when R² is morpholinyl, unsubstituted or substituted with 1 to 4 groups independently selected from R⁴. Another embodiment of this invention is realized when R² is tetrahydrofuronyl, unsubstituted or substituted with 1 to 4 groups independently selected from R⁴. Another embodiment of this invention is realized when R² is azabicyclohexanyl, unsubstituted or substituted with 1 to 4 groups independently selected from R⁴. Another embodiment of this invention is realized when R² is azabicycloheptanyl, unsubstituted or substituted with 1 to 4 groups independently selected from R⁴. Another embodiment of this invention is realized when R² is azaspiroheptanyl, unsubstituted or substituted with 1 to 4 groups independently selected from R⁴. Another embodiment of this invention is realized when R² is azaspirononanyl, unsubstituted or substituted with 1 to 4 groups independently selected from R⁴. Another embodiment of this invention is realized when R² is diazaspirononanyl, unsubstituted or substituted with 1 to 4 groups independently selected from R⁴. Another embodiment of this invention is realized when R² is azaspirooctanyl, unsubstituted or substituted with 1 to 4 groups independently selected from R⁴.

Another embodiment of the invention is realized when R³ is hydrogen. Another embodiment of the invention is realized when R³ is CH₃. Another embodiment of the invention is realized when R³ is CF₃. Another embodiment of the invention is realized when R³ is cyclopropyl.

Another embodiment of the invention is realized when each R⁴ is independently selected from the group consisting of hydrogen, CH₃, OCH₃, C(CH₃)₂OH, C(CH₃)₂CN, CF₃, CH₂F, CHF₂, C(CH3)₂F, cyclopropyl, cyclobutyl, oxetanyl, oxo, OH, and fluoro, said oxetanyl optionally substituted with 1 to 3 CH₃ groups. A subembodiment of this aspect of the invention is realized when R⁴ is C(CH₃)₂OH. Another subembodiment of this aspect of the invention is realized when each R⁴ is independently selected from CH₃ and OH. Still another subembodiment of this aspect of the invention is realized when R⁴ is fluorine. Still another subembodiment of this aspect of the invention is realized when each R⁴ is independently selected from optionally substituted oxetanyl and CH₃.

Another embodiment of this invention is represented by structural Formula II: 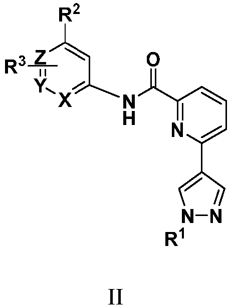 or a pharmaceutically acceptable salt thereof, wherein R¹, R², and R³ are as described herein, and X, Y, and Z are represented and selected from the group consisting of:
1) X=Z=N, and Y=CH or C-CH3;
2) Y=N, Z=C, and X=C-CH3, C-CF3, or C-cyclopropyl;
3) Z=N, Y=C, and X= C-CH3, C-CF3, or C-cyclopropyl;
4) Y=Z=N and X=C-CH3 or C-cyclopropyl;
5) X=Y=N= and Z=CH; and
6) Z=CH, Y=CH or C-CH3 and X=N.

A subembodiment of the invention of formula II is realized when X, Y, and Z combined to form pyridyl represented and selected from the group consisting of 2), 3), 5) and 6).

A subembodiment of the invention of formula II is realized when X, Y, and Z combined are pyridyl represented and selected from the group consisting of 2), 3), 5) and 6), R¹ is selected from the group consisting of CH₃, CH₂CH₃, CH₂CF₃, CH(CH₃)CF₃, -(CH₂)ₙcyclopropyl, and - (CH₂)ₙcyclobutyl, said CH₃, CH₂CH₃, CH₂CF₃, CH(CH₃)CF₃, -(CH₂)ₙcyclopropyl, and - (CH₂)ₙcyclobutyl optionally substituted with 1 to 3 groups selected from CN and CF₃; R² is selected from pyrrolidinyl, piperidinyl, piperazinyl, azetidinyl, morpholinyl and tetrahydrofuronyl, said pyrrolidinyl, piperidinyl, piperazinyl, azetidinyl, morpholinyl and tetrahydrofuronyl unsubstituted or substituted with 1 to 4 groups independently selected from R⁴; R³ is selected from the group consisting of hydrogen, CH₃, CF₃ and cyclopropyl; and each R⁴ is independently selected from the group consisting of hydrogen, CH₃, OCH₃, C(CH₃)₂OH, C(CH₃)₂CN, CF₃, CH₂F, C(CH3)₂F, cyclopropyl, oxetanyl, oxo, OH, and fluoro, said oxetanyl optionally substituted with 1 to 3 groups of CH₃. An aspect of the invention of formula II wherein X, Y, and Z combine to form pyridyl is represented by group 2). Another aspect of the invention of formula II wherein X, Y, and Z combine to form pyridyl is represented by group 3). Another aspect of the invention of formula II wherein X, Y, and Z combine to form pyridyl is represented by group 5). Another aspect of the invention of formula II wherein X, Y, and Z combine to form pyridyl is represented by group 6).

Another subembodiment of the invention of formula II is realized when X, Y, and Z combined to form pyrimidinyl represented by group 1). A subembodiment of the invention of formula II where X, Y, and Z combined to form pyrimidinyl represented by group 1) is realized when R¹ is selected from the group consisting of CH₃, CH₂CH₃, CH₂CF₃, CH(CH₃)CF₃, - (CH₂)ₙcyclopropyl, and -(CH₂)ₙcyclobutyl, said CH₃, CH₂CH₃, CH₂CF₃, CH(CH₃)CF₃, - (CH₂)ₙcyclopropyl, and -(CH₂)ₙcyclobutyl optionally substituted with 1 to 3 groups selected from CN and CF₃; R² is selected from pyrrolidinyl, piperidinyl, piperazinyl, azetidinyl, morpholinyl and tetrahydrofuronyl, said pyrrolidinyl, piperidinyl, piperazinyl, azetidinyl, morpholinyl and tetrahydrofuronyl unsubstituted or substituted with 1 to 4 groups independently selected from R⁴; R³ is selected from the group consisting of hydrogen, CH₃, CF₃ and cyclopropyl; and each R⁴ is independently selected from the group consisting of hydrogen, CH₃, OCH₃, C(CH₃)₂OH, C(CH₃)₂CN, CF₃, CH₂F, C(CH3)₂F, cyclopropyl, oxetanyl, oxo, OH, and fluoro, said oxetanyl optionally substituted with 1 to 3 groups of CH₃.

Another subembodiment of the invention of formula II is realized when X, Y, and Z combined to form pyridazinyl represented by group 4). A subembodiment of the invention of formula II where X, Y, and Z combined to form pyridazinyl represented by group 4) is realized when R¹ is selected from the group consisting of CH₃, CH₂CH₃, CH₂CF₃, CH(CH₃)CF₃, - (CH₂)ₙcyclopropyl, and -(CH₂)ₙcyclobutyl, said CH₃, CH₂CH₃, CH₂CF₃, CH(CH₃)CF₃, - (CH₂)ₙcyclopropyl, and -(CH₂)ₙcyclobutyl optionally substituted with 1 to 3 groups selected from CN and CF₃ R² is selected from pyrrolidinyl, piperidinyl, piperazinyl, azetidinyl, morpholinyl and tetrahydrofuronyl, said pyrrolidinyl, piperidinyl, piperazinyl, azetidinyl, morpholinyl and tetrahydrofuronyl unsubstituted or substituted with 1 to 4 groups independently selected from R⁴; R³ is selected from the group consisting of hydrogen, CH₃, CF₃ and cyclopropyl; and each R⁴ is independently selected from the group consisting of hydrogen, CH₃, OCH₃, C(CH₃)₂OH, C(CH₃)₂CN, CF₃, CH₂F, C(CH3)₂F, cyclopropyl, oxetanyl, oxo, OH, and fluoro, said oxetanyl optionally substituted with 1 to 3 groups of CH₃.

In another embodiment, the compounds of the invention include those identified herein as Examples in the tables below, and pharmaceutically acceptable salts thereof.

In another embodiment, the present invention provides pharmaceutical compositions comprising a pharmaceutically acceptable carrier and a compound of the invention or a pharmaceutically acceptable salt thereof.

In another embodiment, the present invention provides a method of treating a disease or disorder in which the LRRK2 kinase is involved, or one or more symptoms or conditions associated with said diseases or disorders, said method comprising administering to a subject (e.g., mammal, person, or patient) in need of such treatment an effective amount of a compound of the invention, or a pharmaceutically acceptable salt thereof, or pharmaceutically acceptable composition thereof. Non-limiting examples of such diseases or disorders, and symptoms associated with such diseases or disorders, each of which comprise additional independent embodiments of the invention, are described below.

Another embodiment provides the use of a compound of the invention, or a pharmaceutically acceptable salt thereof, and a pharmaceutically acceptable carrier, for the manufacture of a medicament for the treatment of Parkinson's Disease. The invention may also encompass the use of a compound of the invention, or a pharmaceutically acceptable salt thereof, and a pharmaceutically acceptable carrier, in therapy.

Another embodiment provides for medicaments or pharmaceutical compositions which may be useful for treating diseases or disorders in which LRRK2 is involved, such as Parkinson's Disease, which comprise a compound of the invention, or a pharmaceutically acceptable salt thereof, and a pharmaceutically acceptable carrier.

Another embodiment provides for the use of a compound of the invention which may be useful for treating diseases or disorders in which LRRK2 is involved, such as Parkinson's Disease.

Another embodiment provides a method for the manufacture of a medicament or a composition which may be useful for treating diseases or disorders in which LRRK2 is involved, such as Parkinson's Disease, comprising combining a compound of the invention with one or more pharmaceutically acceptable carriers.

The compounds of the invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. Unless a specific stereochemistry is indicated, the present invention is meant to encompass all such isomeric forms of these compounds.

The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration.

If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art.

Alternatively, any enantiomer of a compound may be obtained by stereoselective synthesis using optically pure starting materials or reagents of known configuration by methods well known in the art.

In the compounds of Formulae I and II the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formulae I and II. For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds within generic Formulae I and II can be prepared without undue experimentation by conventional techniques well known to those skilled in the art or by processes analogous to those described in the Schemes and Examples herein using appropriate isotopically-enriched reagents and/or intermediates.

When a compound of the invention is capable of forming tautomers, all such tautomeric forms are also included within the scope of the present invention. For example, compounds including carbonyl -CH₂C(O)- groups (keto forms) may undergo tautomerism to form hydroxyl -CH=C(OH)- groups (enol forms). Both keto and enol forms, where present, are included within the scope of the present invention.

When any variable (e.g. R⁵, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is bicyclic, it is intended that the bond be attached to any of the suitable atoms on either ring of the bicyclic moiety.

It is understood that one or more silicon (Si) atoms can be incorporated into the compounds of the instant invention in place of one or more carbon atoms by one of ordinary skill in the art to provide compounds that are chemically stable and that can be readily synthesized by techniques known in the art from readily available starting materials. Carbon and silicon differ in their covalent radius leading to differences in bond distance and the steric arrangement when comparing analogous C-element and Si-element bonds. These differences lead to subtle changes in the size and shape of silicon-containing compounds when compared to carbon. One of ordinary skill in the art would understand that size and shape differences can lead to subtle or dramatic changes in potency, solubility, lack of off-target activity, packaging properties, and so on. (Diass, J. O. et al. Organometallics (2006) 5:1188-1198; Showell, G.A. et al. Bioorganic & Medicinal Chemistry Letters (2006) 16:2555-2558).

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted with one or more substituents" should be understood as meaning that the group in question is either unsubstituted or may be substituted with one or more substituents.

Absolute stereochemistry is illustrated by the use of hashed and solid wedge bonds. As shown in Illus-I and Illus-II. Accordingly, the methyl group of Illus-I is emerging from the page of the paper and the ethyl group in Illus-II is descending into the page, where the cyclohexene ring resides within the plane of the paper. It is assumed that the hydrogen on the same carbon as the methyl group of Illus-I descends into the page and the hydrogen on the same carbon as the ethyl group of Illus-II emerges from the page. The convention is the same where both a hashed and solid rectangle are appended to the same carbon as in Illus-III, the methyl group is emerging from the plane of the paper and the ethyl group is descending into the plane of the paper with the cyclohexene ring in the plane of the paper.

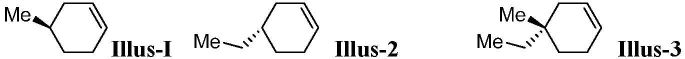

As is conventional, unless otherwise noted in accompanying text, ordinary "stick" bonds or "wavy" bonds indicate that all possible stereochemistry is represented, including, pure compounds, mixtures of isomers, and racemic mixtures.

As used herein, unless otherwise specified, the following terms have the following meanings:
The phrase "at least one" used in reference to the number of components comprising a composition, for example, "at least one pharmaceutical excipient" means that one member of the specified group is present in the composition, and more than one may additionally be present. Components of a composition are typically aliquots of isolated pure material added to the composition, where the purity level of the isolated material added into the composition is the normally accepted purity level for a reagent of the type.

"at least one" used in reference to substituents appended to a compound substrate, for example, a halogen or a moiety appended to a portion of a structure replacing a hydrogen, means that one substituent of the group of substituents specified is present, and more than one of said substituents may be bonded to any of the defined or chemically accessible bonding points of the substrate.

Whether used in reference to a substituent on a compound or a component of a pharmaceutical composition the phrase "one or more", means the same as "at least one";
"concurrently" and "contemporaneously" both include in their meaning (1) simultaneously in time (e.g., at the same time); and (2) at different times but within the course of a common treatment schedule;
"consecutively" means one following the other;
"sequentially" refers to a series administration of therapeutic agents that awaits a period of efficacy to transpire between administering each additional agent; this is to say that after administration of one component, the next component is administered after an effective time period after the first component; the effective time period is the amount of time given for realization of a benefit from the administration of the first component;
"effective amount" or "therapeutically effective amount" is meant to describe the provision of an amount of at least one compound of the invention or of a composition comprising at least one compound of the invention which is effective in treating or inhibiting a disease or condition described herein, and thus produce the desired therapeutic, ameliorative, inhibitory or preventative effect. For example, in treating central nervous system diseases or disorders with one or more of the compounds described herein "effective amount" (or "therapeutically effective amount") means, for example, providing the amount of at least one compound of Formula I, Formula II, Formula III, or Formula IV that results in a therapeutic response in a patient afflicted with a central nervous system disease or disorder ("condition"), including a response suitable to manage, alleviate, ameliorate, or treat the condition or alleviate, ameliorate, reduce, or eradicate one or more symptoms attributed to the condition and/or long-term stabilization of the condition, for example, as may be determined by the analysis of pharmacodynamic markers or clinical evaluation of patients afflicted with the condition;
"patient" and "subject" means an animal, such as a mammal (e.g., a human being) and is preferably a human being;
"prodrug" means compounds that are rapidly transformed, for example, by hydrolysis in blood, *in vivo* to the parent compound, e.g., conversion of a prodrug of Formula I through Formula IV to a compound of Formula I, Formula II, Formula III, or Formula IV or to a salt thereof; a thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

The term "substituted" means that one or more of the enumerated substituents can occupy one or more of the bonding positions on the substrate typically occupied by "-H", provided that such substitution does not exceed the normal valency rules for the atom in the bonding configuration presented in the substrate, and that the substitution ultimately provides a stable compound, which is to say that such substitution does not provide compounds with mutually reactive substituents located geminal or vicinal to each other; and wherein the substitution provides a compound sufficiently robust to survive isolation to a useful degree of purity from a reaction mixture.

Where optional substitution of a moiety is described (e.g. "optionally substituted") the term means that if substituents are present, one or more of the enumerated substituents for the specified substrate can be present on the substrate in a bonding position normally occupied by the default substituent normally occupying that position. For example, a default substituent on the carbon atoms of an alkyl moiety is a hydrogen atom, an optional substituent can replace the default substituent.

As used herein, unless otherwise specified, the following terms used to describe moieties, whether comprising the entire definition of a variable portion of a structural representation of a compound of the invention or a substituent appended to a variable portion of a structural representation of a group of compounds of the invention have the following meanings, and unless otherwise specified, the definitions of each term (i.e., moiety or substituent) apply when that term is used individually or as a component of another term (e.g., the definition of aryl is the same for aryl and for the aryl portion of arylalkyl, alkylaryl, arylalkynyl moieties, and the like); moieties are equivalently described herein by structure, typographical representation or chemical terminology without intending any differentiation in meaning, for example, an "acyl" substituent may be equivalently described herein by the term "acyl", by typographical representations "R'-(C=O)-" or "R'-C(O)-", or by a structural representation: 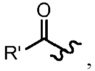 equally, with no differentiation implied using any or all of these representations;

The term "alkyl" (including the alkyl portions of other moieties, such as trifluoromethyl- alkyl- and alkoxy-) means a straight or branched aliphatic hydrocarbon moiety comprising up to about 20 carbon atoms (for example, a designation of "C₁₋₂₀ -alkyl" indicates an aliphatic hydrocarbon moiety of from 1 to 20 carbon atoms). In some embodiments, alkyls preferably comprise up to about 10 carbon atoms, unless the term is modified by an indication that a shorter chain is contemplated, for example, an alkyl moiety of from 1 up to 8 carbon atoms is designated herein "C₁₋₈-alkyl". Where the term "alkyl" is indicated with two hyphens (i.e., "-alkyl-" it indicates that the alkyl moiety is bonded in a manner that the alkyl moiety connects the substituents on either side of it, for example, "-alkyl-OH" indicates an alkyl moiety connecting a hydroxyl moiety to a substrate.

The term "haloalkyl" refers to an alkyl in which one or more hydrogen atoms are replaced by halo, and includes alkyl moieties in which all hydrogens have been replaced by halo (e.g., perfluoroalkyl). Alkyl and haloalkyl groups may be optionally inserted with O, N, or S.

The term "cycloalkyl" means a moiety having a main hydrocarbon chain forming a mono- or bicyclo- cyclic aliphatic moiety comprising at least 3 carbon atoms (the minimum number necessary to provide a monocyclic moiety) up to the maximum number of specified carbon atoms, generally 8 for a monocyclic moiety and 10 for a bicyclic moiety. Examples of cycloalkyl moieties include, but are not limited to, cyclopropyl, cyclobutyl, cyclopentyl or cyclohexyl. The term "cycloalkyl" also includes non-aromatic, fused multicyclic ring system comprising up to 20 carbon atoms which may optionally be substituted as defined herein for "alkyl" generally. Suitable multicyclic cycloalkyls are, for example, but are not limited to: 1-decalin; norbornyl; adamantly; and the like;

As used herein, when the term "alkyl" is modified by "substituted" or "optionally substituted", it means that one or more C-H bonds in the alkyl moiety group is substituted, or optionally may be substituted, by a substituent bonded to the alkyl substrate which is called out in defining the moiety.

Where a structural formula represents bonding between a moiety and a substrate using a bonding line that terminates in the middle of the structure, for example the following representations: 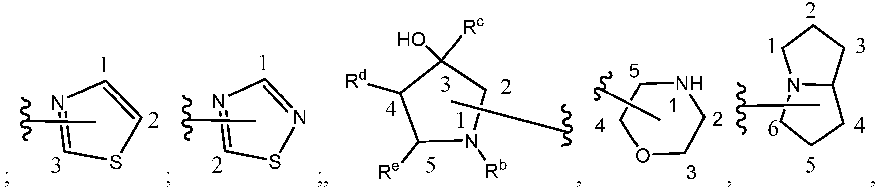 whether or not numbered the structure indicates that unless otherwise defined the moiety may be bonded to the substrate through any of available ring atom, for example, the numbered atoms of the example moieties;

The term "heterocyclyl" (or heterocycloalkyl) means a non-aromatic saturated monocyclic or multicyclic ring system comprising 3 to 10 ring atoms, preferably 5 to 10 ring atoms, in which one or more of the atoms in the ring system is an element other than carbon, for example nitrogen (e.g. piperidyl- or pyrrolidinyl), oxygen (e.g. furanyl and tetrahydropyranyl) or sulfur (e.g. tetrahydrothiopheneyl and tetrahydrothiopyranyl); and wherein the heteroatoms can be alone or in combination provided that the moiety does not contain adjacent oxygen and/or sulfur atoms present in the ring system; preferred heterocyclyl moieties contain 5 to 6 ring atoms; the prefix aza, oxa or thia before the heterocyclyl root name means that at least one nitrogen, oxygen or sulfur atom, respectively, is present as a ring atom; the heterocyclyl can be optionally substituted by one or more independently selected substituents;

The nitrogen or sulfur atom of the heterocyclyl can be optionally oxidized to the corresponding N-oxide, S-oxide or S,S-dioxide (SO₂); non-limiting examples of suitable monocyclic heterocyclyl rings include piperidyl, pyrrolidinyl, piperazinyl, morpholinyl - 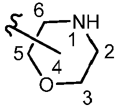 (where unless otherwise noted the moiety is bonded to the substrate through any of ring carbon atoms C2, C3, C5, or C6), thiomorpholinyl, thiazolidinyl, 1,3-dioxolanyl, 1,4-dioxanyl, tetrahydrofuranyl, tetrahydrothiophenyl, tetrahydrothiopyranyl, and the like; and polycyclicheterocyclyl compounds, for example, moieties of the structure: 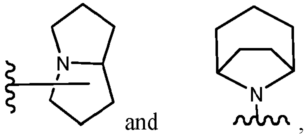 and the like.

The term "halogen" means fluorine, chlorine, bromine, or iodine; preferred halogens, unless specified otherwise where the term is used, are fluorine, chlorine and bromine, a substituent which is a halogen atom means -F, -Cl, -Br, or -I, and "halo" means fluoro, chloro, bromo, or iodo substituents bonded to the moiety defined, for example, "haloalkyl" means an alkyl, as defined above, wherein one or more of the bonding positions on the alkyl moiety typically occupied by hydrogen atoms are instead occupied by a halo group, perhaloalkyl (or "fully halogenated" alkyl) means that all bonding positions not participating in bonding the alkyl substituent to a substrate are occupied by a halogen, for example, where the alkyl is selected to be methyl, the term perfluoroalkyl means -CF₃;

The term "hydroxyl" and "hydroxy" means an HO- group, "hydroxyalkyl" means a substituent of the formula: "HO-alkyl-",wherein the alkyl group is bonded to the substrate and may be substituted or unsubstituted as defined above; preferred hydroxyalkyl moieties comprise a lower alkyl; Non-limiting examples of suitable hydroxyalkyl groups include hydroxymethyl and 2-hydroxyethyl; and

The bonding sequence is indicated by hyphens where moieties are represented in text, for example -alkyl, indicates a single bond between a substrate and an alkyl moiety, -alkyl-X, indicates that an alkyl group bonds an "X" substituent to a substrate, and in structural representation, bonding sequence is indicated by a wavy line terminating a bond representation, for example: 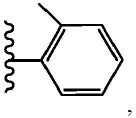 indicates that the methylphenyl moiety is bonded to a substrate through a carbon atom ortho to the methyl substituent, while a bond representation terminated with a wavy line and drawn into a structure without any particular indication of an atom to which it is bonded indicates that the moiety may be bonded to a substrate via any of the atoms in the moiety which are available for bonding as described in the examples above.

The line -, as a bond generally indicates a mixture of, or either of, the possible isomers, e.g., containing (R)- and (S)- stereochemical configuration. For example: 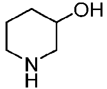 encompasses

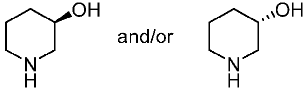

Furthermore, unwedged-bolded or unwedged-hashed lines are used in structures containing multiple stereocenters in order to depict relative configuration where it is known. For example: 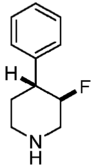 means that the fluorine and hydrogen atoms are on the same face of the piperidine ring, but represents a mixture of, or one of, the possible isomers at right 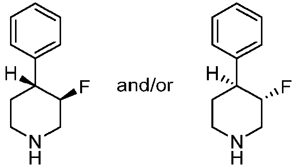 whereas: 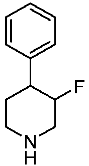 represents a mixture of, or one of, the possible isomers at right

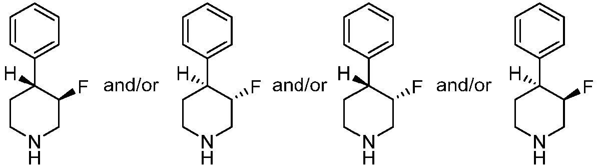

In all cases, compound name(s) accompany the structure drawn and are intended to capture each of the stereochemical permutations that are possible for a given structural isomer based on the synthetic operations employed in its preparation. Lists of discrete stereoisomers that are conjoined using or indicate that the presented compound (e.g. 'Example number') was isolated as a single stereoisomer, and that the identity of that stereoisomer corresponds to one of the possible configurations listed. Lists of discrete stereoisomers that are conjoined using and indicate that the presented compound was isolated as a racemic mixture or diastereomeric mixture.

A specific absolute configuration is indicated by use of a wedged-bolded or wedged-hashed line. Unless a specific absolute configuration is indicated, the present invention is meant to encompass all such stereoisomeric forms of these compounds.

In this specification, where there are multiple oxygen and/or sulfur atoms in a ring system, there cannot be any adjacent oxygen and/or sulfur present in said ring system.

As well known in the art, a bond drawn from a particular atom wherein no moiety is depicted at the terminal end of the bond indicates a methyl group bound through that bond to the atom, unless stated otherwise. For example: 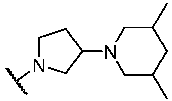 represents

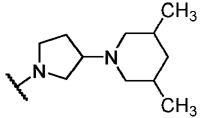

Unsatisfied valences in the text, schemes, examples, structural formulae, and any Tables herein is assumed to have a hydrogen atom or atoms of sufficient number to satisfy the valences.

One or more compounds of the invention may also exist as, or optionally be converted to, a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, and hemisolvate, including hydrates (where the solvent is water or aqueous-based) and the like are described by E. C. van Tonder et al, AAPS PharmSciTech., 5(1), article 12 (2004); and A. L. Bingham et al, Chem. Commun., 603-604 (2001). A typical, non-limiting, process involves dissolving the inventive compound in desired amounts of the desired solvent (for example, an organic solvent, an aqueous solvent, water or mixtures of two or more thereof) at a higher than ambient temperature, and cooling the solution, with or without an antisolvent present, at a rate sufficient to form crystals which are then isolated by standard methods. Analytical techniques such as, for example I.R. spectroscopy, show the presence of the solvent (including water) in the crystals as a solvate (or hydrate in the case where water is incorporated into the crystalline form).

This invention also includes the compounds of this invention in isolated and purified form obtained by routine techniques. Polymorphic forms of the compounds of Formula I, Formula II, Formula III, and Formula IV and of the salts and solvates of the compounds of Formula I, Formula II, Formula III, and Formula IV are intended to be included in the present invention. Certain compounds of the invention may exist in different isomeric forms (e.g., enantiomers, diastereoisomers, atropisomers). The inventive compounds include all isomeric forms thereof, both in pure form and admixtures of two or more, including racemic mixtures.

In the same manner, unless indicated otherwise, presenting a structural representation of any tautomeric form of a compound which exhibits tautomerism is meant to include all such tautomeric forms of the compound. Accordingly, where compounds of the invention, their salts, and solvates thereof, may exist in different tautomeric forms or in equilibrium among such forms, all such forms of the compound are embraced by, and included within the scope of the invention. Examples of such tautomers include, but are not limited to, ketone/enol tautomeric forms, imine-enamine tautomeric forms, and for example heteroaromatic forms such as the following moieties:

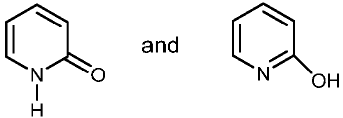

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives wherein the parent compound is modified by making acid or base salts thereof. Salts in the solid form may exist in more than one crystal structure and may also be in the form of hydrates. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. Salts derived from inorganic bases include aluminum, ammonium, calcium, copper, ferric, ferrous, lithium, magnesium, manganic salts, manganous, potassium, sodium, zinc, and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. In one aspect of the invention the salts are citric, hydrobromic, hydrochloric, maleic, phosphoric, sulfuric, fumaric, and tartaric acids. Similarly, the salts of the acidic compounds are formed by reactions with the appropriate inorganic or organic base.

The terms "treating" or "treatment" (of, e.g., a disease, disorder, or conditions or associated symptoms, which together or individually may be referred to as "indications") as used herein include: inhibiting the disease, disorder or condition, i.e., arresting or reducing the development of the disease or its biological processes or progression or clinical symptoms thereof; or relieving the disease, i.e., causing regression of the disease or its biological processes or progression and/or clinical symptoms thereof. "Treatment" as used herein also refers to control, amelioration, or reduction of risks to the subject afflicted with a disease, disorder or condition in which LRRK2 is involved. The terms "preventing" or "prevention" or "prophylaxis" of a disease, disorder or condition as used herein includes: impeding the development or progression of clinical symptoms of the disease, disorder, or condition in a mammal that may be exposed to or predisposed to the disease, disorder or condition but does not yet experience or display symptoms of the disease, and the like.

As would be evident to those skilled in the art, subjects treated by the methods described herein are generally mammals, including humans and non-human animals (e.g., laboratory animals and companion animals), in whom the inhibition of LRRK2 kinase activity is indicated or desired. The term "therapeutically effective amount" means the amount of the subject compound that will elicit the biological or medical response of a tissue, system, animal or human that is being sought by the researcher, veterinarian, medical doctor or other clinician.

The term "composition" as used herein is intended to encompass a product comprising a compound of the invention or a pharmaceutically acceptable salt thereof, together with one or more additional specified ingredients in the specified amounts, as well as any product which results, directly or indirectly, from combination of the specified ingredients in the specified amounts. Such term in relation to a pharmaceutical composition, is intended to encompass a product comprising the active ingredient(s), which include a compound of the invention or a pharmaceutically acceptable salt thereof, optionally together with one or more additional active ingredients, and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention, or a pharmaceutically acceptable salt thereof, and a pharmaceutically acceptable carrier. By "pharmaceutically acceptable" it is meant the carrier, diluent or excipient must be compatible with the other ingredients of the formulation and not deleterious to the recipient thereof.

As noted above, additional embodiments of the present invention are each directed to a method for the treatment a disease, disorder, or condition, or one or more symptoms thereof ("indications") in which the LRRK2 kinase is involved and for which the inhibition of LRRK2 kinase is desired, which method comprises administering to a subject in need of such treatment a therapeutically effective amount of a compound of the invention, or a pharmaceutically acceptable salt thereof, or a pharmaceutical composition comprising said compound or salt thereof.

In another embodiment, the present invention is directed to a method for the manufacture of a medicament for inhibition of LRRK2 receptor activity in a subject comprising combining a compound of the present invention, or a pharmaceutically acceptable salt thereof, with a pharmaceutical carrier or diluent.

One such embodiment provides a method of treating Parkinson's disease in a subject in need thereof, said method comprising administering to a subject in need of such treatment a therapeutically effective amount of a compound of the invention, or a pharmaceutically acceptable salt thereof, or a pharmaceutical composition comprising said compound or salt thereof. In one such embodiment, the subject is a human.

Another embodiment provides a method for the treatment or prophylaxis of neurologic damage associated with Parkinson's disease in a subject in need thereof. Another embodiment provides a method of treating or improving dopaminergic tone to provide symptomatic relief in a subject in need thereof, for example, in treating, alleviating, ameliorating, or managing motor and non-motor symptoms of Parkinson's disease.

Another embodiment provides a method for the treatment or prophylaxis of abnormal motor symptoms associated with Parkinson's disease (including but not limited to bradykinesia, rigidity and resting tremor). Another embodiment provides a method for the treatment or prophylaxis of abnormal non-motor symptoms associated with Parkinson's disease (including but not limited to cognitive dysfunction, autonomic dysfunction, emotional changes and sleep disruption); Lewy body dementia; and L-Dopa induced dyskinesias. Each said method independently comprises administering to a patient in need of such treatment an effective amount of a compound of the invention, or a pharmaceutically acceptable salt thereof, or pharmaceutically acceptable composition thereof.

Non-limiting examples of additional indications in which LRRK2 is involved and in which the treatment or prophylaxis of said indications in a subject in need thereof are contemplated include the following, each of which, alone or in combination, comprise additional embodiments of the invention: Alzheimer's disease, mild cognitive impairment, the transition from mild cognitive impairment to Alzheimer's disease, tauopathy disorders characterized by hyperphosphorylation of tau such as argyrophilic grain disease, Picks disease, corticobasal degeneration, progressive supranuclear palsy, inherited frontotemporal dementia, and Parkinson's disease linked to chromosome 17.

Additional indications include neuroinflammation, including neuroinflammation associated with of microglial inflammatory responses associated with multiple sclerosis, HIV-induced dementia, ALS, ischemic stroke, traumatic brain injury and spinal cord injury.

Additional indications include diseases of the immune system including lymphomas, leukemias, multiple sclerosis, rheumatoid arthritis, systemic lupus erythematosus, autoimmune hemolytic anemia, pure red cell aplasia, idiopathic thrombocytopenic pupura (ITP), Evans Syndrome, vasculitis, bullous skin disorder, type I diabetes mellitus, Sjorgen's syndrome, Delvic's disease, inflammatory myopathies, and ankylosing spondylitis.

Additional indications include renal cancer, breast cancer, lung cancer, prostate cancer, and acute myelogenous leukemia (AML) in subjects expressing the LRRK2 G2019S mutation.

Additional indications include papillary renal and thyroid carcinomas in a subject in whom LRRK2 is amplified or overexpressed.

Additional indications include chronic autoimmune diseases including Crohn's disease and leprosy.

Disclosed but not part of the invention are prodrugs of the compounds of this invention. In general, such prodrugs will be functional derivatives of the compounds of this invention which are readily convertible *in vivo* into the required compound. Thus, in the methods of treatment of the present invention, the terms "administration of" or "administering a" compound shall encompass the treatment of the various conditions described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs," ed. H. Bundgaard, Elsevier, 1985. Metabolites of these compounds include active species produced upon introduction of compounds of this invention into the biological milieu.

The compounds of the present invention may be used in combination with one or more other drugs in the treatment, prevention, control, amelioration, or reduction of risk of diseases or conditions for which compounds of Formula I and Formula II, or the other drugs may have utility, where the combination of the drugs together are safer or more effective than either drug alone. Such other drug(s) may be administered, by a route and in an amount commonly used therefore, contemporaneously or sequentially with a compound of Formula I. When a compound Formula I and Formula II is used contemporaneously with one or more other drugs, a pharmaceutical composition in unit dosage form containing such other drugs and the compound of Formula I and Formula II is preferred. However, the combination therapy may also include therapies in which the compound of Formula I and Formula II and one or more other drugs are administered on different overlapping schedules. It is also contemplated that when used in combination with one or more other active ingredients, the compounds of the present invention and the other active ingredients may be used in lower doses than when each is used singly. Accordingly, the pharmaceutical compositions of the present invention include those that contain one or more other active ingredients, in addition to a compound of Formula I or Formula II.

For example, the present compounds may be used in conjunction with one or more additional therapeutic agents, for example: L-DOPA; dopaminergic agonists such as quinpirole, ropinirole, pramipexole, pergolide and bromocriptine; MAO-B inhibitors such as rasagiline, deprenyl and selegiline; DOPA decarboxylase inhibitors such as carbidopa and benserazide; and COMT inhibitors such as tolcapone and entacapone;or potential therapies such as an adenosine A2a antagonists, metabotropic glutamate receptor 4 modulators, or growth factors such as brain derived neurotrophic factor (BDNF), and a pharmaceutically acceptable carrier.

The above combinations include combinations of a compound of the present invention not only with one other active compound, but also with two or more other active compounds. Likewise, compounds of the present invention may be used in combination with other drugs that are used in the prevention, treatment, control, amelioration, or reduction of risk of the diseases or conditions for which compounds of the present invention are useful. Such other drugs may be administered, by a route and in an amount commonly used therefore, contemporaneously or sequentially with a compound of the present invention. When a compound of the present invention is used contemporaneously with one or more other drugs, a pharmaceutical composition containing such other drugs in addition to the compound of the present invention is preferred. Accordingly, the pharmaceutical compositions of the present invention include those that also contain one or more other active ingredients, in addition to a compound of the present invention.

The weight ratio of the compound of the present invention to the other active ingredient(s) may be varied and will depend upon the effective dose of each ingredient. Generally, an effective dose of each will be used. Thus, for example, when a compound of the present invention is combined with another agent, the weight ratio of the compound of the present invention to the other agent will generally range from about 1000:1 to about 1: 1000, or from about 200:1 to about 1:200. Combinations of a compound of the present invention and other active ingredients will generally also be within the aforementioned range, but in each case, an effective dose of each active ingredient should be used.

In such combinations the compound of the present invention and other active agents may be administered separately or in conjunction. In addition, the administration of one element may be prior to, concurrent to, or subsequent to the administration of other agent(s), and via the same or different routes of administration.

The compounds of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, buccal or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the compounds of the invention are effective for use in humans.

The pharmaceutical compositions for the administration of the compounds of this invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, com starch, or alginic acid; binding agents, for example starch, gelatin or acacia; and lubricating agents, for example magnesium stearate, stearic acid or talc. The tablets may be uncoated, or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,166,452; and 4,265,874 to form osmotic therapeutic tablets for control release. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or acetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compounds of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

The pharmaceutical composition and method of the present invention may further comprise other therapeutically active compounds as noted herein which are usually applied in the treatment of the above-mentioned pathological conditions.

In the treatment, prevention, control, amelioration, or reduction of risk of conditions which require inhibition of LRRK2 kinase activity an appropriate dosage level will generally be about 0.01 to 500 mg per kg patient body weight per day which can be administered in single or multiple doses. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions may be provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day or may be administered once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Methods for preparing the compounds of this invention are illustrated in the following Schemes and Examples. Starting materials are made according to procedures known in the art or as illustrated herein.

### Preparative Examples

The compounds of the present invention can be prepared according to the following schemes and specific examples, or modifications thereof, using readily available starting materials, reagents and conventional synthesis procedures. It is also possible to make use of variants which are themselves known to those of ordinary skill in this art but are not mentioned in detail. The general procedures for making the compounds claimed in this invention can be readily understood by one skilled in the art from viewing the following schemes and descriptions. Abbreviations used in the experimentals may include, but are not limited to the following:

| | |
|---|---|
| AcOH | Acetic Acid |
| NH₄Cl | Ammonium Chloride |
| calc'd | Calculated |
| COMU | (1-Cyano-2-ethoxy-2-oxoethylidenaminooxy)dimethylamino-morpholino-carbenium hexafluorophosphate |
| DAST | Diethylaminosulfur trifluoride |
| DCE | Dichloroethane |
| DCM | Dichloromethane |
| DIAD | Diisopropyl azodicarboxylate |
| DIEA | N,N-Diisopropylethylamine |
| DMAP | 4-Dimethylaminopyridine |
| DMF | Dimethylformamide |
| DMSO | Dimethyl sulfoxide |
| EGTA | Ethylene glycol tetraacetic acid |
| EtOAc | Ethyl Acetate |
| FDPP | Pentafluorophenyl diphenylphosphinate |
| ¹H-NMR | Proton nuclear magnetic resonance |
| HPLC | High performance liquid chromatography |
| HATU | 1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide |
| Hr(s) | Hours |
| HCl | Hydrochloric Acid |
| iPr₂NEt | N,N-Diisopropylethylamine |
| K₃PO₄ | Potassium Phosphate |
| MeCN | Acetonitrile |
| Min | Minutes |
| MPTP | 1-methyl-4-phenyl-1,2,3,6-tetrahydropyridine |
| MS | Mass spectrometry |
| m/z | Mass to charge ratio |
| Na₂SO₄ | Sodium Sulfate |
| NMP | *N*-Methyl-2-pyrrolidone |
| NMR | Nuclear Magnetic Resonance |
| PCy3 | Tricyclohexylphosphine |
| Pd(OAc)₂ | Palladium(II) acetate |
| PhMe | Toluene |
| PyAOP | (7-Azabenzotriazol-1-yloxy)trispyrrolidinophosphonium hexafluorophosphate |
| RT | Room Temperature |
| RuPhos Pd G2 | Chloro(2-dicyclohexylphosphino-2',6'-diisopropoxy-1,1'-biphenyl)[2-(2'-amino-1,1'-biphenyl)]palladium(II) chloride |
| | 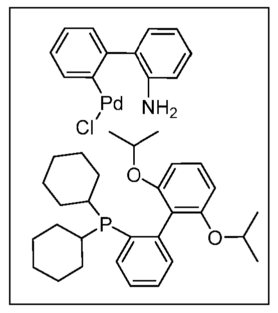 |
| | |
| RuPhos Pd G3 | 2-Dicyclohexylphosphino-2',6'-diisopropoxy-1,1'-biphenyl)[2-(2'-amino-1,1'-biphenyl)]palladium(II) methanesulfonate |
| | 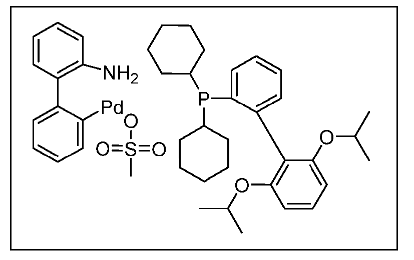 |
| SEM | Trimethylsilylethoxymethyl |
| STAB | Sodium Triacetoxyborohydride |
| TEA | Triethylamine |
| TFA | Trifluoroacetic Acid |
| THF | Tetrahydrofuran |
| t_{R} | Retention time |
| XantPhos Pd G2 | Chloro[(4,5-bis(diphenylphosphino)-9,9-dimethylxanthene)-2-(2'-amino-1,1'-biphenyl)]palladium(II) |
| | 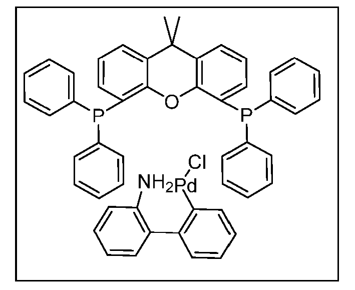 |
| XantPhos Pd G3 | [(4,5-Bis(diphenylphosphino)-9,9-dimethylxanthene)-2-(2'-amino-1,1'-biphenyl)]palladium(II) methanesulfonate |
| | 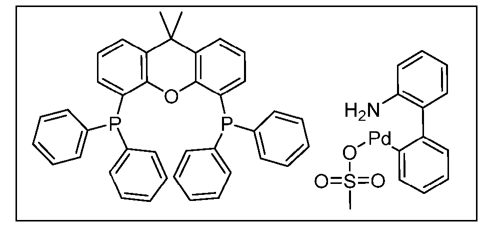 |
| | Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride |
| Xphos Pd G2 | 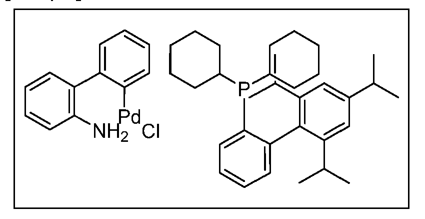 |

### General Experimental Information:

Unless otherwise noted, all reactions are magnetically stirred. Unless otherwise noted, "concentrated" and/or "solvent removed under reduced pressure" means evaporating the solvent from a solution or mixture using a rotary evaporator or vacuum pump. Unless otherwise noted, flash chromatography is carried out on a Teledyne Isco (Lincoln, NE), Analogix (Burlington, WI), or Biotage (Stockholm, SWE) automated chromatography system using a commercially available cartridge as the column. Columns may be purchased from Teledyne Isco, Analogix, Biotage, Varian (Palo Alto, CA), or Supelco (Bellefonte, PA) and are usually filled with silica gel as the stationary phase. Reverse phase prep-HPLC conditions, where used, can be found at the end of each experimental section. Aqueous solutions were concentrated on a Genevac (Ipswich, ENG) or by freeze-drying/lyophilization. Unless otherwise noted, all LRRK2 pIC₅₀ data presented in tables refers to the LRRK2 G2019S Kₘ ATP LanthaScreen^{™} assay (Life Technologies Corp., Carlsbad, CA) that is described in the Biological Assay section.

### SYNTHESIS OF COMMON INTERMEDIATES

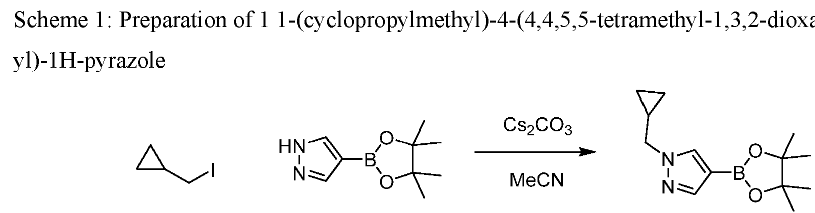

General Procedure: A 30 mL microwave vial was charged with 4-(4,4,5,5-tetramethyl-1,3,2-dioxaborolan-2-yl)-1H-pyrazole (200 mg, 1.03 mmol), (iodomethyl)cyclopropane (167 mg, 1.24 mmol) and Cs₂CO₃ (672 mg, 2.06 mmol) in MeCN (5 mL). The reaction was stirred at 80 °C for 12 hrs. At 12 hrs, the mixture was diluted with DCM and poured into a separatory funnel containing deionized water (20 mL). The phases were separated and the aqueous phase was extracted with DCM (3 x 25 mL). The organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 1. MS (ESI) m/z 249 [(M+H)⁺ calc'd for C₁₃H₂₁BN₂O₂: 249]

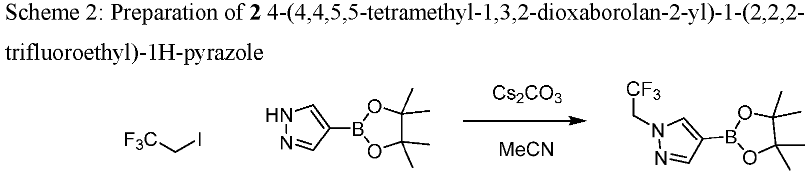

General Procedure: A 250 mL round bottom flask was charged with 4-(4,4,5,5-tetramethyl-1,3,2-dioxaborolan-2-yl)-1H-pyrazole (5 g, 25.8 mmol), Cs₂CO₃ (12.6 g, 38.7 mmol), MeCN (100 mL) and 1,1,1-trifluoro-2-iodoethane (8.11 g, 38.7 mmol). The reaction mixture was stirred at 80 °C for 12 hrs. At 12 hrs, the mixture was diluted with DCM and poured into a separatory funnel containing deionized water (100 mL). The phases were separated, and the aqueous phase was extracted with DCM (3 x 100 mL). The organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 2. MS (ESI) m/z 277 [(M+H)⁺ calc'd for C₁₁H₁₆BF₃N₂O₂: 277]

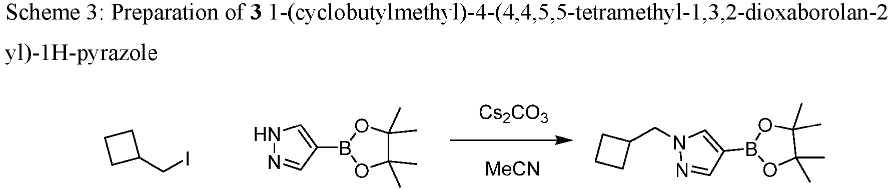

General Procedure: A 30 mL microwave vial was charged with 4-(4,4,5,5-tetramethyl-1,3,2-dioxaborolan-2-yl)-1H-pyrazole (350 mg, 1.8 mmol), Cs₂CO₃ (1.17 g, 3.61 mmol), MeCN (10 mL) and (iodomethyl)cyclobutane (323 mg, 2.16 mmol). The reaction mixture was stirred at 80 °C overnight for 12 hrs. At 12 hrs, the mixture was diluted with DCM and poured into a separatory funnel containing deionized water (20 mL). The phases were separated, and the aqueous phase was extracted with DCM (3 x 25 mL). The organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 3. MS (ESI) m/z 263 [(M+H)⁺ calc'd for C₁₄H₂₃BN₂O₂: 263]

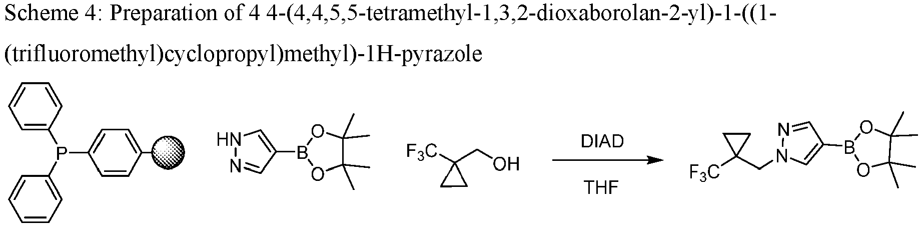

General Procedure: A 150 mL round-bottom flask was charged with 4-(4,4,5,5-tetramethyl-1,3,2-dioxaborolan-2-yl)-1H-pyrazole (1.71 g, 8.76 mmol), triphenylphosphine-resin (5.87 g, 17.5 mmol), 1-(trifluoromethyl)cyclopropyl)methanol (1.14 g, 8.12 mmol), and THF (22 mL). The flask was cooled to 0 °C and a solution of DIAD (3.44 mL, 17.5 mmol) in THF (22 mL) was added. The reaction mixture was allowed to warm to RT overnight for 12 hrs. At 12 hrs, the mixture was filtered and concentrated under reduced pressure. The crude mixture was purified by flash chromatography using a pre-packed RediSep R_{f} silica gel column on a Teledyne Isco CombiFlash R_{f} automated chromatography system using 0-50% EtOAc/hexanes to afford the title compound 4. MS (ESI) m/z 317 [(M+H)⁺ calc'd for C₁₄H₂₀BF₃N₂O₂: 317]

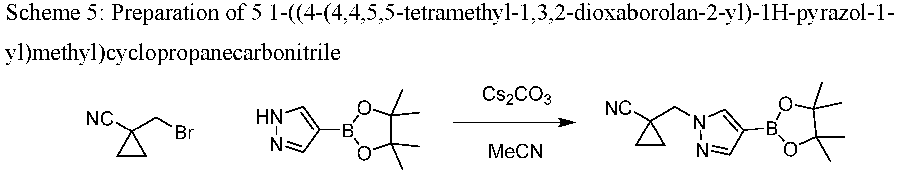

General Procedure: A 30 mL microwave vial was charged with 4-(4,4,5,5-tetramethyl-1,3,2-dioxaborolan-2-yl)-1H-pyrazole (67.2 mg, 0.341 mmol), Cs₂CO₃ (305 mg, 0.941 mmol), MeCN (5 mL) and 1-(bromomethyl)cyclopropanecarbonitrile (50 mg, 0.31 mmol). The reaction mixture was stirred at 80 °C for 12 hrs. At 12 hrs, the mixture was diluted with DCM and poured into a separatory funnel containing deionized water (20 mL). The phases were separated, and the aqueous phase was extracted with DCM (3 x 25 mL). The organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 5. MS (ESI) m/z 274 [(M+H)⁺ calc'd for C₁₄H₂₀BN₃O₂: 274]

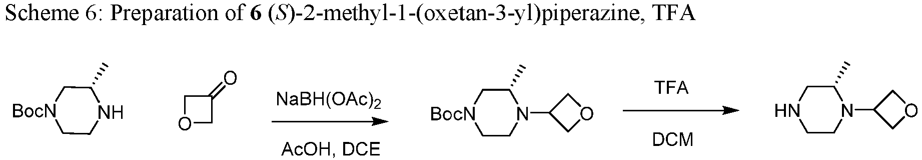

### Step 1: Preparation for tert-butyl (S)-3-methyl-4-(oxetan-3-yl)piperazine-1-carboxylate

General Procedure: A 1 L round bottom flask was charged with tert-butyl (3S)-3-methylpiperazine-1-carboxylate (170 g, 0.851 mol), DCE (1.7 L), STAB (342 g, 1.62 mol), AcOH (71.4 g, 1.19 mol), and oxetan-3-one (122 g, 1.71 mol). The resulting solution was stirred for 12 h at RT. At 12 hrs, the reaction was quenched by the addition of 500 mL of water. The resulting solution was extracted with DCM (3 x 500 mL) and the organic layers combined. The organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using EtOAc/petroleum ether 0-80% to afford tert-butyl (3*S*)-3-methyl-4-(oxetan-3-yl)piperazine-1-carboxylate. MS (ESI) m/z 257 [(M+H)⁺ calc'd for C₁₃H₂₄N₂O₃: 257].

### Step 2: Preparation of (S)-2-methyl-1-(oxetan-3-yl)piperazine, TFA

General Procedure: A 1-L round-bottom flask was charged with TFA (554 mL) and the mixture was cooled down at 0 °C. tert-butyl (3*S*)-3-methyl-4-(oxetan-3-yl)piperazine-1-carboxylate (185 g) was added slowly to the round bottom flask. The resulting solution was stirred for 4 h at RT. The resulting mixture was concentrated under vacuum to afford the title compound 6. MS (ESI) m/z 157 [(M+H)⁺ calc'd for C₈H₁₆N₂O: 157].

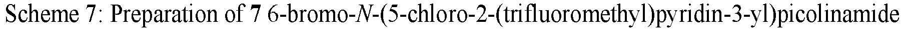

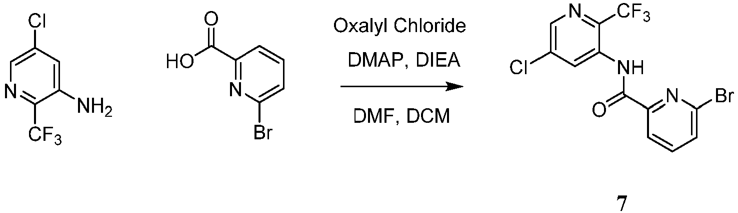

General Procedure: A 100 mL round bottom flask was charged with 6-bromopicolinic acid (3.3 g, 15 mmol) and DCM (15.3 mL) under inert atmosphere. The reaction mixture was cooled to 0°C and to the stirring mixture, DMF (2 drops) and oxalyl chloride (3.31 mL, 37.6 mmol) were added. The resultant mixture was stirred at 0 °C for 30 min. The reaction mixture was concentrated under reduced pressure and diluted in DCM (15.4 mL). Under inert atmosphere, 5-chloro-2-(trifluoromethyl)pyridin-3-amine, HCl (1.75 g, 7.51 mmol), DIEA (6.56 mL, 37.6 mmol), and DMAP (0.91 g, 7.5 mmol) were added. The reaction mixture was stirred at 0 °C overnight for 12 hrs. At 12 hrs, the reaction mixture was diluted with EtOAc and washed twice with saturated NH₄Cl (2 x 100 mL). The organic fractions were combined, dried over anhydrous Na₂SO₄, filtered, and concentrated under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 7. MS (ESI) m/z 380 [(M+H)⁺ calc'd for C₁₂H₆BrClF₃N₃O: 380].

### GENERAL SYNTHETIC SCHEMES AND PREPARATIVE EXAMPLES

The compounds of the invention may be prepared by methods known in the art of organic synthesis as set forth in part by the following general synthetic schemes and specific preparative examples. Starting materials are available commercially or may be prepared by known methods. 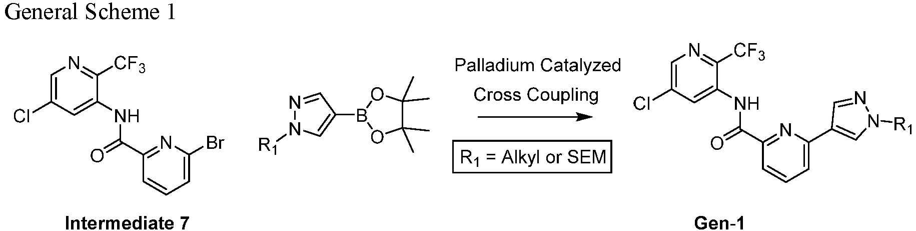 In General Scheme 1, intermediate 7 was transformed under palladium-catalyzed Suzuki coupling with either commercially available or synthetically prepared boronates 1-5 described above to afford picolinamides of the form Gen-1. Representative preparative examples are described in more detail below.

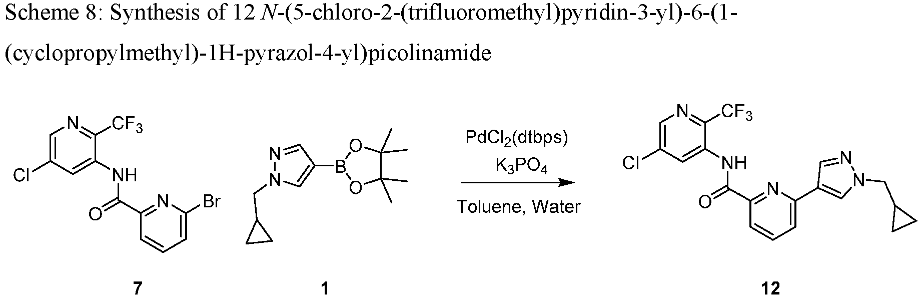

General Procedure: A 30 mL microwave vial was charged with intermediate 7 (700 mg, 1.84 mmol), 1,1'-bis(di-tert-butylphosphino)ferrocene palladium dichloride (240 mg, 0.371 mmol), K₃PO₄ (781 mg, 3.68 mmol), and intermediate 1 (548 mg, 2.21 mmol) under inert atmosphere. Toluene (5.3 mL) was added and the resulting mixture was allowed to stir at RT for 7 hrs. At 7 hrs, the reaction mixture was concentrated under reduced pressure. The crude mixture was purified by flash chromatography using a pre-packed RediSep R_{f} silica gel column on a Teledyne Isco CombiFlash R_{f} automated chromatography system using 0-100% 3:1 EtOAc/Ethanol in hexanes to afford the title compound 12. MS (ESI) m/z 422 [(M+H)⁺ calc'd for C₁₉H₁₅ClF₃N₅O: 422].

Each of the elaborated picolinamides presented in Table 1 below were prepared in accordance with the synthetic routes in General Scheme 1, using procedures analogous to those described above.

**Table 1.**

| Intermediate | Structure |
|---|---|
| 8 | 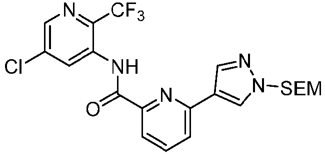 |
| 9 | 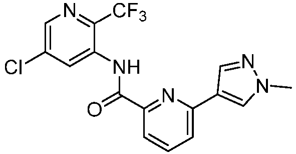 |
| 10 | 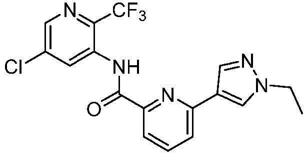 |
| 11 | 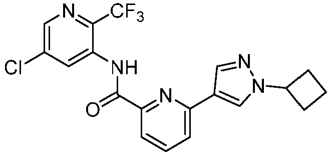 |
| 12 | 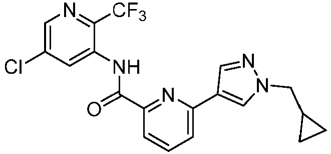 |
| 13 | 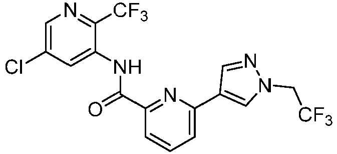 |
| 14 | 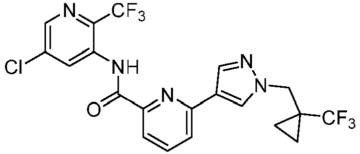 |
| 15 | 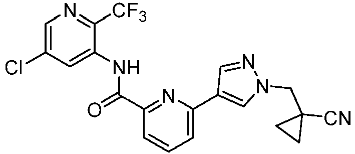 |

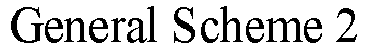

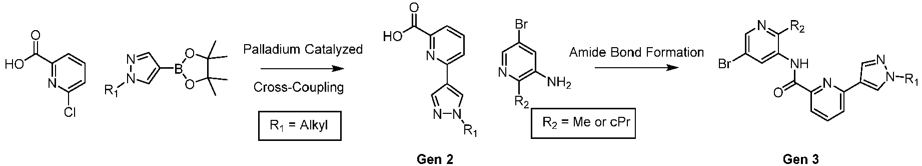

In General Scheme 2, 6-chloropicolinic acid was transformed under palladium-catalyzed Suzuki coupling with either commercially available or synthetically prepared boronates to afford picolinic acids Gen 2 which were condensed with 5-bromo-2-methylpyridin-3-amine or 5-bromo-2-cyclopropylpyridin-3-amine to afford bromo picolinamides of the form Gen 3. Representative preparative examples are described in more detail below.

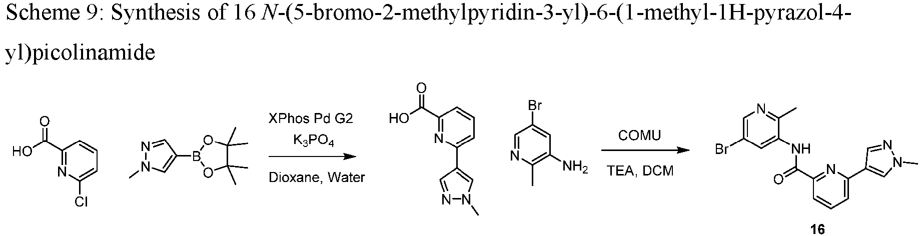

### Step 1: Preparation for 6-(1-methyl-1H-pyrazol-4-yl)picolinic acid

General Procedure: A 100 mL round bottom flask was charged with 6-chloropicolinic acid (2.7 g, 17.1 mmol), XPhos Pd G2 (0.674 g, 0.857 mmol), 1-methyl-4-(4,4,5,5-tetramethyl-1,3,2-dioxaborolan-2-yl)-1H-pyrazole (3.92 g, 18.8 mmol), dioxane (35 ml), K₃PO₄ (10.9 g, 51.4 mmol), and H₂O (10 mL). The reaction mixture was purged with nitrogen 3 times and stirred at 80 °C for 12 hrs. The reaction mixture was cooled to RT, filtered and concentrated under reduced pressure. The crude mixture was dissolved in 3:1 Chloroform/Isopropanol mixture and extracted with saturated NH₄Cl (2 x 50 mL). The combined organic phases were combined, dried over Na₂SO₄, and the solvent removed under reduced pressure to afford the title compound which was taken forward without further purification. MS (ESI) m/z 204 [(M+H)⁺ calc'd for C₁₀H₉N₃O₂: 204]

### Step 2: Preparation for N-(5-bromo-2-methylpyridin-3-yl)-6-(1-methyl-1H-pyrazol-4-yl)picolinamide

General Procedure: A 100 mL round bottom flask was charged with 6-(1-methyl-1H-pyrazol-4-yl)picolinic acid (0.5 g, 2.46 mmol), COMU (1.12 g, 2.46 mmol), DCM (12.3 mL), and TEA (1 mL, 7.38 mmol). The reaction mixture was stirred for 5 min and 5-bromo-2-methylpyridin-3-amine (0.46 g, 2.46 mmol) was added. The reaction mixture was purged with nitrogen 3 times and stirred at RT for 48 hrs. The reaction mixture was diluted with EtOAc and washed twice with saturated NH₄Cl (2 x 50 mL) and once with brine (50 mL). The organic fractions were combined, dried over anhydrous Na₂SO₄, filtered and concentrated under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound 16. MS (ESI) m/z 372 [(M+H)⁺ calc'd for C₁₆H₁₄BrN₅O: 372].

Each of the elaborated picolinamides presented in Table 2 below were prepared in accordance with the synthetic routes in General Scheme 2, using procedures analogous to those described above.

**Table 2.**

| Intermediate | Structure |
|---|---|
| 16 | 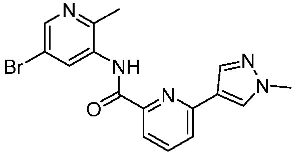 |
| 17 | 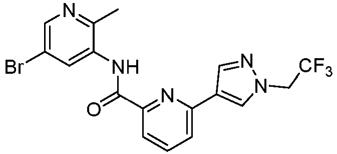 |
| 18 | 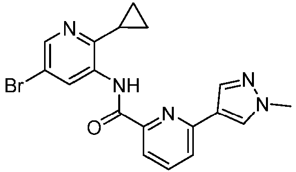 |
| 19 | 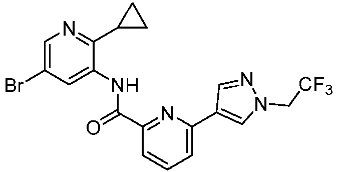 |

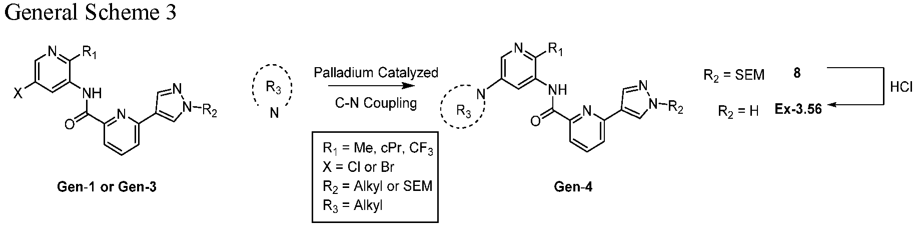

In General Scheme 3, Gen 1 or Gen 3 picolinamides were transformed under palladium-catalyzed C-N coupling conditions with either commercially available amines to afford elaborated compounds of the form Gen-4. The representative compounds are described in more detail below.

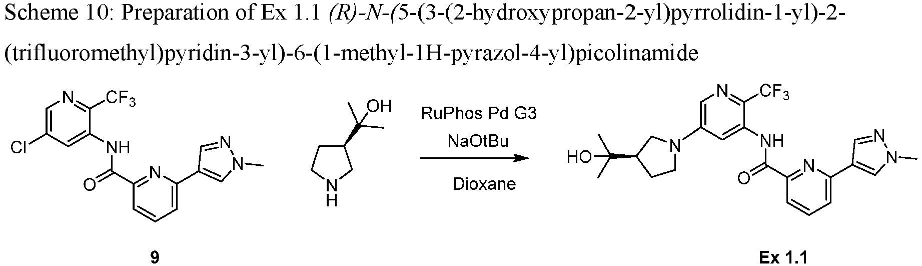

General Procedure: A 30 mL microwave vial was charged with intermediate 9 (500 mg, 1.31 mmol), (R)-2-(pyrrolidin-3-yl)propan-2-ol (250 mg, 1.94 mmol), RuPhos Pd G3 (200 mg, 0.241 mmol), sodium tert-Butoxide (2 mL, 4.0 mmol, 2M in THF) and dioxane (6 mL). The reaction mixture was purged with nitrogen three times and the reaction mixture was then allowed to stir at 80 °C for 12 hrs. The reaction mixture was then diluted with EtOAc (25 mL) and washed twice with saturated NH₄Cl (2 x 25 mL) and once with brine (25 mL). The organic fractions were combined, dried over anhydrous Na₂SO₄, filtered, and concentrated under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound Ex 1.1. ¹H NMR (499 MHz, DMSO-d6) δ 10.71 (s, 1H), 8.43 (s, 1H), 8.14 (s, 1H), 8.07 (t, J = 7.7 Hz, 1H), 7.95 (dd, J = 7.6, 3.2 Hz, 2H), 7.84 (d, J = 6.2 Hz, 2H), 4.46 (s, 1H), 3.93 (s, 3H), 3.53 (t, J = 8.7 Hz, 1H), 3.40 (t, J = 8.9 Hz, 1H), 2.36 (q, J = 8.8 Hz, 1H), 2.00 (d, J = 7.0 Hz, 1H), 1.97 - 1.88 (m, 3H), 1.17 (d, J = 10.3 Hz, 6H). MS (ESI) m/z 475 [(M+H)⁺ calc'd for C₂₃H₂₅F₃N₆O₂: 475].

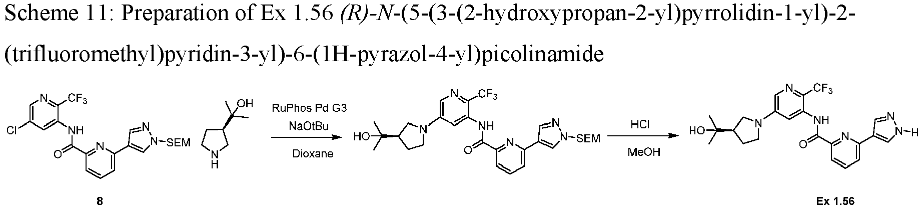

### Step 1: Preparation of (R)-N-(5-(3-(2-hydroxypropan-2-yl)pyrrolidin-1-yl)-2-(trifluoromethyl)pyridin-3-yl)-6-(1-((2-(trimethylsilyl)ethoxy)methyl)-1H-pyrazol-4-yl)picolinamide, TFA

General Procedure: A 30 mL microwave vial was charged with intermediate 8 (100 mg, 0.2 mmol), *(R)*-2-(pyrrolidin-3-yl)propan-2-ol, HCl (50 mg, 0.3 mmol), RuPhos Pd G3 (34 mg, 0.04 mmol), sodium tert-butoxide (58 mg, 0.61 mmol), and dioxane (0.7 mL). The reaction mixture was purged with nitrogen three times and stirred at 80 °C for 12 hrs. At 12 hrs, the reaction mixture was cooled to RT and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound. MS (ESI) m/z 591 [(M+H)⁺ calc'd for C₂₈H₃₇F₃N₆O₃Si: 591].

### Step 2: Preparation of (R)-N-(5-(3-(2-hydroxypropan-2-yl)pyrrolidin-1-yl)-2-(trifluoromethyl)pyridin-3-yl)-6-(1H-pyrazol-4-vl)picolinamide, TFA

General Procedure: A 30 mL microwave vial was charged with *(R)*-*N*-(5-(3-(2-hydroxypropan-2-yl)pyrrolidin-1-yl)-2-(trifluoromethyl)pyridin-3-yl)-6-(1-((2-(trimethylsilyl)ethoxy)methyl)-1H-pyrazol-4-yl)picolinamide, TFA (54 mg, 0.081 mmol), HCl (94 µl, 0.38 mmol), and methanol (0.4 mL). The reaction mixture was purged with nitrogen three times and stirred for 3 h at 50 °C. The reaction mixture was cooled to RT and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound Ex 1.56. ¹H NMR (499 MHz, DMSO-d6) δ 10.71 (s, 1H), 8.30 (s, 2H), 8.02 (t, *J =* 7.5 Hz, 1H), 7.97 (d, *J* = 7.5 Hz, 1H), 7.90 (d, *J* = 7.2 Hz, 1H), 7.85 (s, 1H), 7.79 (s, 1H), 3.50 - 3.46 (m, 2H), 3.26 (dd, *J* = 18.1, 8.9 Hz, 4H), 2.31 (d, *J =* 7.9 Hz, 1H), 1.99 - 1.84 (m, 2H), 1.12 (d, *J* = 12.8 Hz, 6H). MS (ESI) m/z 461 [(M+H)⁺ calc'd for C₂₂H₂₃F₃N₆O₂: 461].

Each of the elaborated picolinamides presented in Table 3 below were prepared in accordance with the synthetic routes in General Scheme 3 and Scheme 10 and 11, using procedures analogous to those described above.

**Table 3.**

| Ex | Structure | Name | Exact Mass [M+H]⁺ |
|---|---|---|---|
| Ex-1.1 | 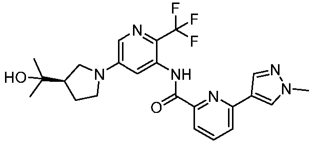 | *(R)*-*N*-(5-(3-(2-hydroxypropan-2-yl)pyrrolidin-1-yl)-2-(trifluoromethyl)pyridin-3-yl)-6-(1-methyl-1H-pyrazol-4-yl)picolinamide | Calc'd 475, found 475 |
| Ex-1.2 | 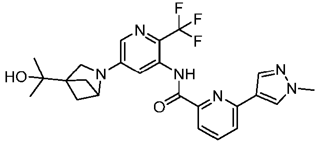 | *N*-(5-(4-(2-hydroxypropan-2-yl)-2-azabicyclo[2.1.1]hexan-2-yl)-2-(trifluoromethyl)pyridin-3-yl)-6-(1-methyl-1H-pyrazol-4-yl)picolinamide | Calc'd 487, found 487 |
| Ex-1.3 | 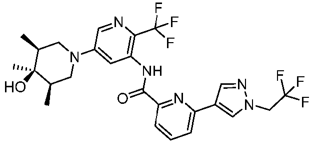 | *N*-(5-((3S,4s,5R)-4-hydroxy-3,4,5-trimethylpiperidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.4 | 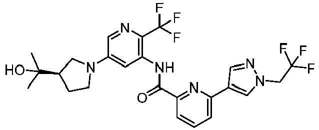 | *(R)*-*N*-(5-(3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide | Calc'd 543, found 543 |
| Ex-1.5 | 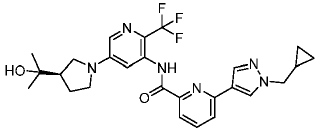 | *(R)*-6-(1-(cyclopropylmethyl)-1H-pyrazol-4-yl)-*N*-(5-(3-(2-hydroxypropan-2-yl)pyrrolidin-1-yl)-2- | Calc'd 515, found 515 |
| | | (trifluoromethyl)pyridin-3-yl)picolinamide | |
| Ex-1.6 | 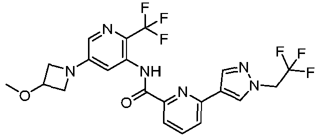 | *N*-(5-(3-methoxyazetidin-1-yl)-2-(trifluoromethyl)pyridin-3-yl)-6-(1-(2,2,2-trifluoroethyl)-1H-pyrazol-4-yl)picolinamide | Calc'd 501, found 501 |
| Ex-1.7 | 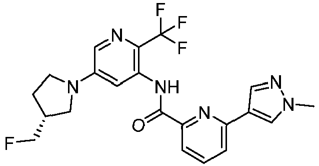 | *(R)*-*N*-(5-(3-(fluoromethyl)pyrrolidin-1-yl)-2-(trifluoromethyl)pyridin-3-yl)-6-(1-methyl-1H-pyrazol-4-yl)picolinamide | Calc'd 449, found 449 |
| Ex-1.8 | 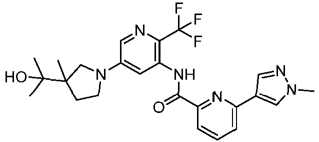 | *N*-(5-(3-(2-hydroxypropan-2-yl)-3-methylpyrrolidin-1-yl)-2-(trifluoromethyl)pyridin-3-yl)-6-(1-methyl-1H-pyrazol-4-yl)picolinamide | Calc'd 489, found 489 |
| Ex-1.9 | 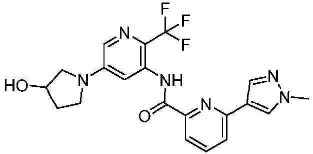 | *N*-(5-(3-hydroxypyrrolidin-1-yl)-2-(trifluoromethyl)pyridin-3-yl)-6-(1-methyl-1H-pyrazol-4-yl)picolinamide | Calc'd 433, found 433 |
| Ex-1.10 | 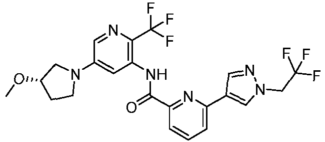 | *(S)-N*-(5-(3-methoxypyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.11 | 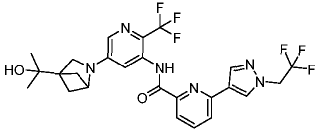 | *N*-(5-(4-(2-hydroxypropan-2-yl)-2-azabicyclo[2.1.1]hexan-2-yl)-2-(trifluoromethyl)pyridin-3-yl)-6-(1-(2,2,2-trifluoroethyl)-1H-pyrazol-4-yl)picolinamide | Calc'd *555,* found 555 |
| Ex-1.12 | 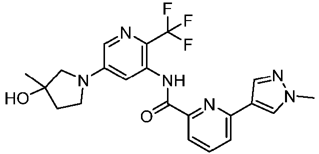 | *(R)* or *(S)*-*N*-(5-(3-hydroxy-3-methylpyrrolidin-1-yl)-2-(trifluoromethyl)pyridin-3-yl)-6-(1-methyl-1H-pyrazol-4-yl)picolinamide | Calc'd 447, found 447 |
| Ex-1.13 | 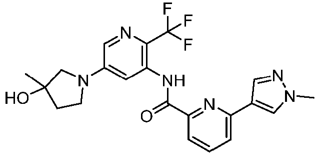 | *(R)* or *(S)*-*N*-(5-(3-hydroxy-3-methylpyrrolidin-1-yl)-2-(trifluoromethyl)pyridin-3-yl)-6-(1-methyl-1H-pyrazol-4-yl)picolinamide | Calc'd 447, found 447 |
| Ex-1.14 | 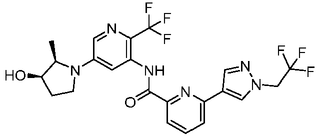 | *N*-(5-((2*R*,3*R*)-3-hydroxy-2-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.15 | 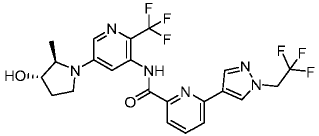 | *N*-(5-((2*R*,3*S*)-3-hydroxy-2-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.16 | 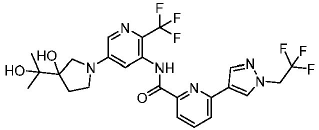 | *(R)* or *(S) N*-(5-(3-hydroxy-3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide | Calc'd 559, found 559 |
| Ex-1.17 | 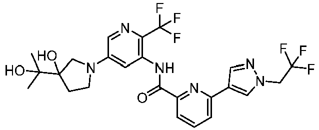 | *(R)* or *(S) N*-(5-(3-hydroxy-3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide | Calc'd 559, found 559 |
| Ex-1.18 | 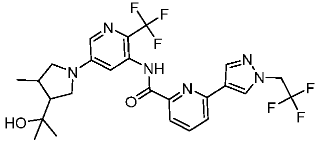 | *(R*, *S*) or *(S, R) N*-(5-(3-(2-hydroxypropan-2-yl)-4-methylpyrrolidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.19 | 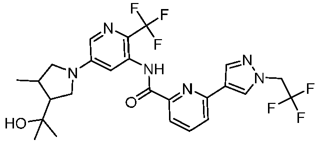 | *(R, S)* or *(S, R) N*-(5-(3-(2-hydroxypropan-2-yl)-4-methylpyrrolidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.20 | 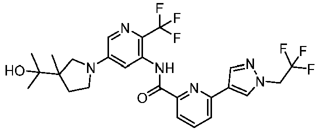 | *(R)* or *(S) N*-(5-(3-(2-hydroxypropan-2-yl)-3-methylpyrrolidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.21 | 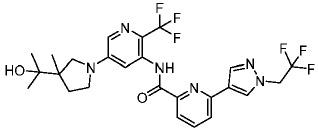 | *(R)* or *(S) N*-(5-(3-(2-hydroxypropan-2-yl)-3-methylpyrrolidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.22 | 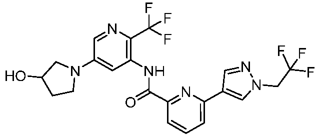 | *(R)* or *(S) N*-(5-(3-hydroxypyrrolidin-1-yl)-2-(trifluoromethyl)pyridin-3-yl)-6-(1-(2,2,2-trifluoroethyl)-1H-pyrazol-4-yl)picolinamide | Calc'd 501, found 501 |
| Ex-1.23 | 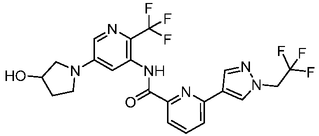 | *(R)* or *(S) N*-(5-(3-hydroxypyrrolidin-1-yl)-2-(trifluoromethyl)pyridin-3-yl)-6-(1-(2,2,2-trifluoroethyl)-1H-pyrazol-4-yl)picolinamide | Calc'd 557, found 557 |
| Ex-1.24 | 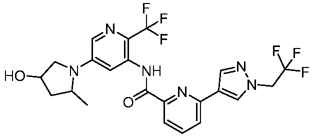 | *(R, R)* or *(S, S)* or *(S, R),* or *(R, S) N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.25 | 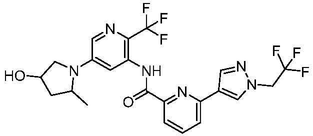 | *(R, R)* or *(S, S)* or *(S, R),* or *(R, S) N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.26 | 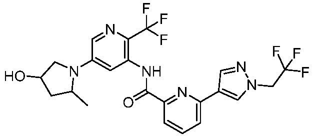 | *(R, R)* or *(S, S)* or *(S, R),* or *(R, S*) *N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.27 | 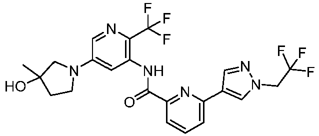 | *(R)* or *(S) N*-(5-(3-hydroxy-3-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.28 | 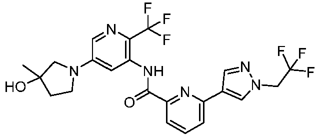 | *(R)* or *(S) N*-(5-(3-hydroxy-3-methylpyrrolidin-1-yl)-2-(trifluoromethyl)pyridin-3-yl)-6-(1-(2,2,2-trifluoroethyl)-1H-pyrazol-4-yl)picolinamide | Calc'd 515, found 515 |
| Ex-1.29 | 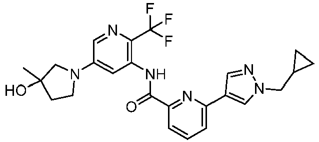 | *(R)* or *(S)* 6-(1-(cyclopropylmethyl)-1H-pyrazol-4-yl)-*N*-(5-(3-hydroxy-3-methylpyrrolidin-1-yl)-2-(trifluoromethyl)pyridin-3-yl)picolinamide | Calc'd 487, found 487 |
| Ex-1.30 | 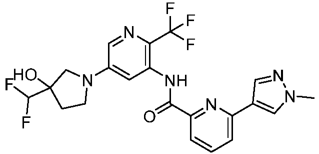 | *(R)* or *(S) N*-(5-(3-(difluoromethyl)-3-hydroxypyrrolidin-1-yl)-2-(trifluoromethyl)pyridin-3-yl)-6-(1-methyl-1H-pyrazol-4-yl)picolinamide | Calc'd 483, found 483 |
| Ex-1.31 | 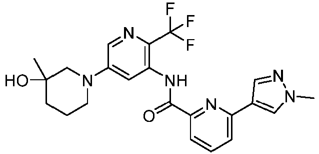 | *(R)* or *(S) N*-(5-(3-hydroxy-3-methylpiperidin-1-yl)-2-(trifluoromethyl)pyridin-3-yl)-6-(1-methyl-1H-pyrazol-4-yl)picolinamide | Calc'd 461, found 461 |
| Ex-1.32 | 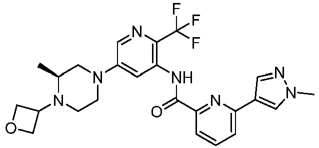 | *(S)*-6-(1-methyl-1H-pyrazol-4-yl)-*N*-(5-(3-methyl-4-(oxetan-3-yl)piperazin-1-yl)-2-(trifluoromethyl)pyridin-3-yl)picolinamide | Calc'd 502, found 502 |
| Ex-1.33 | 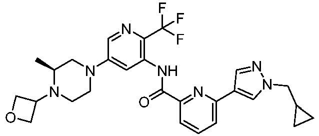 | *(S)*-6-(1-(cyclopropylmethyl)-1H-pyrazol-4-yl)-N-(5-(3-methyl-4-(oxetan-3-yl)piperazin-1-yl)-2-(trifluoromethyl)pyridin-3-yl)picolinamide | Calc'd 542, found 542 |
| Ex-1.34 | 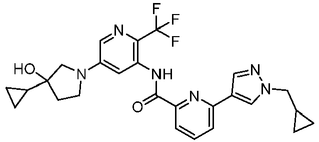 | *(R)* or *(S) N*-(5-(3-cyclopropyl-3-hydroxypyrrolidin-1-yl)-2-(trifluoromethyl)pyridin-3-yl)-6-(1-(cyclopropylmethyl)-1H-pyrazol-4-yl)picolinamide | Calc'd 513, found 513 |
| Ex-1.35 | 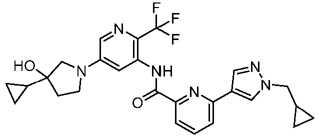 | *(R)* or *(S) N*-(5-(3-cyclopropyl-3-hydroxypyrrolidin-1-yl)-2-(trifluoromethyl)pyridin-3-yl)-6-(1-(cyclopropylmethyl)-1H-pyrazol-4-yl)picolinamide | Calc'd 513, found 513 |
| Ex-1.36 | 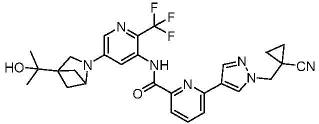 | 6-(1-((1-cyanocyclopropyl)methyl)-1H-pyrazol-4-yl)-*N*-(5-(4-(2-hydroxypropan-2-yl)-2-azabicyclo[2.1.1]hexan-2-yl)-2(trifluoromethyl)pyridin-3-yl)picolinamide | Calc'd 552, found 552 |
| Ex-1.37 | 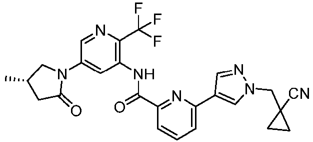 | *(S)*-6-(1-((1-cyanocyclopropyl)methyl)-1H-pyrazol-4-yl)-*N*-(5-(4-methyl-2-oxopyrrolidin-1-yl)-2-(trifluoromethyl)pyridin-3-yl)picolinamide | Calc'd 510, found 510 |
| Ex-1.38 | 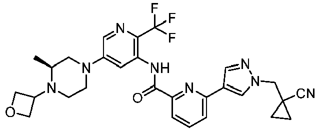 | *(S)*-6-(1-((1-cyanocyclopropyl)methyl)-1H-pyrazol-4-yl)-*N*-(5-(3-methyl-4-(oxetan-3-yl)piperazin-1-yl)-2-(trifluoromethyl)pyridin-3-yl)picolinamide | Calc'd 567, found 567 |
| Ex-1.39 | 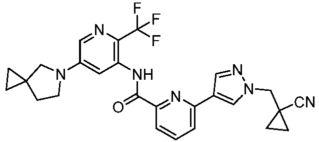 | *N*-(5-(5-azaspiro[2.4]heptan-5-yl)-2-(trifluoromethyl)pyridin-3-yl)-6-(1-((1-cyanocyclopropyl)methyl)-1H-pyrazol-4-yl)picolinamide | Calc'd 508, found 508 |
| Ex-1.40 | 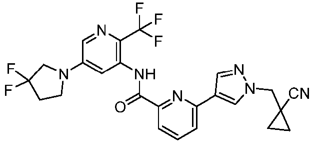 | 6-(1-((1-cyanocyclopropyl)methyl)-1H-pyrazol-4-yl)-*N*-(5-(3,3-difluoropyrrolidin-1-yl)-2-(trifluoromethyl)pyridin-3-yl)picolinamide | Calc'd 518, found 518 |
| Ex-1.41 | 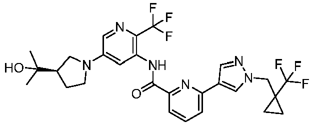 | *(R)*-*N*-(5-(3-(2-hydroxypropan-2-yl)pyrrolidin-1-yl)-2-(trifluoromethyl)pyridin-3-yl)-6-(1-((1-(trifluoromethyl)cyclopropyl)m ethyl)-1H-pyrazol-4-yl)picolinamide | Calc'd 583, found 583 |
| Ex-1.42 | 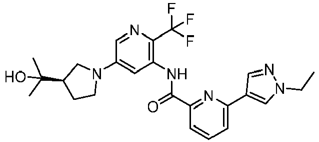 | *(R)*-6-(1-ethyl-1H-pyrazol-4-yl)-*N*-(5-(3-(2-hydroxypropan-2-yl)pyrrolidin-1-yl)-2-(trifluoromethyl)pyridin-3-yl)picolinamide | Calc'd 489, found 489 |
| Ex-1.43 | 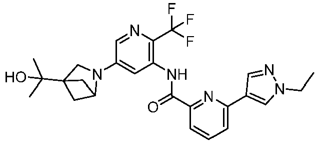 | 6-(1-ethyl-1H-pyrazol-4-yl)-*N-*(5-(4-(2-hydroxypropan-2-yl)-2-azabicyclo[2.1.1]hexan-2-yl)-2-(trifluoromethyl)pyridin-3-yl)picolinamide | Calc'd 501, found 501 |
| Ex-1.44 | 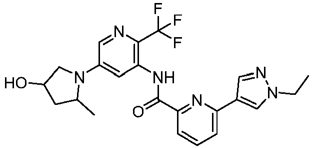 | *(R, R)* or *(S, S)* or *(S, R),* or *(R, S)* 6-(1-ethyl-1H-pyrazol-4-yl)-*N*-(5-(4-hydroxy-2-methylpyrrolidin-1-yl)-2-(trifluoromethyl)pyridin-3-yl)picolinamide | Calc'd 461, found 461 |
| Ex-1.45 | 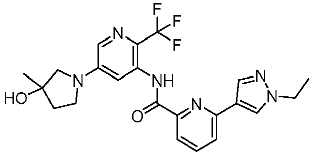 | *(R)* or *(S)* 6-(1-ethyl-1H-pyrazol-4-yl)-*N*-(5-(3-hydroxy-3-methylpyrrolidin-1-yl)-2-(trifluoromethyl)pyridin-3-yl)picolinamide | Calc'd 461, found 461 |
| Ex-1.46 | 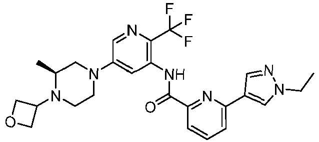 | *(S)*-6-(1-ethyl-1H-pyrazol-4-yl)-*N*-(5-(3-methyl-4-(oxetan-3-yl)piperazin-1-yl)-2-(trifluoromethyl)pyridin-3-yl)picolinamide | Calc'd 516, found 516 |
| Ex-1.47 | 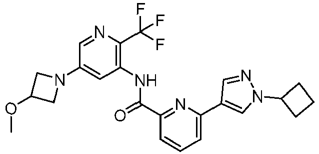 | 6-(1-cyclobutyl-1H-pyrazol-4-yl)-*N*-(5-(3-methoxyazetidin-1-yl)-2-(trifluoromethyl)pyridin-3-yl)picolinamide | Calc'd 473, found 473 |
| Ex-1.48 | 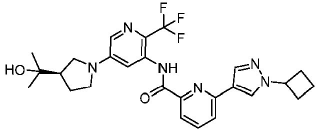 | *(R)*-6-(1-cyclobutyl-1H-pyrazol-4-yl)-*N*-(5-(3-(2-hydroxypropan-2-yl)pyrrolidin-1-yl)-2-(trifluoromethyl)pyridin-3-yl)picolinamide | Calc'd 515, found 515 |
| Ex-1.49 | 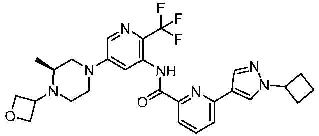 | *(S)*-6-(1-cyclobutyl-1H-pyrazol-4-yl)-*N*-(5-(3-methyl-4-(oxetan-3-yl)piperazin-1-yl)-2-(trifluoromethyl)pyridin-3-yl)picolinamide | Calc'd 542, found 542 |
| Ex-1.50 | 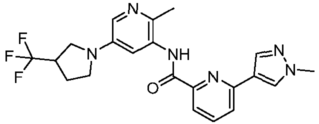 | 6-(1-methyl-1H-pyrazol-4-yl)-*N*-(2-methyl-5-(3-(trifluoromethyl)pyrrolidin-1-yl)pyridin-3-yl)picolinamide | Calc'd 431, found 431 |
| Ex-1.51 | 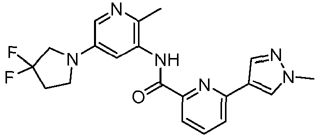 | *N*-(5-(3,3-difluoropyrrolidin-1-yl)-2-methylpyridin-3-yl)-6-(1-methyl-1H-pyrazol-4-yl)picolinamide | Calc'd 399, found 399 |
| Ex-1.52 | 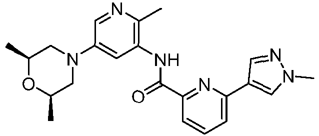 | *N*-(5-((2*S*,6*R*)-2,6-dimethylmorpholino)-2-methylpyridin-3-yl)-6-(1-methyl-1H-pyrazol-4-yl)picolinamide | Calc'd 407, found 407 |
| Ex-1.53 | 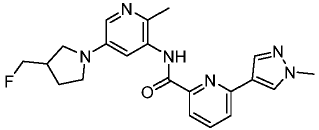 | *N*-(5-(3-(fluoromethyl)pyrrolidin-1-yl)-2-methylpyridin-3-yl)-6-(1-methyl-1H-pyrazol-4-yl)picolinamide | Calc'd 395, found 395 |
| Ex-1.54 | 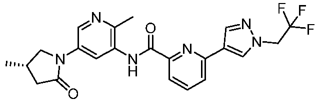 | *(S)-N*-(2-methyl-5-(4-methyl-2-oxopyrrolidin-1-yl)pyridin-3-yl)-6-(1-(2,2,2-trifluoroethyl)-1H-pyrazol-4-yl)picolinamide | Calc'd 459, found 459 |
| Ex-1.55 | 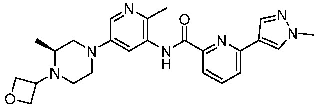 | *(S)*-6-(1-methyl-1H-pyrazol-4-yl)-*N*-(2-methyl-5-(3-methyl-4-(oxetan-3-yl)piperazin-1-yl)pyridin-3-yl)picolinamide | Calc'd 448, found 448 |
| Ex-1.56 | 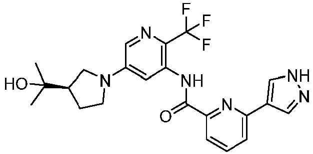 | *(R)*-*N*-(5-(3-(2-hydroxypropan-2-yl)pyrrolidin-1-yl)-2-(trifluoromethyl)pyridin-3-yl)-6-(1H-pyrazol-4-yl)picolinamide | Calc'd 461, found 461 |
| Ex-1.57 | 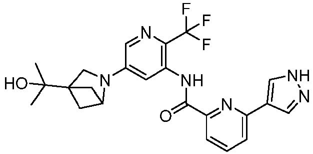 | *N*-(5-(4-(2-hydroxypropan-2-yl)-2-azabicyclo[2.1.1]hexan-2-yl)-2-(trifluoromethyl)pyridin-3-yl)-6-(1H-pyrazol-4-yl)picolinamide | Calc'd 473, found 473 |
| Ex-1.58 | 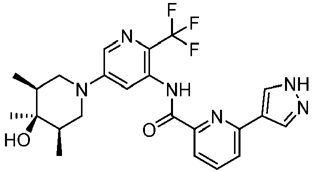 | *N*-(5-((3*S*,4*s*,5*R*)-4-hydroxy-3,4,5-trimethylpiperidin-1-yl)-2-(trifluoromethyl)pyridin-3-yl)-6-(1H-pyrazol-4-yl)picolinamide | Calc'd 475, found 475 |
| Ex-1.59 | 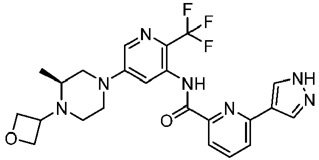 | *(S)-N*-(5-(3-methyl-4-(oxetan-3-yl)piperazin-1-yl)-2-(trifluoromethyl)pyridin-3-yl)-6-(1H-pyrazol-4-yl)picolinamide | Calc'd 488, found 488 |
| Ex-1.60 | 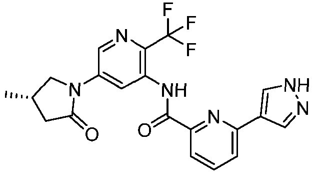 | *(S)*-*N*-(5-(4-methyl-2-oxopyrrolidin-1-yl)-2-(trifluoromethyl)pyridin-3-yl)-6-(1H-pyrazol-4-yl)picolinamide | Calc'd 431, found 431 |
| Ex-1.61 | 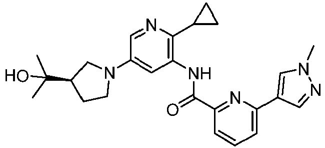 | *(R)*-*N*-(2-cyclopropyl-5-(3-(2-hydroxypropan-2-yl)pyrrolidin-1-yl)pyridin-3-yl)-6-(1-methyl-1H-pyrazol-4-yl)picolinamide | Calc'd 447 Found 447 |
| Ex-1.62 | 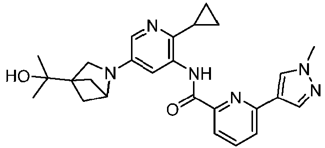 | *N*-(2-cyclopropyl-5-(4-(2-hydroxypropan-2-yl)-2-azabicyclo[2.1.1]hexan-2-yl)pyridin-3-yl)-6-(1-methyl-1H-pyrazol-4-yl)picolinamide | Calc'd 459, found 459 |
| Ex-1.63 | 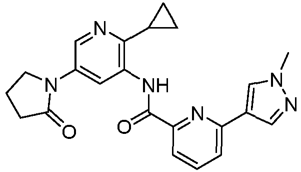 | *N*-(2-cyclopropyl-5-(2-oxopyrrolidin-1-yl)pyridin-3-yl)-6-(1-methyl-1H-pyrazol-4-yl)picolinamide | Calc'd 403, found 403 |
| Ex-1.64 | 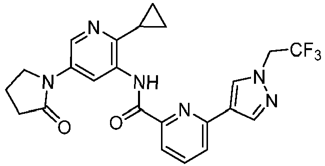 | *N*-(2-cyclopropyl-5-(2-oxopyrrolidin-1-yl)pyridin-3-yl)-6-(1-(2,2,2-trifluoroethyl)-1H-pyrazol-4-yl)picolinamide | calc'd 471, found 471 |
| Ex-1.65 | 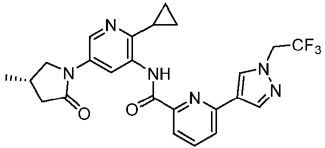 | *(S)*-*N*-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide | Calc'd 485, found 485 |
| Ex-1.66 | 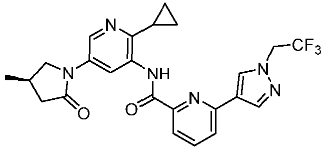 | *(R)-N*-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide | Calc'd 485, found 485 |
| Ex-1.67 | 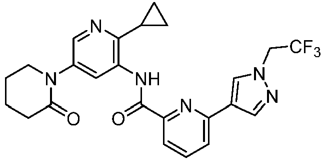 | *N*-(2-cyclopropyl-5-(2-oxopiperidin-1-yl)pyridin-3-yl)-6-(1-(2,2,2-trifluoroethyl)-1H-pyrazol-4-yl)picolinamide | Calc'd 485, found 485 |
| Ex-1.68 | 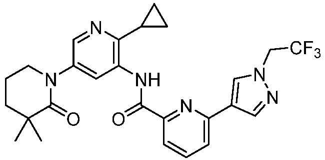 | *N*-(2-cyclopropyl-5-(3,3-dimethyl-2-oxopiperidin-1-yl)pyridin-3-yl)-6-(1-(2,2,2-trifluoroethyl)-1H-pyrazol-4-yl)picolinamide | Calc'd 513, found 513 |
| Ex-1.69 | 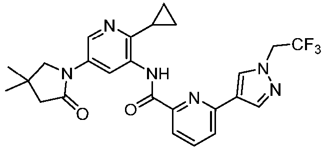 | *N*-(2-cyclopropyl-5-(4,4-dimethyl-2-oxopyrrolidin-1-yl)pyridin-3-yl)-6-(1-(2,2,2-trifluoroethyl)-1H-pyrazol-4-yl)picolinamide | Calc'd 499, found 499 |
| Ex-1.70 | 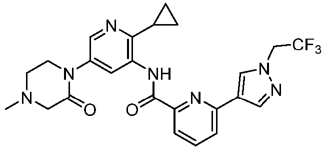 | *N*-(2-cyclopropyl-5-(4-methyl-2-oxopiperazin-1-yl)pyridin-3-yl)-6-(1-(2,2,2-trifluoroethyl)-1H-pyrazol-4-yl)picolinamide | Calc'd 500, found 500 |
| Ex-1.71 | 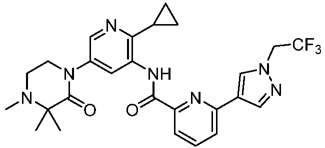 | *N*-(2-cyclopropyl-5-(3,3,4-trimethyl-2-oxopiperazin-1-yl)pyridin-3-yl)-6-(1-(2,2,2-trifluoroethyl)-1H-pyrazol-4-yl)picolinamide | Calc'd 528, found 528 |
| Ex-1.72 | 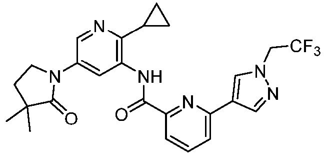 | *N*-(2-cyclopropyl-5-(3,3-dimethyl-2-oxopyrrolidin-1-yl)pyridin-3-yl)-6-(1-(2,2,2-trifluoroethyl)-1H-pyrazol-4-yl)picolinamide | Calc'd 499, found 499 |
| Ex-1.73 | 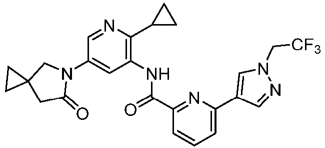 | *N*-(2-cyclopropyl-5-(6-oxo-5-azaspiro[2.4]heptan-5-yl)pyridin-3-yl)-6-(1-(2,2,2-trifluoroethyl)-1H-pyrazol-4-yl)picolinamide | Calc'd 497, found 497 |
| Ex-1.74 | 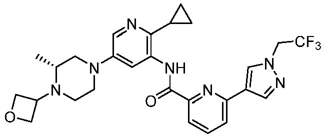 | *(R)-N*-(2-cyclopropyl-5-(3-methyl-4-(oxetan-3-yl)piperazin-1-yl)pyridin-3-yl)-6-(1-(2,2,2-trifluoroethyl)-1H-pyrazol-4-yl)picolinamide | Calc'd 542, found 542 |
| Ex-1.75 | 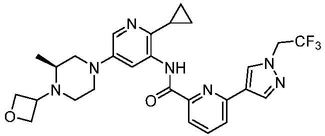 | *(S)-N-*(2-cyclopropyl-5-(3-methyl-4-(oxetan-3-yl)piperazin-1-yl)pyridin-3-yl)-6-(1-(2,2,2-trifluoroethyl)-1H-pyrazol-4-yl)picolinamide | Calc'd 542, found 542 |
| Ex-1.76 | 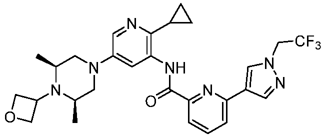 | *N*-(2-cyclopropyl-5-((3S,5R)-3,5-dimethyl-4-(oxetan-3-yl)piperazin-1-yl)pyridin-3-yl)-6-(1-(2,2,2-trifluoroethyl)-1H-pyrazol-4-yl)picolinamide | Calc'd 556, found 556 |
| Ex-1.77 | 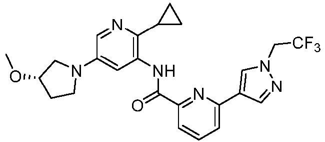 | *(S)*-N-(2-cyclopropyl-5-(3-methoxypyrrolidin-1-yl)pyridin-3-yl)-6-(1-(2,2,2-trifluoroethyl)-1H-pyrazol-4-yl)picolinamide | Calc'd 487, found 487 |
| Ex-1.78 | 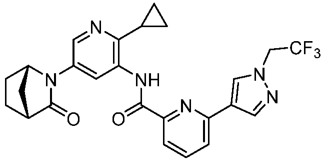 | *N*-(2-cyclopropyl-5-((1S,4R)-3-oxo-2-azabicyclo[2.2.1]heptan-2-yl)pyridin-3-yl)-6-(1-(2,2,2-trifluoroethyl)-1H-pyrazol-4-yl)picolinamide | Calc'd 497, found 497 |
| Ex-1.79 | 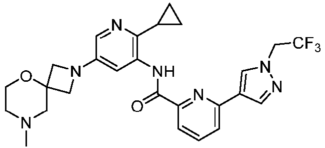 | *N*-(2-cyclopropyl-5-(8-methyl-5-oxa-2,8-diazaspiro[3.5]nonan-2-yl)pyridin-3-yl)-6-(1-(2,2,2-trifluoroethyl)-1H-pyrazol-4-yl)picolinamide | Calc'd 528, found 528 |
| Ex-1.80 | 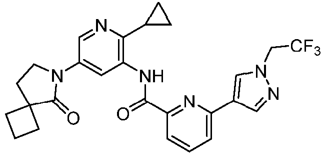 | *N*-(2-cyclopropyl-5-(5-oxo-6-azaspiro[3.4]octan-6-yl)pyridin-3-yl)-6-(1-(2,2,2-trifluoroethyl)-1H-pyrazol-4-yl)picolinamide | Calc'd 511, found 511 |
| Ex-1.81 | 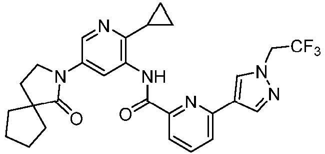 | *N*-(2-cyclopropyl-5-(1-oxo-2-azaspiro[4.4]nonan-2-yl)pyridin-3-yl)-6-(1-(2,2,2-trifluoroethyl)-1H-pyrazol-4-yl)picolinamide | Calc'd 525, found 525 |
| Ex-1.82 | 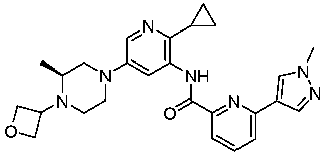 | *(S)-N*-(2-cyclopropyl-5-(3-methyl-4-(oxetan-3-yl)piperazin-1-yl)pyridin-3-yl)-6-(1-methyl-1H-pyrazol-4-yl)picolinamide | Calc'd 474, found 474 |
| Ex-1.83 | 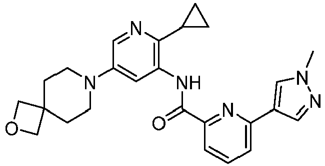 | *N*-(2-cyclopropyl-5-(2-oxa-7-azaspiro[3.5]nonan-7-yl)pyridin-3-yl)-6-(1-methyl-1H-pyrazol-4-yl)picolinamide | Calc'd 445, found 445 |

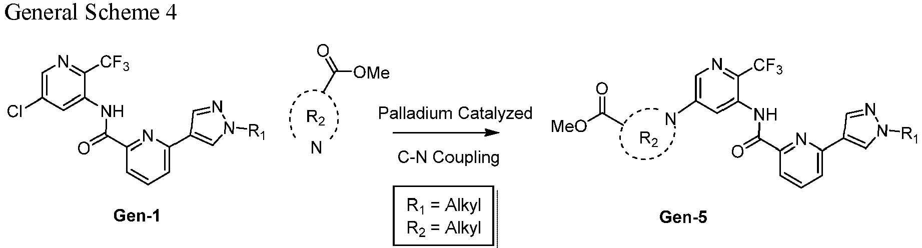

In General Scheme 4, Gen 1 picolinamides were transformed under palladium-catalyzed C-N coupling conditions with commercially available esters to afford picolinamides of the form Gen 5. Representative preparative examples are described in more detail below.

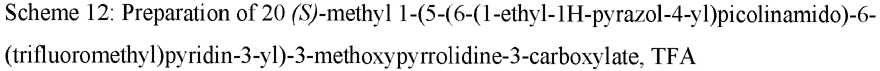

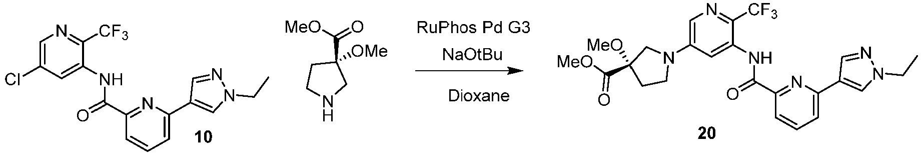

General Procedure: A 30 mL microwave vial was charged with intermediate 10 (100 mg, 0.25 mmol), *(S)*-methyl 3-methoxypyrrolidine-3-carboxylate 2,3,4-trihydroxypentanedioate (129 mg, 0.38 mmol), RuPhos Pd G3 (21 mg, 0.03 mmol), cesium carbonate (250 mg, 0.77 mmol), and dioxane (0.85 mL). The reaction vessel was purged with nitrogen and stirred at 80 °C for 12 hrs. The reaction mixture was then cooled to RT and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound 20. MS (ESI) m/z 519 [(M+H)⁺calc'd for C₂₄H₂sF₃N₆O₄: 519].

Each of the elaborated picolinamides presented in Table 4 below were prepared in accordance with the synthetic routes in General Scheme 4, using procedures analogous to those described above.

**Table 4.**

| Intermediate | Structure |
|---|---|
| 20 | 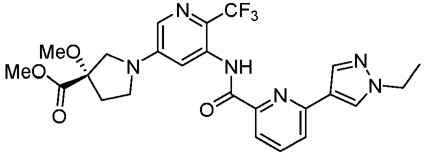 |
| 21 | 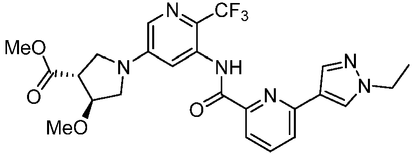 |
| 22 | 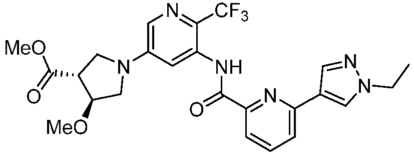 |
| 23 | 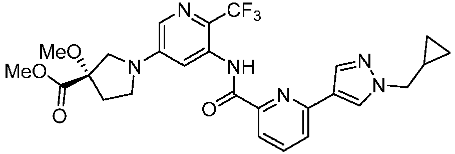 |
| 24 | 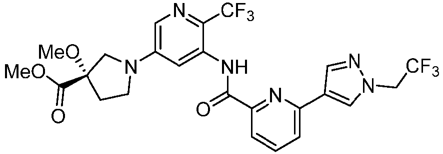 |
| 25 | 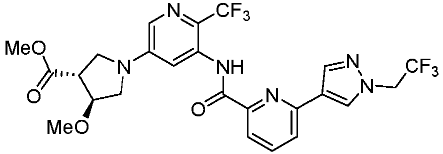 |

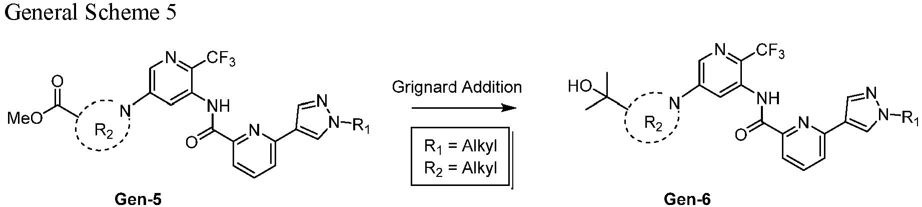

In General Scheme 5, Gen 5 picolinamides were transformed using commerically available Grignard reagents to form elaborated picolinamide compounds of the form Gen 6. The representative compounds are described in more detail below.

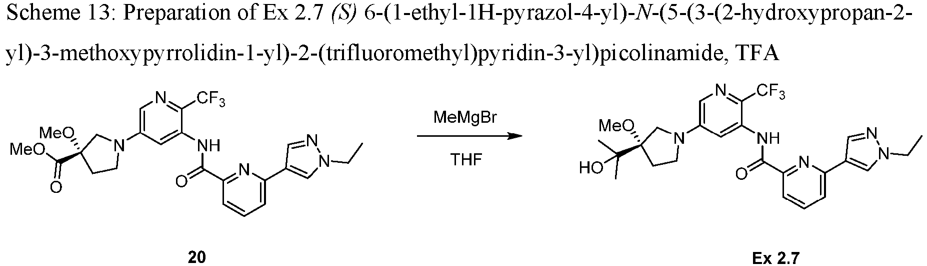

General Procedure: A 30 mL microwave vial was charged with intermediate 20 (11 mg, 0.02 mmol) and THF (0.2 mL). The reaction vessel was cooled to 0 °C and purged with nitrogen. Methylmagnesium bromide (0.02 mL, 0.06 mmol, 3 M in THF) was added dropwise and the reaction mixture was stirred to RT for 2 hrs. At 2 hrs, the reaction mixture was quenched with EtOAc and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound Ex 2.7. ¹H NMR (499 MHz, DMSO-d6) δ 10.70 (s, 1H), 8.45 (s, 1H), 8.12 (s, 1H), 8.03 (t, *J=* 7.6 Hz, 1H), 7.92 (t, *J* = 8.5 Hz, 2H), 7.85 (d, *J* = 16.9 Hz, 2H), 4.18 (q, *J = 6.9* Hz, 2H), 3.77 (s, 4H), 3.67 (d, *J* = 11.4 Hz, 2H), 3.50 (dd, *J =* 21.7, 9.6 Hz, 2H), 3.42 (d, *J =* 11.2 Hz, 1H), 2.04 (dd, *J=* 11.9, 7.1 Hz, 1H), 1.41 (t, *J=* 7.1 Hz, 3H), 1.15 (d, *J* = 12.7 Hz, 6H). MS (ESI) m/z 519 [(M+H)⁺ calc'd for C₂₅H₂₉F₃N₆O₃: 519].

Each of the elaborated picolinamides presented in Table 5 below were prepared in accordance with the synthetic routes in General Scheme 5 and Scheme 13, using procedures analogous to those described above.

**Table 5**

| Ex | Structure | Name | Exact Mass [M+H]⁺ |
|---|---|---|---|
| Ex-2.1 | 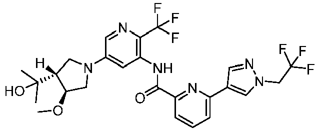 | *N*-(5-((3R,4S)-3-(2-hydroxypropan-2-yl)-4-methoxypyrrolidin-1-yl)-2-(trifluoromethyl)pyridin-3-yl)-6-(1-(2,2,2-trifluoroethyl)-1H-pyrazol-4-yl)picolinamide | Calc'd 573, found 573 |
| **Ex-2.2** | 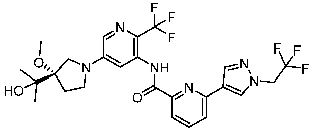 | *(S)-N-*(5-(3-(2-hydroxypropan-2-yl)-3-methoxypyrrolidin-1-yl)-2-(trifluoromethyl)pyridin-3-yl)-6-(1-(2,2,2-trifluoroethyl)-1H-pyrazol-4-yl)picolinamide | Calc'd 573, found 573 |
| **Ex-2.3** | 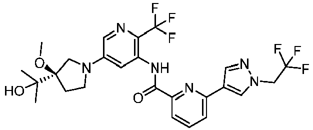 | *(R)*-*N*-(5-(3-(2-hydroxypropan-2-yl)-3-methoxypyrrolidin-1-yl)-2-(trifluoromethyl)pyridin-3-yl)-6-(1-(2,2,2-trifluoroethyl)-1H-pyrazol-4-yl)picolinamide | Calc'd 573, found 573 |
| Ex-2.4 | 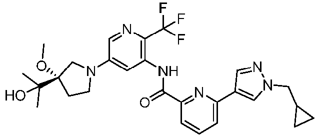 | *(S)*-6-(1-(cyclopropylmethyl)-1H-pyrazol-4-yl)-*N*-(5-(3-(2-hydroxypropan-2-yl)-3-methoxypyrrolidin-1-yl)-2-(trifluoromethyl)pyridin-3-yl)picolinamide | Calc'd 545, found 545 |
| Ex-2.5 | 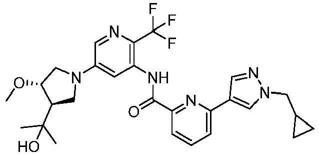 | 6-(1-(cyclopropylmethyl)-1H-pyrazol-4-yl)-*N*-(5-((3R,4S)-3-(2-hydroxypropan-2-yl)-4-methoxypyrrolidin-1-yl)-2-(trifluoromethyl)pyridin-3-yl)picolinamide | Calc'd 545, found 545 |
| Ex-2.6 | 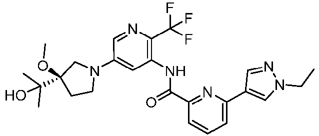 | *(R)*-6-(1-ethyl-1H-pyrazol-4-yl)-*N*-(5-(3-(2-hydroxypropan-2-yl)-3-methoxypyrrolidin-1-yl)-2-(trifluoromethyl)pyridin-3-yl)picolinamide | Calc'd 519, found 519 |
| Ex-2.7 | 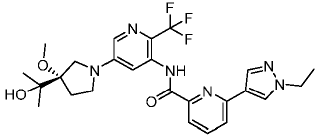 | *(S)*-6-(1-ethyl-1H-pyrazol-4-yl)-*N*-(5-(3-(2-hydroxypropan-2-yl)-3-methoxypyrrolidin-1-yl)-2-(trifluoromethyl)pyridin-3-yl)picolinamide | Calc'd 519, found 519 |
| Ex-2.8 | 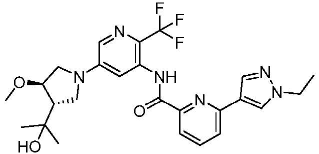 | 6-(1-ethyl-1H-pyrazol-4-yl)-*N-*(5-((3S,4R)-3-(2-hydroxypropan-2-yl)-4-methoxypyrrolidin-1-yl)-2-(trifluoromethyl)pyridin-3-yl)picolinamide | Calc'd 519, found 519 |
| Ex-2.9 | 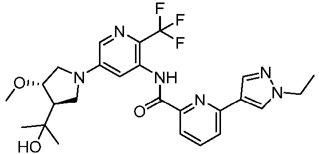 | 6-(1-ethyl-1H-pyrazol-4-yl)-*N-*(5-((3R,4S)-3-(2-hydroxypropan-2-yl)-4-methoxypyrrolidin-1-yl)-2-(trifluoromethyl)pyridin-3-yl)picolinamide | Calc'd 519, found 519 |

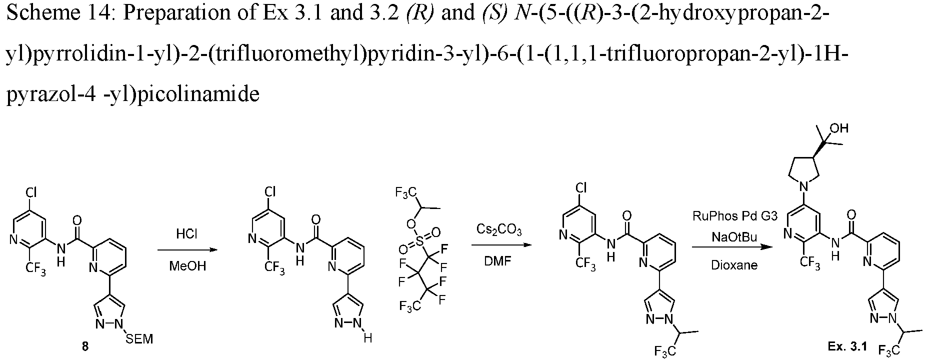

### Step 1: Preparation of N-(5-chloro-2-(trifluoromethyl)pyridin-3-yl)-6-(1H-pyrazol-4-yl)picolinamide

General Procedure: A 30 mL microwave vial was charged with intermediate 8 (245 mg, 0.49 mmol), HCl (1.2 mL, 4.9 mmol, 4N in Dioxane), and methanol (1 mL). The reaction mixture was purged with nitrogen and stirred at 50 °C for 3 h. The reaction mixture was cooled to RT and concentrated under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound. MS (ESI) m/z 368 [(M+H)⁺ calc'd for C₁₅H₉ClF₃N₅O: 368]

### Step 2: Preparation of N-(5-chloro-2-(trifluoromethyl)pyridin-3-yl)-6-(1-(1,1,1-trifluoropropan-2-yl)-1H-pyrazol-4-yl)picolinamide

General Procedure: A 30 mL microwave vial was charged with *N*-(5-chloro-2-(trifluoromethyl)pyridin-3-yl)-6-(1H-pyrazol-4-yl)picolinamide (100 mg, 0.27 mmol), Cs₂CO₃ (270 mg, 0.83 mmol), 1,1,1-trifluoropropan-2-yl-1,1,2,2,3,3,4,4,4-nonafluorobutane-1-sulfonate (215 mg, 0.54 mmol), and DMF (0.9 mL). The reaction mixture was purged with nitrogen and stirred at RT for 12 hrs. At 12 hrs, the reaction mixture was concentrated under reduced pressure and was purified by flash chromatography using pre-packed RediSep R_{f} silica gel columns on a Teledyne Isco CombiFlash R_{f} automated chromatography system using 0-100% EtOAc/hexanes to afford the title compound. MS (ESI) m/z 464 [(M+H)⁺ calc'd for C₁₈H₁₂ClF₆N₅O: 464]

### Step 3: Preparation of (R) and (S) N-(5-((R)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4 -yl)picolinamide

General Procedure: A 30 mL microwave vial was charged with N-(5-chloro-2-(trifluoromethyl)pyridin-3-yl)-6-(1-(1,1,1-trifluoropropan-2-yl)-1H-pyrazol-4-yl)picolinamide (95 mg, 0.21 mmol), (R)-2-(pyrrolidin-3-yl)propan-2-ol hydrochloride (51 mg, 0.31 mmol), RuPhos Pd G3 (34 mg, 0.04 mmol), Cs₂CO₃ (200 mg, 0.62 mmol), dioxane (1 mL) and the reaction mixture was stirred at 80 °C for 12 hrs. At 12 h, the reaction mixture was cooled to RT and concentrated under reduced pressure. The crude product was diluted in 4 mL of DMSO, filtered and purified by mass-directed reverse phase chromatography eluting with MeCN/H₂O with 0.1% TFA with a linear gradient to afford the title compound as a racemate. The racemic material could be resolved to its component enantiomers by chiral preparative SFC (Column & dimensions: AD-H, 21 mm x 250 mm; Mobile phase A: CO₂; Mobile phase B: MeOH with 0.25% DMEA) to afford title compound Ex 3.1 (tR = 3.74-5.14 min) and Ex 3.2 (tR = 5.36-6.96 min). ¹H NMR (499 MHz, DMSO-d6) δ 10.67 (s, 1H), 8.64 (s, 1H), 8.27 (s, 1H), 8.07 (t, *J =* 7.4 Hz, 1H), 8.00 (d, *J =* 7.5 Hz, 1H), 7.95 (d, *J =* 7.2 Hz, 1H), 7.80 (d, *J =* 13.3 Hz, 2H), 5.53 - 5.44 (m, 1H), 4.42 (s, 1H), 3.48 (s, 1H), 3.35 (d, *J* = 8.3 Hz, 1H), 3.25 (s, 1H), 2.32 (d, *J* = 7.9 Hz, 1H), 2.00 - 1.83 (m, 3H), 1.71 (d, *J =* 6.5 Hz, 3H), 1.13 (d, *J* = 12.4 Hz, 6H). MS (ESI) m/z 557 [(M+H)⁺ calc'd for C₂₅H₂₆F₆N₆O₂: 557]. ¹H NMR (499 MHz, DMSO-*d*6) δ 10.68 (s, 1H), 8.64 (s, 1H), 8.27 (s, 1H), 8.08 (t, J = 7.5 Hz, 1H), 8.00 (d, J = 7.6 Hz, 1H), 7.96 (d, J = 7.3 Hz, 1H), 7.80 (d, J = 17.4 Hz, 2H), 5.49 (d, J = 6.8 Hz, 1H), 4.42 (s, 1H), 3.48 (d, J = 7.8 Hz, 1H), 3.35 (d, J = 8.7 Hz, 2H), 2.37 - 2.28 (m, 1H), 1.91 (dd, J = 29.4, 19.5 Hz, 3H), 1.71 (d, J = 6.6 Hz, 3H), 1.13 (d, J = 12.4 Hz, 6H). MS (ESI) m/z 557 [(M+H)⁺ calc'd for C₂₅H₂₆F₆N₆O₂: 557].

The elaborated picolinamides presented in Table 6 below were prepared in accordance with the synthetic routes in Scheme 14, using procedures described above.

**Table 6.**

| Ex | Structure | Name | Exact Mass[M+H]⁺ |
|---|---|---|---|
| Ex-3.1 | 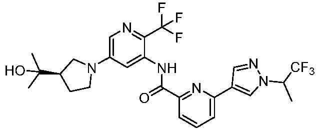 | *(R)* or *(S) N*-(5-((*R*)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4 -yl)picolinamide | Calc'd 557, found 557 |
| Ex-3.2 | 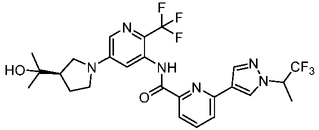 | *(R)* or *(S) N*-(5-((*R*)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4 -yl)picolinamide | Calc'd 557, found 557 |

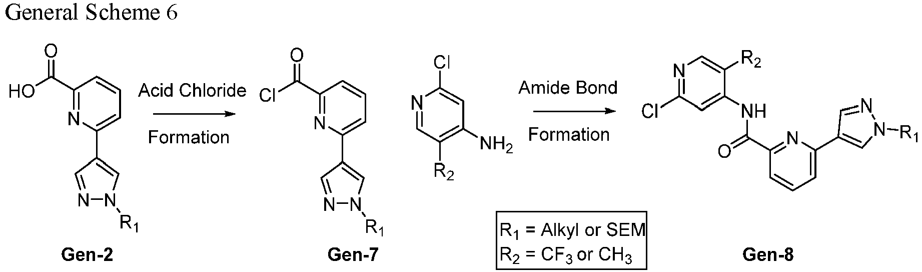

In General Scheme 6, Gen 2 picolinic acids were transformed to picolinoyl chlorides Gen 7 using oxalyl chloride which were condensed with commercially available pyridin-4-amines to afford picolinamides of the form Gen 8. Representative preparative examples are described in more detail below.

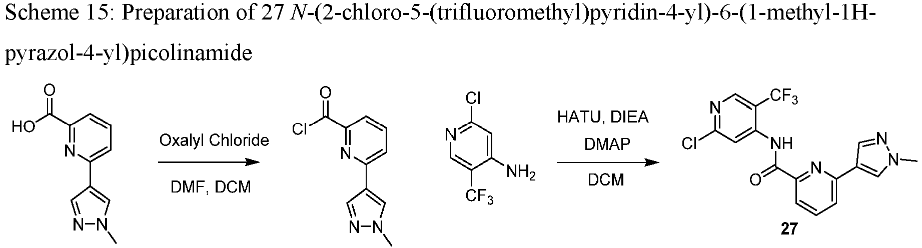

### Step 1: Preparation of 6-(1-methyl-1H-pyrazol-4-yl)picolinoyl chloride

General Procedure: A 30 mL microwave vial was charged with 6-(1-methyl-1H-pyrazol-4-yl)picolinic acid (801 mg, 3.9 mmol), DCM (8 mL), DMF (0.04 mL, 0.4 mmol), and oxalyl chloride (0.7 mL, 8.0 mmol). The vial was purged with nitrogen and stirred at RT for 3 hrs. At 3 hrs, the reaction mixture was concentrated and dried over high vacuum overnight to afford the title compound. MS (ESI) m/z 222 [(M+H)⁺ calc'd for C₁₀H₈ClN₃O: 222]

### Step 2: Preparation of N-(2-chloro-5-(trifluoromethyl)pyridin-4-yl)-6-(1-methyl-1H-pyrazol-4-yl)picolinamide

General Procedure: A 30 mL microwave vial was charged with 2-chloro-5-(trifluoromethyl)pyridin-4-amine (2 g, 10.0 mmol), 6-(1-methyl-1H-pyrazol-4-yl)picolinoyl chloride (4.2 g, 19.0 mmol), DMAP (1.24 g, 10.2 mmol), DIEA (8.9 mL, 51 mmol), DCM (34 mL) and the reaction mixture was stirred at RT for 12 hrs. The reaction mixture was then poured into a separatory funnel containing deionized water (20 mL). The phases were separated, and the aqueous phase was extracted with DCM (3 x 25 mL). The combined organic phases were combined, dried over Na₂SO₄, and the solvent removed under reduced pressure. The crude mixture was purified by flash chromatography using a pre-packed RediSep R_{f} silica gel column on a Teledyne Isco CombiFlash R_{f} automated chromatography system using 0-100% EtOAc/hexanes to afford the title compound **27**. MS (ESI) m/z 382 [(M+H)⁺ calc'd for C₁₆H₁₁ClF₃N₅O: 382].

Each of the elaborated picolinamides presented in Table 7 below were prepared in accordance with the synthetic routes in General Scheme 6, using procedures analogous to those described above.

**Table 7.**

| Intermediate | Structure |
|---|---|
| 26 | 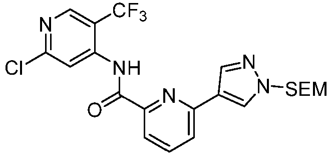 |
| 27 | 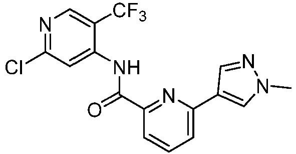 |
| 28 | 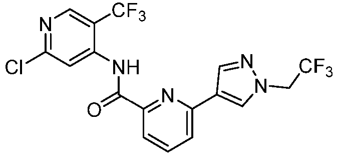 |
| 29 | 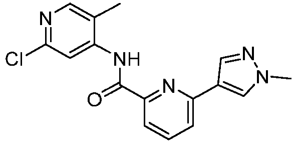 |

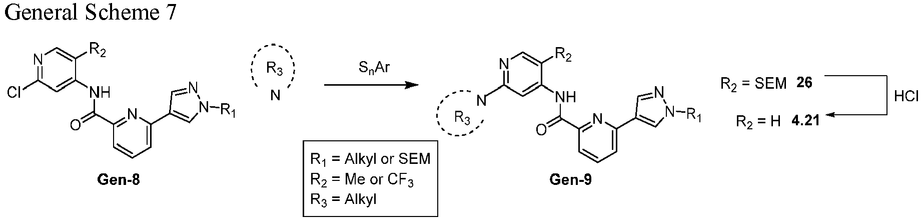

In General Scheme 7, Gen 8 picolinamides were transformed under S_{N}Ar conditions with commercially available amines to afford elaborated compounds of the form Gen-9. The representative compounds are described in more detail below.

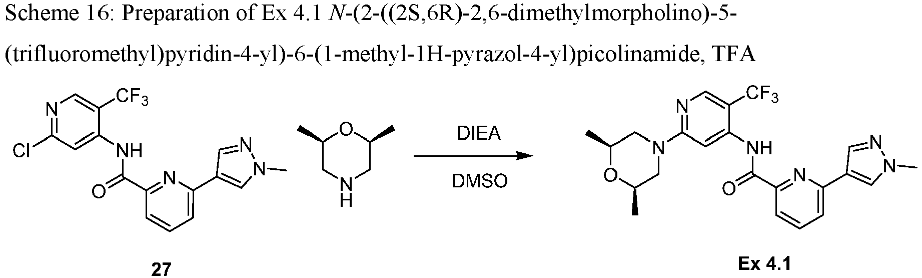

General Procedure: A 30 mL microwave vial was charged with intermediate **27** (75 mg, 0.21 mmol) *(2R,6S)*-2,6-dimethylmorpholine (226 mg, 1.97 mmol), DIEA (343 µl, 1.97 mmol), DMSO (1 mL) and the reaction was heated to 120 °C for 2 hrs. At 2 hrs, the reaction was cooled to RT and concentrated under reduced pressure. The crude product was diluted in 4 mL of DMSO, filtered and purified by mass-directed reverse phase chromatography eluting with MeCN/H₂O with 0.1% TFA with a linear gradient to afford the title compound Ex 4.1. ¹H NMR (499 MHz, DMSO-*d*₆) δ 10.76 (s, 1H), 8.37 (s, 1H), 8.33 (s, 1H), 8.04 (d, J = 9.0 Hz, 2H), 7.96 - 7.89 (m, 3H), 4.15 (d, J = 11.5 Hz, 2H), 3.58 (s, 2H), 2.46 (s, 4H), 1.14 (d, J = 6.0 Hz, 6H), -0.05 (s, 1H).. MS (ESI) m/z 461 [(M+H)⁺ calc'd for C₂₂H₂₃F₃N₆O₂: 461].

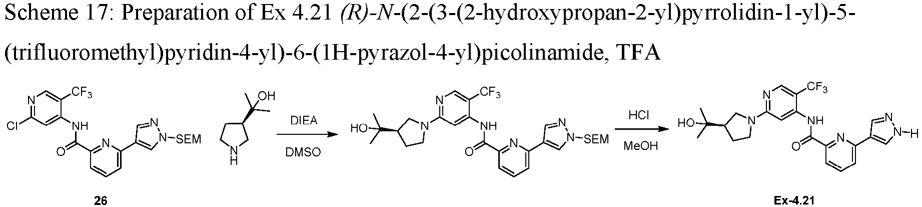

### Step 1: Preparation of (R)-N-(2-(3-(2-hydroxypropan-2-yl)pyrrolidin-1-yl)-5-(trifluoromethyl)pyridin-4-yl)-6-(1-((2-(trimethylsilyl)ethoxy)methyl)-1H-pyrazol-4-yl) picolinamide

General Procedure: A 30 mL microwave vial was charged with intermediate 26 (100 mg, 0.2 mmol), *(R)*-2-(pyrrolidin-3-yl)propan-2-ol (31 mg, 0.24 mmol), DIEA (190 µl, 1.2 mmol), DMSO (1 mL) and the reaction was heated to 120 °C for 2 hrs. At 2 hrs, the reaction mixture was poured into a separatory funnel containing deionized water (20 mL). The phases were separated, and the aqueous phase was extracted with DCM (3 x 25 mL). The organic phases were combined, dried over Na₂SO₄, and the solvent removed under reduced pressure. The crude product was purified by flash chromatography using pre-packed RediSep R_{f} silica gel columns on a Teledyne Isco CombiFlash R_{f} automated chromatography system using 0-100% EtOAc/hexanes to afford the title compound. MS (ESI) m/z 591 [(M+H)⁺ calc'd for C₂₈H₃₇F₃N₆O₃Si: 591].

### Step 2: Preparation of (R)-N-(2-(3-(2-hydroxypropan-2-yl)pyrrolidin-1-yl)-5-(trifluoromethyl)pyridin-4-yl)-6-(1H-pyrazol-4-yl)picolinamide, TFA

General Procedure: A 30 mL microwave vial was charged with *(R)*-*N*-(2-(3-(2-hydroxypropan-2-yl)pyrrolidin-1-yl)-5-(trifluoromethyl)pyridin-4-yl)-6-(1-((2-(trimethylsilyl)ethoxy)methyl)-1H-pyrazol-4-yl)picolinamide (99 mg, 0.17 mmol), MeOH (0.34 mL), HCl 4N in Dioxane (0.28 mL, 3.4 mmol) and the reaction was allowed to stir at RT for 12 hrs. At 12 hrs, the reaction mixture was concentrated under reduced pressure. The crude product was diluted in 4 mL of DMSO, filtered and purified by mass-directed reverse phase chromatography eluting with MeCN/H₂O with 0.1% TFA with a linear gradient to afford the title compound **Ex 4.21**. ¹H NMR (499 MHz, DMSO-*d*₆) δ 10.82 (s, 1H), 8.34 (s, 1H), 8.26 (s, 2H), 8.06 (t, J = 7.5 Hz, 1H), 8.01 (d, J = 7.6 Hz, 1H), 7.94 (d, J = 7.2 Hz, 1H), 7.71 (s, 1H), 3.52 (d, J = 63.5 Hz, 2H), 3.32 (dd, J = 19.1, 9.2 Hz, 2H), 2.32 (d, J = 23.9 Hz, 1H), 1.90 (d, J = 39.5 Hz, 2H), 1.12 (d, J = 12.1 Hz, 6H), 0.05 (s, 2H). MS (ESI) m/z 461 [(M+H)⁺ calc'd for C₂₂H₂₃F₃N₆O₂: 461].

Each of the elaborated picolinamides presented in Table 8 below were prepared in accordance with the synthetic routes in General Scheme 7, Scheme 16 and 17, using procedures analogous to those described above.

**Table 8.**

| Ex | Structure | Name | Exact Mass [M+H]⁺ |
|---|---|---|---|
| Ex-4.1 | 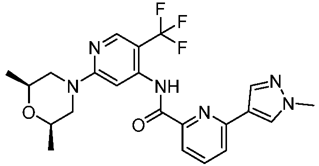 | *N*-(2-((2*R*,6*S*)-2,6-dimethylmorpholino)-5-(trifluoromethyl)pyridin-4-yl)-6-(1-methyl-1H-pyrazol-4-yl)picolinamide | Calc'd 461, found 461 |
| Ex-4.2 | 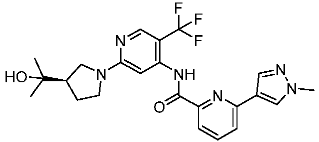 | *(R)*-*N*-(2-(3-(2-hydroxypropan-2-yl)pyrrolidin-1-yl)-5-(trifluoromethyl)pyridin-4-yl)-6-(1-methyl-1H-pyrazol-4-yl)picolinamide | Calc'd 475, found 475 |
| Ex-4.3 | 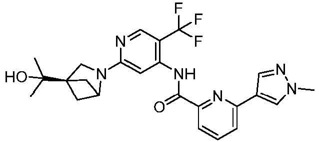 | *N*-(2-(4-(2-hydroxypropan-2-yl)-2-azabicyclo[2.1.1]hexan-2-yl)-5-(trifluoromethyl)pyridin-4-yl)-6-(1-methyl-1H-pyrazol-4-yl)picolinamide | Calc'd 487, found 487 |
| Ex-4.4 | 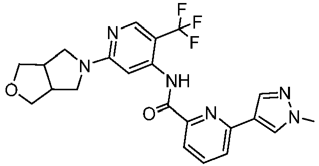 | Meso 6-(1-methyl-1H-pyrazol-4-yl)-*N*-(2-(tetrahydro-1H-furo[3,4-c]pyrrol-5(3H)-yl)-5-(trifluoromethyl)pyridin-4-yl)picolinamide | Calc'd 459, found 459 |
| Ex-4.5 | 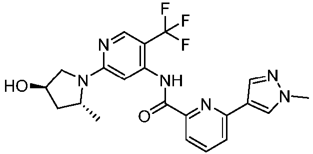 | *N*-(2-((2*R*,4*R*)-4-hydroxy-2-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.6 | 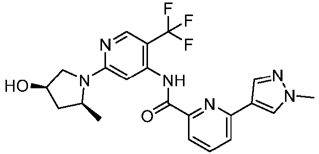 | *N*-(2-((2*S*,4*R*)-4-hydroxy-2-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.7 | 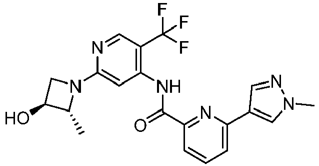 | *N*-(2-((2*R,*3*S*)-3-hydroxy-2-methylazetidin-1-yl)-5-(trifluoromethyl)pyridin-4-yl)-6-(1-methyl-1H-pyrazol-4-yl)picolinamide | Calc'd 433, found 433 |
| Ex-4.8 | 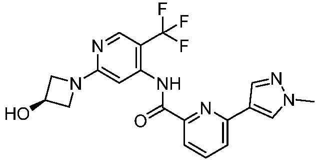 | *N*-(2-(3-hydroxyazetidin-1-yl)-5-(trifluoromethyl)pyridin-4-yl)-6-(1-methyl-1H-pyrazol-4-yl)picolinamide | Calc'd 419, found 419 |
| Ex-4.9 | 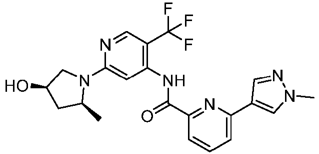 | *N*-(2-((3*R*,5*S*)-4-hydroxy-2-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.10 | 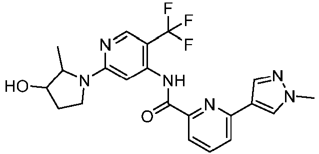 | *(R, R)* or *(S*, *S) N*-(2-(3-hydroxy-2-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.11 | 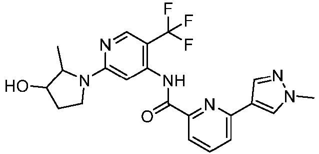 | *(R, R)* or *(S, S) N*-(2-(3-hydroxy-2-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.12 | 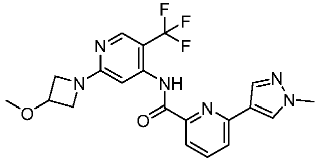 | *N*-(2-(3-methoxyazetidin-1-yl)-5-(trifluoromethyl)pyridin-4-yl)-6-(1-methyl-1H-pyrazol-4-yl)picolinamide | Calc'd 433, found 433 |
| Ex-4.13 | 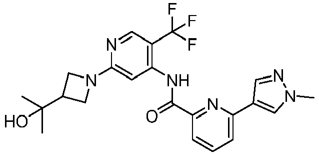 | *N*-(2-(3-(2-hydroxypropan-2-yl)azetidin-1-yl)-5-(trifluoromethyl)pyridin-4-yl)-6-(1-methyl-1H-pyrazol-4-yl)picolinamide | Calc'd 461, found 461 |
| Ex-4.14 | 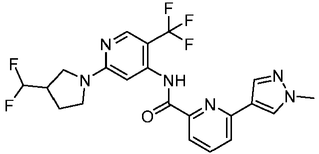 | *N-(2-(3-*(difluoromethyl)pyrrolidin-1-yl)-5-(trifluoromethyl)pyridin-4-yl)-6-(1-methyl-1H-pyrazol-4-yl)picolinamide | Calc'd 467, found 467 |
| Ex-4.15 | 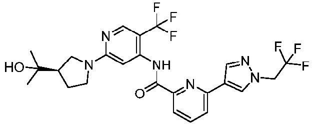 | *(R)-N-*(2-(3-(2-hydroxypropan-2-yl)pyrrolidin-1-yl)-5-(trifluoromethyl)pyridin-4-yl)-6-(1-(2,2,2-trifluoroethyl)-1H-pyrazol-4-yl)picolinamide | Calc'd 543, found 543 |
| Ex-4.16 | 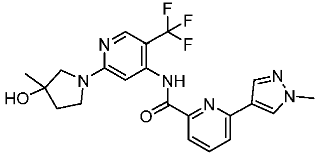 | *(R)* or *(S) N*-(2-(3-hydroxy-3-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.17 | 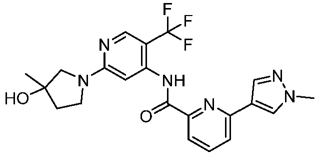 | *(R)* or *(S) N*-(2-(3-hydroxy-3-methylpyrrolidin-1-yl)-5-(trifluoromethyl)pyridin-4-yl)-6-(1-methyl-1H-pyrazol-4-yl)picolinamide | Calc'd 447, found 447 |
| Ex-4.18 | 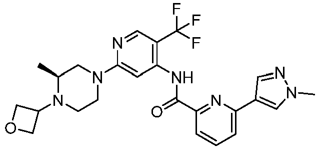 | *(S)*-6-(1-methyl-1H-pyrazol-4-yl)-*N*-(2-(3-methyl-4-(oxetan-3-yl)piperazin-1-yl)-5-(trifluoromethyl)pyridin-4-yl)picolinamide | Calc'd 502, found 502 |
| Ex-4.19 | 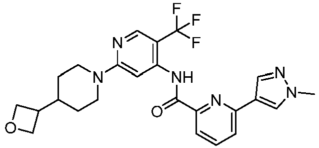 | 6-(1-methyl-1H-pyrazol-4-yl)-N-(2-(4-(oxetan-3-yl)piperidin-1-yl)-5-(trifluoromethyl)pyridin-4-yl)picolinamide | Calc'd 487, found 487 |
| Ex-4.20 | 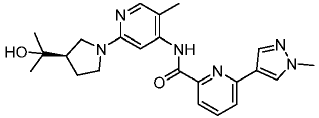 | *(R)-N-*(2-(3-(2-hydroxypropan-2-yl)pyrrolidin-1-yl)-5-methylpyridin-4-yl)-6-(1-methyl-1H-pyrazol-4-yl)picolinamide | Calc'd 421, found 421 |
| Ex-4.21 | 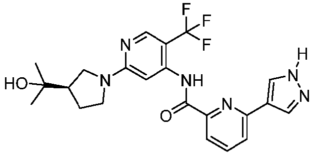 | *(R)*-*N*-(2-(3-(2-hydroxypropan-2-yl)pyrrolidin-1-yl)-5-(trifluoromethyl)pyridin-4-yl)-6-(1H-pyrazol-4-yl)picolinamide | Calc'd 461, found 461 |
| Ex-4.22 | 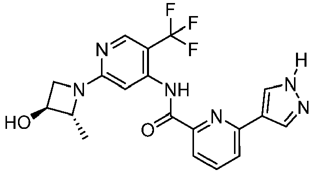 | *N*-(2-((2R,3S)-3-hydroxy-2-methylazetidin-1-yl)-5-(trifluoromethyl)pyridin-4-yl)-6-(1H-pyrazol-4-yl)picolinamide | Calc'd 419, found 419 |
| Ex-4.23 | 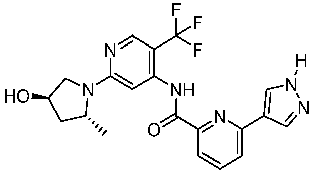 | *N-*(2-((*2R,*4*R*)-4-hydroxy-2-methylpyrrolidin-1-yl)-5-(trifluoromethyl)pyridin-4-yl)-6-(1H-pyrazol-4-yl)picolinamide | Calc'd 433, found 433 |
| Ex-4.24 | 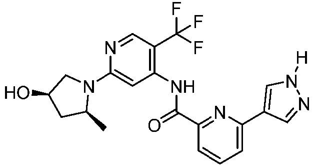 | *N*-(2-((2*S*,4*R*)-4-hydroxy-2-methylpyrrolidin-1-yl)-5-(trifluoromethyl)pyridin-4-yl)-6-(1H-pyrazol-4-yl)picolinamide | Calc'd 433, found 433 |
| Ex-4.25 | 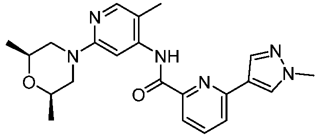 | *N-(2-((2S,6R)-2,6-*dimethylmorpholino)-5-methylpyridin-4-yl)-6-(1-methyl-1H-pyrazol-4-yl)picolinamide | Calc'd 407, found 407 |

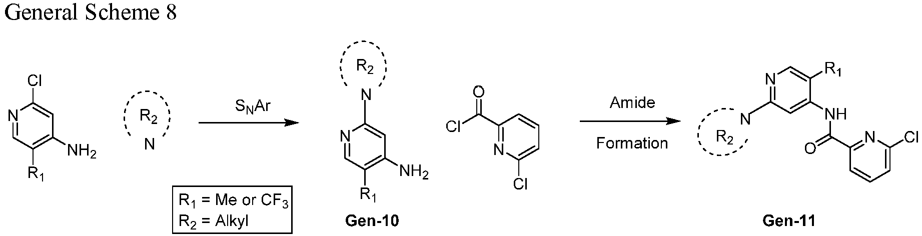

In General Scheme 8, chloro pyridin-4-amines were transformed under S_{N}Ar conditions with commercially available amines to afford Gen-10 pyridin-4-amines. These were condensed with 6-chloropicolinoyl chloride to afford picolinamides Gen-11. The representative compounds are described in more detail below.

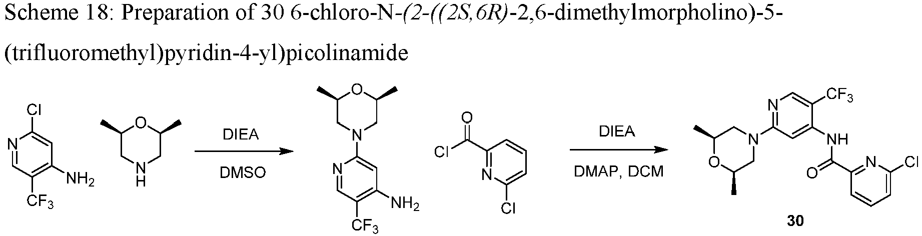

### Step 1: Preparation of 2-((2S,6R)-2,6-dimethylmorpholino)-5-(trifluoromethyl)pyridin-4-amine

General Procedure: A 30 mL microwave vial was charged with *(*2*S,* 6*R)-*2,6-dimethylmorpholine (0.81 mL, 6.5 mmol), 2-chloro-5-(trifluoromethyl)pyridin-4-amine (250 mg, 1.3 mmol), DMSO (5 mL), DIEA (1.1 mL, 6.3 mmol) and the reaction mixture was stirred at 120 °C for 12 hrs. The mixture was then allowed to cool to RT and the product was precipitated in water. The off-white precipitate was dried under vacuum overnight to afford the title compound. MS (ESI) m/z 276 [(M+H)⁺ calc'd for C₁₂H₁₆F₃N₃O: 276].

### Step 2: Preparation of 6-chloro-N-(2-((2S,6R)-2,6-dimethylmorpholino)-5-(trifluoromethyl)pyridin-4-yl)picolinamide

General Procedure: A 30 mL microwave vial was charged with, 2-((2*S*,6*R*)-2,6-dimethylmorpholino)-5-(trifluoromethyl)pyridin-4-amine (466 mg, 1.71 mmol), DIEA (1.5 mL, 8.5 mmol), DMAP (210 mg, 1.7 mmol), DCM (5.5 mL) and the reaction was allowed to stir at RT for 12 hrs. At 12 hrs, the reaction mixture was concentrated under reduced pressure. The crude mixture was purified by flash chromatography using pre-packed RediSep R_{f} silica gel columns on a Teledyne Isco CombiFlash R_{f} automated chromatography system using 0-100% EtOAc/hexanes to afford the title compound 30. MS (ESI) m/z 415 [(M+H)⁺ calc'd for C₁₈H₁₈ClF₃NaO₂: 415].

Each of the elaborated picolinamides presented in Table 9 below were prepared in accordance with the synthetic routes in General Scheme 8, using procedures analogous to those described above.

**Table 9.**

| Intermediate | Structure |
|---|---|
| 30 | 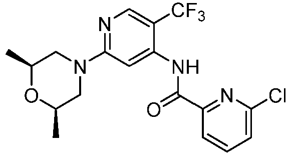 |
| 31 | 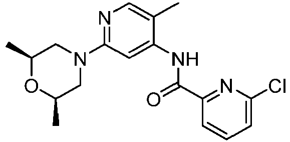 |
| 32 | 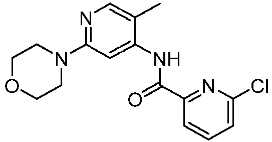 |

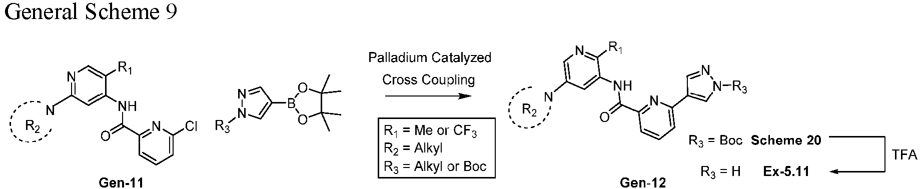

In General Scheme 9, Gen-11 picolinamides were transformed under palladium-catalyzed Suzuki coupling with either commercially available or synthetically prepared boronates 1-5 to afford elaborated compounds of the form Gen-12. Representative preparative examples are described in more detail below.

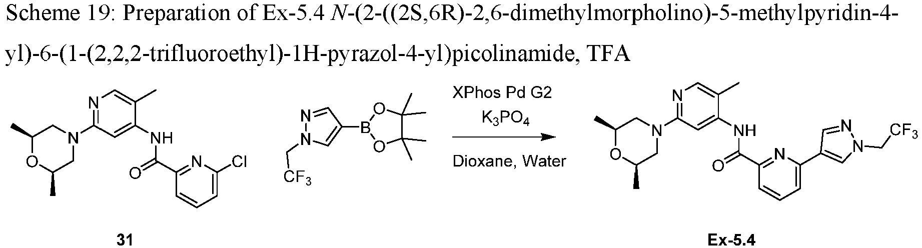

General Procedure: A 30 mL microwave vial was charged with intermediate 31 (75 mg, 0.21 mmol), XPhos Pd G2 (16 mg, 0.021 mmol), intermediate 2 (63 mg, 0.23 mmol), dioxane (0.8 mL), K₃PO₄ (0.2 ml, 0.41 mmol, 2M in water) and the reaction mixture was stirred at 80 °C for 12 hrs. At 12 hrs, the reaction mixture was cooled to RT and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound Ex-5.4. ¹H NMR (499 MHz, DMSO-*d*₆) δ 10.64 (s, 1H), 8.58 (s, 1H), 8.31 (s, 1H), 8.10 (t, *J* = 7.5 Hz, 1H), 8.07 - 8.02 (m, 2H), 7.99 (s, 2H), 5.24 (d, *J* = 8.7 Hz, 2H), 3.89 (d, *J* = 12.0 Hz, 2H), 3.66 (s, 2H), 2.68 (t, *J =* 10.6 Hz, 2H), 2.37 (s, 3H), 1.15 (d, *J =* 5.6 Hz, 6H). MS (ESI) m/z 475 [(M+H)⁺ calc'd for C₂₃H₂₅F₃N₆O₂: 475].

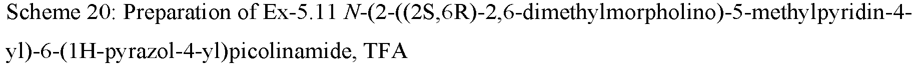

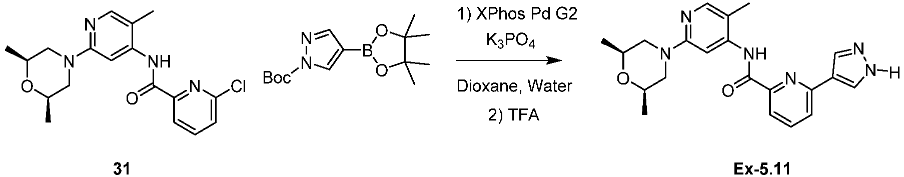

General Procedure: A 30 mL microwave vial was charged with intermediate 31 (36.1 mg, 0.1 mmol), XPhos Pd G2 (7.9 mg, 10.0 µmol), tert-butyl 4-(4,4,5,5-tetramethyl-1,3,2-dioxaborolan-2-yl)-1H-pyrazole-1-carboxylate (32 mg, 0.11 mmol), dioxane (0.4 ml), K₃PO₄ (0.1 ml, 0.2 mmol, 2M in water) and the reaction mixture was heated to 80 °C for 12 hrs. At 12 hrs, the reaction mixture was cooled to RT and TFA (1 mL) was added. The reaction mixture was stirred at RT for 2 hrs. At 2 hrs the mixture was concentrated under reduced pressure, diluted in 4 mL of DMSO, filtered and purified by mass-directed reverse phase chromatography eluting with MeCN/H₂O with 0.1% TFA with a linear gradient to afford the title compound Ex-5.11. ¹H NMR (499 MHz, DMSO-*d*₆) δ 10.73 (s, 1H), 8.40 (s, 2H), 8.04 (dd, *J* = 33.2, 22.5 Hz, 5H), 7.26 - 6.95 (m, 1H), 3.93 (s, 2H), 3.70 (s, 2H), 2.71 (s, 2H), 2.42 (s, 3H), 1.20 (s, 6H). MS (ESI) m/z 393 [(M+H)⁺ calc'd for C₂₁H₂₄N₆O₂: 393].

Each of the elaborated picolinamides presented in Table 10 below were prepared in accordance with the synthetic routes in General Scheme 9 and Scheme 19 and 20, using procedures analogous to those described above.

**Table 10.**

| Ex | Structure | Name | Exact Mass [M+H]⁺ |
|---|---|---|---|
| Ex-5.1 | 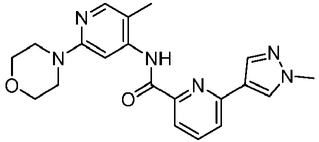 | 6-(1-methyl-1H-pyrazol-4-yl)-N-(5-methyl-2-morpholinopyridin-4-yl)picolinamide | Calc'd 379, found 379 |
| Ex-5.2 | 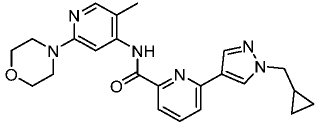 | 6-(1-(cyclopropylmethyl)-1H-pyrazol-4-yl)-N-(5-methyl-2-morpholinopyridin-4-yl)picolinamide | Calc'd 419, found 419 |
| Ex-5.3 | 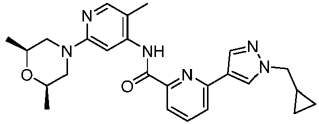 | 6-(1-(cyclopropylmethyl)-1H-pyrazol-4-yl)-N-(2-((2S,6R)-2,6-dimethylmorpholino)-5-methylpyridin-4-yl)picolinamide | Calc'd 447, found 447 |
| Ex-5.4 | 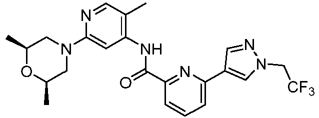 | *N-(2-((2S,6R)-2,6-*dimethylmorpholino)-5-methylpyridin-4-yl)-6-(1-(2,2,2-trifluoroethyl)-1H-pyrazol-4-yl)picolinamide | Calc'd 475, found 475 |
| Ex-5.5 | 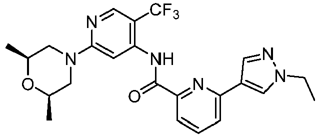 | *N-(2-((2S,6R)-2,6-*dimethylmorpholino)-5-(trifluoromethyl)pyridin-4-yl)-6-(1-ethyl-1H-pyrazol-4-yl)picolinamide | Calc'd 475, found 475 |
| Ex-5.6 | 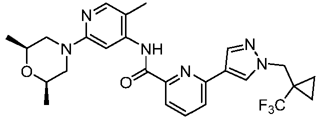 | *N-(2-((2S,6R)-2,6-*dimethylmorpholino)-5-methylpyridin-4-yl)-6-(1-((1-(trifluoromethyl)cyclopropyl)me thyl)-1H-pyrazol-4-yl)picolinamide | Calc'd 515, found 515 |
| Ex-5.7 | 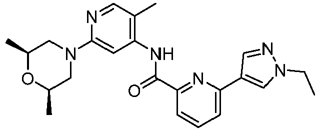 | *N-(2-((2S,6R)-2,6-*dimethylmorpholino)-5-methylpyridin-4-yl)-6-(1-ethyl-1H-pyrazol-4-yl)picolinamide | Calc'd 421, found 421 |
| Ex-5.8 | 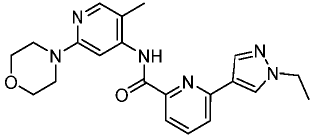 | 6-(1-ethyl-1H-pyrazol-4-yl)-N-(5-methyl-2-morpholinopyridin-4-yl)picolinamide | Calc'd 393, found 393 |
| Ex-5.9 | 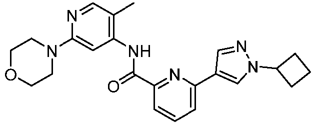 | 6-(1-cyclobutyl-1H-pyrazol-4-yl)-*N*-(5-methyl-2-morpholinopyridin-4-yl)picolinamide | Calc'd 419, found 419 |
| Ex-5.10 | 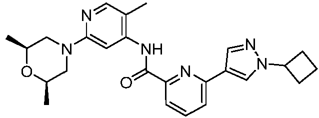 | 6-(1-cyclobutyl-1H-pyrazol-4-*yl)-N-(2-((2S,6R)-2,6-*dimethylmorpholino)-5-methylpyridin-4-yl)picolinamide | Calc'd 447, found 447 |
| Ex-5.11 | 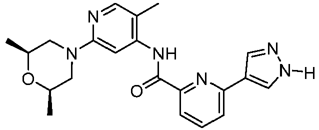 | *N-(2-((2S,6R)-2,6-*dimethylmorpholino)-5-methylpyridin-4-yl)-6-(1H-pyrazol-4-yl)picolinamide | Calc'd 393, found 393 |
| Ex-5.12 | 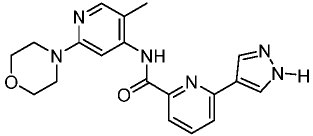 | *N*-(5-methyl-2-morpholinopyridin-4-yl)-6-(1H-pyrazol-4-yl)picolinamide | Calc'd 365, found 365 |

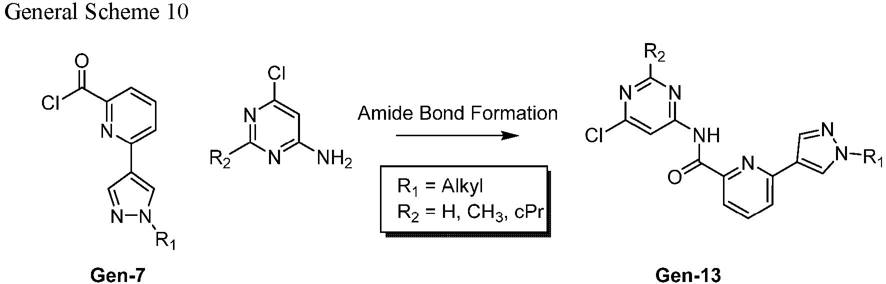

In General Scheme 10, picolinoyl chlorides Gen 7 were condensed with commercially available pyrimidine-4-amines to afford elaborated picolinamides of the form Gen-13. Representative preparative examples are described in more detail below.

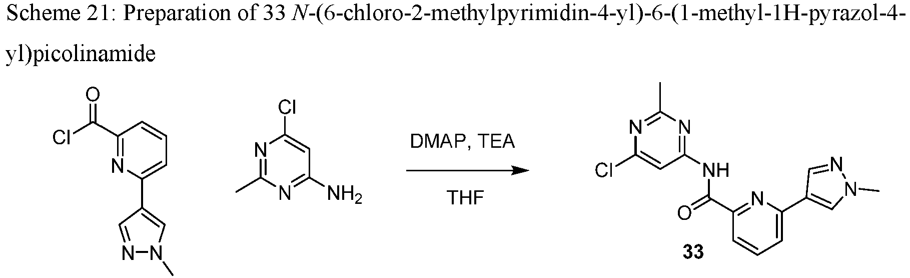

General Procedure: A 100 mL round bottom flask was charged with 6-chloro-2-methylpyrimidin-4-amine (0.7 g, 4.9 mmol), 6-(1-methyl-1H-pyrazol-4-yl)picolinoyl chloride (1.1 g, 4.9 mmol), DMAP (cat.), TEA (2 mL, 14.8 mmol), THF (25 ml) and the reaction mixture was stirred at 80 °C for 12 hrs. At 12 hrs, the reaction mixture was poured into a separatory funnel containing deionized water (20 mL). The phases were separated, and the aqueous phase was extracted with DCM (3 x 25 mL). The organic phases were combined, dried over Na₂SO₄, and the solvent removed under reduced pressure to afford title compound 33 (1.61 g, 100%). MS (ESI) m/z 329 [(M+H)⁺ calc'd for C₁₅H₁₃ClN₆O: 329]

Each of the elaborated picolinamides presented in Table 11 below were prepared in accordance with the synthetic routes in General Scheme 10, using procedures analogous to those described above.

**Table 11.**

| Intermediate | Structure |
|---|---|
| 33 | 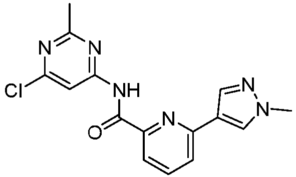 |
| 34 | 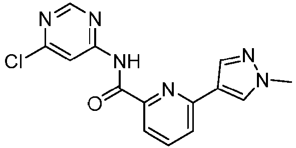 |
| 35 | 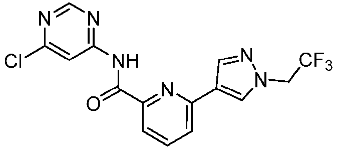 |
| 36 | 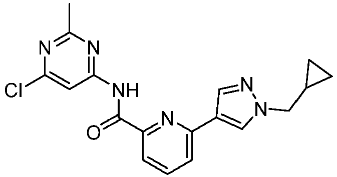 |
| 37 | 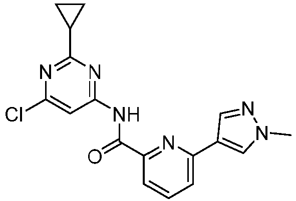 |
| 38 | 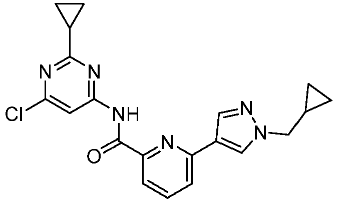 |
| 39 | 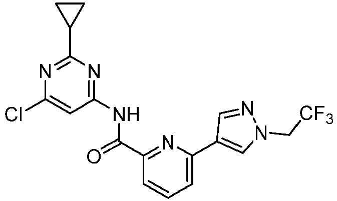 |

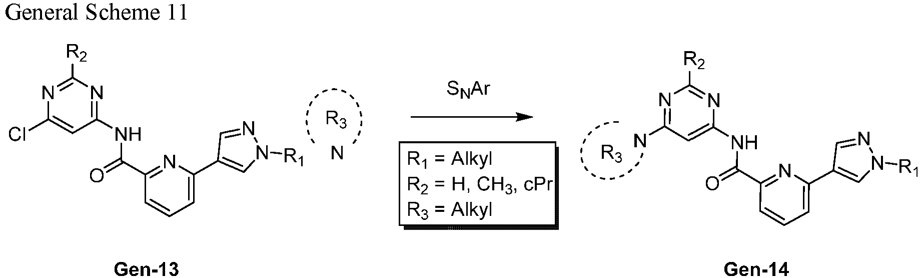

In General Scheme 11, Gen-13 picolinamides were transformed under S_{N}Ar conditions with commercially available amines to afford elaborated compounds Gen-14. Representative preparative examples are described in more detail below.

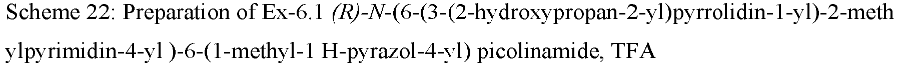

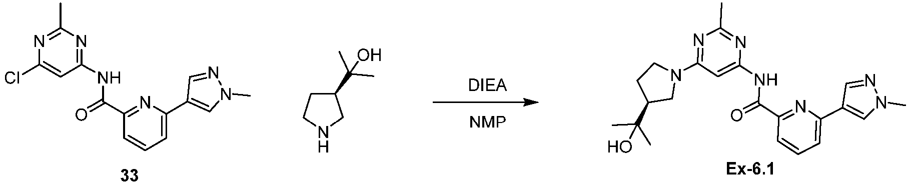

General Procedure: A 30 mL microwave vial was charged with intermediate 33 (132 mg, 0.4 mmol), (*R*)-2-(pyrrolidin-3-yl)propan-2-ol, HCl (130 mg, 0.8 mmol), NMP (0.67 mL), DIEA (105 µl, 0.60 mmol), and mixture was heated to 140 °C for 12 hrs. At 12 hrs, the reaction mixture was cooled to RT and concentrated under reduced pressure. The crude product was diluted in DMSO (4 mL), filtered, and purified by mass-directed reverse phase chromatography eluting with MeCN/H₂O with 0.1% TFA with a linear gradient to afford the title compound Ex-6.1. ¹H NMR (499 MHz, DMSO-*d*₆) δ 10.81 (s, 1H), 8.49 (s, 1H), 8.25 (s, 1H), 8.04 (t, J = 7.5 Hz, 1H), 7.93 (dd, J = 18.5, 7.5 Hz, 2H), 7.24 - 7.15 (m, 1H), 3.89 (s, 4H), 3.75 (s, 1H), 3.58 (s, 1H), 3.49 - 3.29 (m, 3H), 2.35 (s, 1H), 2.24 (s, 1H), 1.98 (s, 1H), 1.91 (s, 1H), 1.81 (d, J = 9.4 Hz, 1H), 1.12 (d, J = 16.9 Hz, 6H). MS (ESI) m/z 422 [(M+H)⁺ calc'd for C₂₂H₂₇N₇O₂: 422].

Each of the elaborated picolinamides presented in Table 12 below were prepared in accordance with the synthetic routes in General Scheme 11 and Scheme 22, using procedures analogous to those described above.

**Table 12.**

| Ex | Structure | Name | Exact Mass [M+H]⁺ |
|---|---|---|---|
| Ex-6.1 | 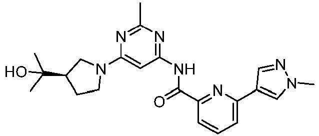 | *(R)-N*-(6-(3-(2-hydroxypropan-2-yl)pyrrolidin-1-yl)-2-methylpyrimidin-4-yl)-6-(1-methyl-1H-pyrazol-4-yl)picolinamide | Calc'd 422, found 422 |
| Ex-6.2 | 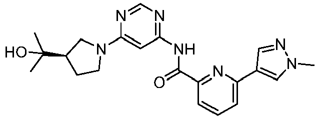 | *(R)-N-*(6-(3-(2-hydroxypropan-2-yl)pyrrolidin-1-yl)pyrimidin-4-yl)-6-(1-methyl-1H-pyrazol-4-yl)picolinamide | Calc'd 408, found 408 |
| Ex-6.3 | 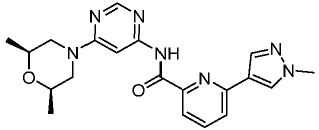 | *N*-(6-((2S,6R)-2,6-dimethylmorpholino)pyrimidin-4-yl)-6-(1-methyl-1H-pyrazol-4-yl)picolinamide | Calc'd 394, found 394 |
| Ex-6.4 | 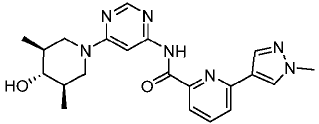 | *N*-(6-((3S,4r,5R)-4-hydroxy-3,5-dimethylpiperidin-1-yl)pyrimidin-4-yl)-6-(1-methyl-1H-pyrazol-4-yl)picolinamide | Calc'd 408, found 408 |
| Ex-6.5 | 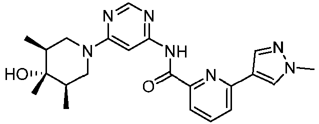 | *N*-(6-((3S,4r,5R)-4-hydroxy-3,4,5-trimethylpiperidin-1-yl)pyrimidin-4-yl)-6-(1-methyl-1H-pyrazol-4-yl)picolinamide | Calc'd 422, found 422 |
| Ex-6.6 | 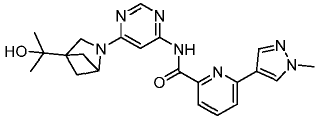 | *N*-(6-(4-(2-hydroxypropan-2-yl)-2-azabicyclo[2.1.1]hexan-2-yl)pyrimidin-4-yl)-6-(1-methyl-1H-pyrazol-4-yl)picolinamide | Calc'd 420, found 420 |
| Ex-6.7 | 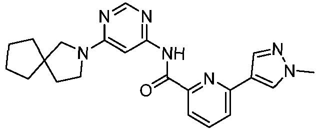 | *N*-(6-(2-azaspiro[4.4]nonan-2-yl)pyrimidin-4-yl)-6-(1-methyl-1H-pyrazol-4-yl)picolinamide | Calc'd 404, found 404 |
| Ex-6.8 | 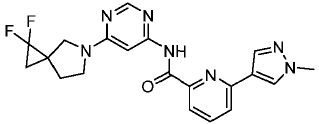 | *(R)* or *(S) N*-(6-(1,1-difluoro-5-azaspiro[2.4]heptan-5-yl)pyrimidin-4-yl)-6-(1-methyl-1H-pyrazol-4-yl)picolinamide | Calc'd 412, found 412 |
| Ex-6.9 | 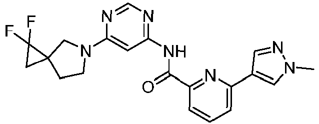 | *(R)* or *(S) N*-(6-(1,1-difluoro-5-azaspiro[2.4]heptan-5-yl)pyrimidin-4-yl)-6-(1-methyl-1H-pyrazol-4-yl)picolinamide | Calc'd 412, found 412 |
| Ex-6.10 | 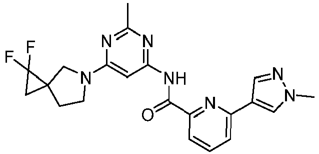 | *(R)* or *(S) N*-(6-(1,1-difluoro-5-azaspiro[2.4]heptan-5-yl)-2-methylpyrimidin-4-yl)-6-(1-methyl-1H-pyrazol-4-yl)picolinamide | Calc'd 426, found 426 |
| Ex-6.11 | 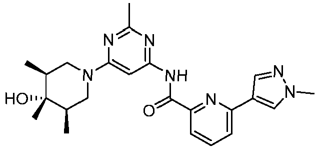 | *N*-(6-((3S,4r,5R)-4-hydroxy-3,4,5-trimethylpiperidin-1-yl)-2-methylpyrimidin-4-yl)-6-(1-methyl-1H-pyrazol-4-yl)picolinamide | Calc'd 436, found 436 |
| Ex-6.12 | 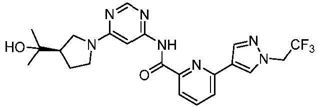 | *(R)-N-(*6-(3-(2-hydroxypropan-2-yl)pyrrolidin-1-yl)pyrimidin-4-yl)-6-(1-(2,2,2-trifluoroethyl)-1H-pyrazol-4-yl)picolinamide | Calc'd 476, found 476 |
| Ex-6.13 | 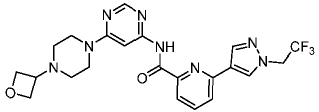 | *N*-(6-(4-(oxetan-3-yl)piperazin-1-yl)pyrimidin-4-yl)-6-(1-(2,2,2-trifluoroethyl)-1H-pyrazol-4-yl)picolinamide | Calc'd 489, found 489 |
| Ex-6.14 | 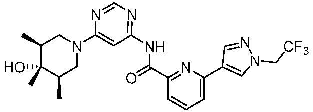 | *N*-(6-((3S,4r,5R)-4-hydroxy-3,4,5-trimethylpiperidin-1-yl)pyrimidin-4-yl)-6-(1-(2,2,2-trifluoroethyl)-1H-pyrazol-4-yl)picolinamide | Calc'd 490, found 490 |
| Ex-6.15 | 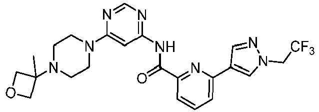 | *N*-(6-(4-(3-methyloxetan-3-yl)piperazin-1-yl)pyrimidin-4-yl)-6-(1-(2,2,2-trifluoroethyl)-1H-pyrazol-4-yl)picolinamide | Calc'd 503, found 503 |
| Ex-6.16 | 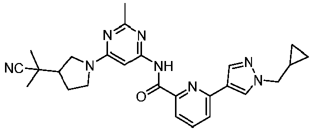 | *N*-(6-(3-(2-cyanopropan-2-yl)pyrrolidin-1-yl)-2-methylpyrimidin-4-yl)-6-(1-(cyclopropylmethyl)-1H-pyrazol-4-yl)picolinamide | Calc'd 471, found 471 |
| Ex-6.17 | 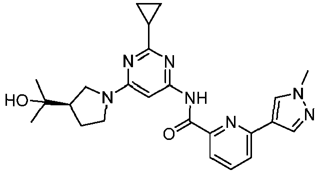 | *(R)*-*N*-(2-cyclopropyl-6-(3-(2-hydroxypropan-2-yl)pyrrolidin-1-yl)pyrimidin-4-yl)-6-(1-methyl-1H-pyrazol-4-yl)picolinamide | Calc'd 448, found 448 |
| Ex-6.18 | 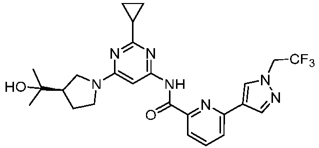 | *(R)*-*N*-(2-cyclopropyl-6-(3-(2-hydroxypropan-2-yl)pyrrolidin-1-yl)pyrimidin-4-yl)-6-(1-(2,2,2-trifluoroethyl)-1H-pyrazol-4-yl)picolinamide | Calc'd 516, found 516 |
| Ex-6.19 | 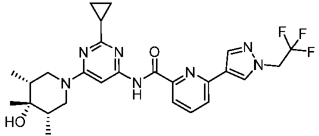 | *N*-(2-cyclopropyl-6-((3S,4s,5R)-4-hydroxy-3,4,5-trimethylpiperidin-1-yl)pyrimidin-4-yl)-6-(1-(2,2,2-trifluoroethyl)-1H-pyrazol-4-yl)picolinamide | Calc'd 530, found 530 |
| Ex-6.20 | 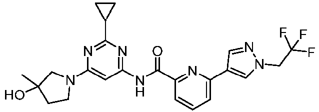 | *N*-(2-cyclopropyl-6-(3-hydroxy-3-methylpyrrolidin-1-yl)pyrimidin-4-yl)-6-(1-(2,2,2-trifluoroethyl)-1H-pyrazol-4-yl)picolinamide | Calc'd 488, found 488 |
| Ex-6.21 | 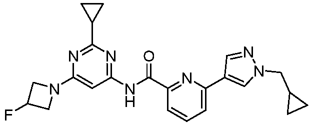 | *N*-(2-cyclopropyl-6-(3-fluoroazetidin-1-yl)pyrimidin-4-yl)-6-(1-(cyclopropylmethyl)-1H-pyrazol-4-yl)picolinamide | Calc'd 434, found 434 |
| Ex-6.22 | 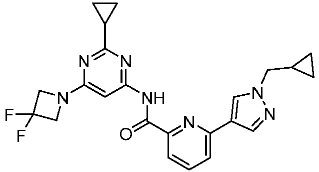 | *N*-(2-cyclopropyl-6-(3,3-difluoroazetidin-1-yl)pyrimidin-4-yl)-6-(1-(cyclopropylmethyl)-1H-pyrazol-4-yl)picolinamide | Calc'd 452, found 452 |
| Ex-6.23 | 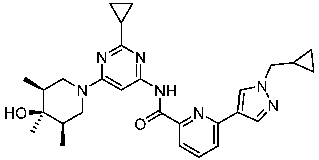 | *N*-(2-cyclopropyl-6-((3S,4s,5R)-4-hydroxy-3,4,5-trimethylpiperidin-1-yl)pyrimidin-4-yl)-6-(1-(cyclopropylmethyl)-1H-pyrazol-4-yl)picolinamide | Calc'd 502, found 502 |

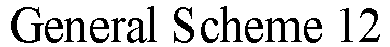

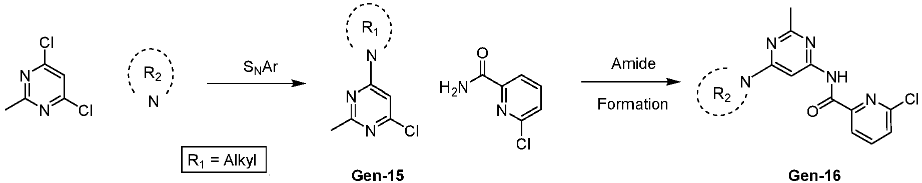

In General Scheme 12, commercially available dichloropyrimidine was transformed under S_{N}Ar conditions with commercially available amines to afford Gen-15 chloropyrimidines. These were condensed with 6-chloropicolinamide using palladium-catalyzed coupling to afford picolinamides Gen-16. The representative compounds are described in more detail below.

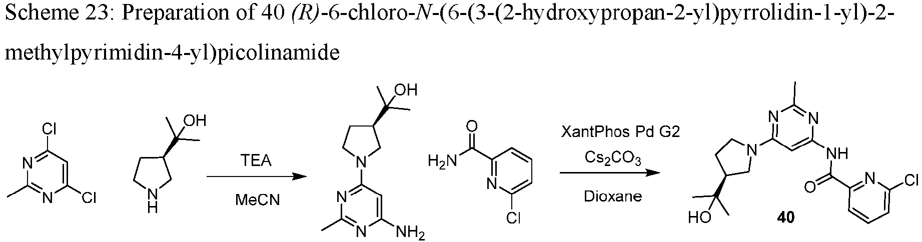

### Step 1: Preparation of (R)-2-(1-(6-chloro-2-methylpyrimidin-4-yl)pyrrolidin-3-yl)propan-2-ol

General Procedure: A 30 mL microwave vial was charged with (*R*)-2-(pyrrolidin-3-yl)propan-2-ol hydrochloride (500 mg, 3.0 mmol), 4,6-dichloro-2-methylpyrimidine (541 mg, 3.3 mmol), acetonitrile (10 ml), and TEA (1.3 ml, 9.1 mmol). The reaction mixture was stirred at 80 °C for 12 hrs. At 12 hrs, the reaction mixture was concentrated under reduced pressure and was purified by flash chromatography using pre-packed RediSep R_{f} silica gel columns on a Teledyne Isco CombiFlash R_{f} automated chromatography system using 0-100% EtOAc/hexanes to afford the afford the title compound. MS (ESI) m/z 237 [(M+H)⁺ calc'd for C₁₂H₂₀N₄O: 237]

### Step 2: Preparation of (R)-6-chloro-N-(6-(3-(2-hydroxypropan-2-yl)pyrrolidin-1-yl)-2-methylpyrimidin-4-yl)picolinamide

General Procedure: A 30 mL microwave vial was charged with 6-chloropicolinamide (60 mg, 0.38 mmol), (*R*)-2-(1-(6-chloro-2-methylpyrimidin-4-yl)pyrrolidin-3-yl)propan-2-ol (196 mg, 0.77 mmol), XantPhos Pd G2 (37 mg, 0.04 mmol), Cs₂CO₃ (375 mg, 1.15 mmol), and Dioxane (1.3 mL). The reaction mixture was purged with nitrogen three times and heated to 70 °C for 3 hrs. At 3 hrs, the reaction mixture was concentrated under reduced pressure and purified by flash chromatography using pre-packed RediSep R_{f} silica gel columns on a Teledyne Isco CombiFlash R_{f} automated chromatography system using 0-100% EtOAc/hexanes to afford the title compound 40. MS (ESI) m/z 376.0 [(M+H)⁺ calc'd for C₁₈H₂₂ClN₅O2: 376]

Each of the elaborated picolinamides presented in Table 13 below were prepared in accordance with the synthetic routes in General Scheme 12, using procedures analogous to those described above.

**Table 13.**

| Intermediate | Structure |
|---|---|
| 40 | 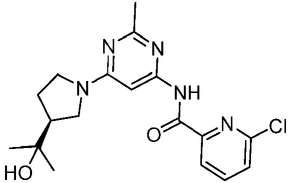 |
| 41 | 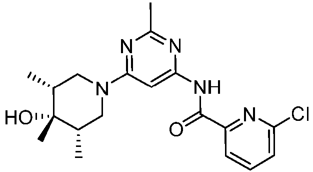 |
| 42 | 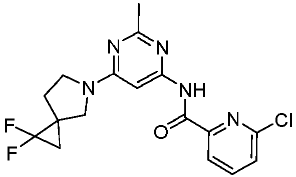 |
| 43 | 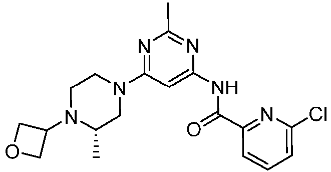 |

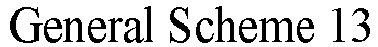

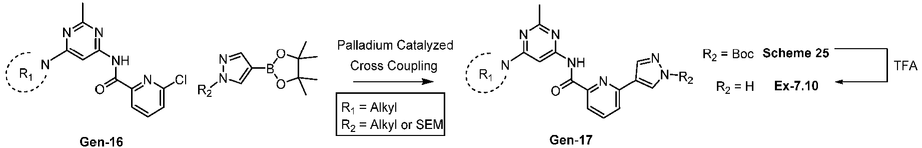

In General Scheme 13, Gen-16 picolinamides were transformed under palladium-catalyzed coupling with either commercially available or synthetically prepared boronates 1-5 to afford elaborated compounds of the form Gen-17. Representative preparative examples are described in more detail below.

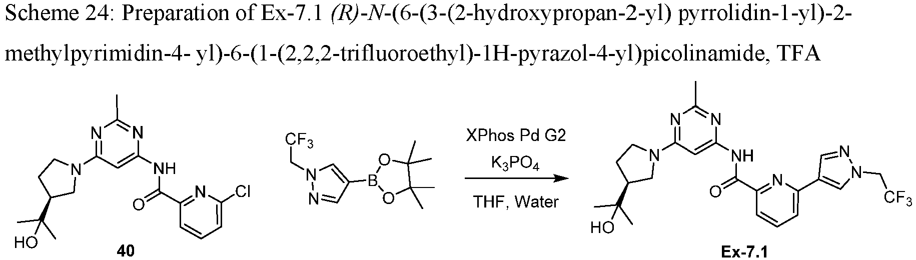

General Procedure: A 30 mL microwave vial was charged with intermediate 40 (120 mg, 0.32 mmol), intermediate 2 (176 mg, 0.64 mmol), Xphos Pd G2 (50.2 mg, 0.064 mmol), K₃PO₄ (203 mg, 0.95 mmol) and THF (1.3 mL). The reaction mixture was purged with nitrogen three times and stirred at 80 °C for 12 hrs. At 12 hrs, the mixture was cooled and concentrated under reduced pressure. The crude product was diluted in 4 mL of DMSO, filtered, and purified by mass-directed reverse phase chromatography eluting with MeCN/H₂O with 0.1% TFA with a linear gradient to afford the title compound Ex-7.1. ¹H NMR (600 MHz, DMSO-*d*6) δ 10.62 (s, 1H), 8.64 (s, 1H), 8.40 (s, 1H), 8.07 (d, J = 7.4 Hz, 1H), 8.03 (d, J = 7.5 Hz, 1H), 7.98 (d, J = 7.2 Hz, 1H), 7.19 (s, 1H), 5.24 (d, J = 8.7 Hz, 2H), 3.87 (s, 2H), 3.74 (s, 2H), 3.56 (s, 1H), 2.29 (d, J = 61.3 Hz, 2H), 1.89 (t, J = 54.3 Hz, 2H), 1.10 (s, 8H). MS (ESI) m/z 490 [(M+H)⁺ calc'd for C₂₃H₂₆F₃N₇O₂: 490].

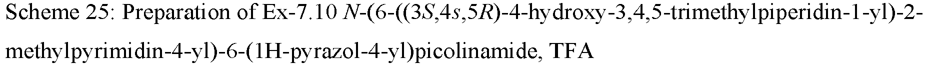

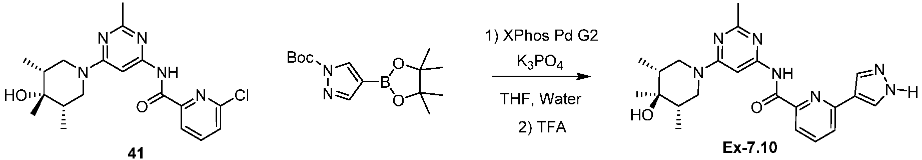

General Procedure: A 30 mL microwave vial was charged with intermediate 41 (78 mg, 0.2 mmol), tert-butyl 4-(4,4,5,5-tetramethyl-1,3,2-dioxaborolan-2-yl)-1H-pyrazole-1-carboxylate (65 mg, 0.22 mmol), XPhos Pd G2 (15.7 mg, 0.02 mmol), Dioxane (0.8 ml), K₃PO₄ (0.2 ml, 0.4 mmol, 2M in water), and the reaction mixture was heated to 80 °C for 12 hrs. At 12 hrs, the reaction mixture was cooled to RT and TFA (0.2 ml, 2.60 mmol) was added. The reaction mixture was stirred at RT for 2 hrs. At 2 hrs, the mixture was filtered, concentrated under reduced pressure and diluted in DMSO (4 mL). The crude product was purified by mass-directed reverse phase chromatography eluting with MeCN/H₂O with 0.1% TFA with a linear gradient to afford the title compound Ex-7.10. ¹H NMR (600 MHz, DMSO-d6) δ 10.81 (s, 1H), 8.45 (s, 2H), 8.02 (dd, *J =* 19.9, 7.5 Hz, 2H), 7.93 (d, *J* = 7.2 Hz, 1H), 7.47 (s, 1H), 4.36 (s, 3H), 2.96 (s, 2H), 1.50 (s, 3H), 1.06 (s, 4H), 0.89 (d, *J* = 6.2 Hz, 8H). MS (ESI) m/z 422 [(M+H)⁺ calc'd for C₂₂H₂₇N₇O₂: 422].

Each of the elaborated picolinamides presented in Table 14 below were prepared in accordance with the synthetic routes in General Scheme 13 and Scheme 24 and 25, using procedures analogous to those described above.

**Table 14.**

| Ex | Structure | Name | Exact Mass [M+H]⁺ |
|---|---|---|---|
| Ex-7.1 | 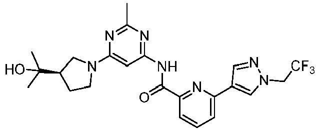 | *(R)*-*N*-(6-(3-(2-hydroxypropan-2-yl)pyrrolidin-1-yl)-2-methylpyrimidin-4-yl)-6-(1-(2,2,2-trifluoroethyl)-1H-pyrazol-4-yl)picolinamide | Calc'd 490, found 490 |
| Ex-7.2 | 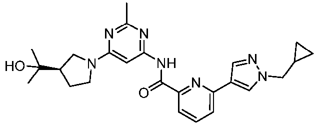 | *(R)*-6-(1-(cyclopropylmethyl)-1H-pyrazol-4-yl)-*N*-(6-(3-(2-hydroxypropan-2-yl)pyrrolidin-1-yl)-2-methylpyrimidin-4-yl)picolinamide | Calc'd 462, found 462 |
| Ex-7.3 | 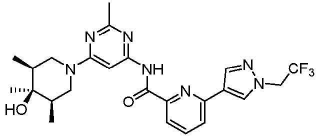 | *N*-(6-((3S,4s,5R)-4-hydroxy-3,4,5-trimethylpiperidin-1-yl)-2-methylpyrimidin-4-yl)-6-(1-(2,2,2-trifluoroethyl)-1H-pyrazol-4-yl)picolinamide | Calc'd 504, found 504 |
| Ex-7.4 | 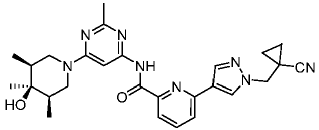 | 6-(1-((1-cyanocyclopropyl)methyl)-1H-pyrazol-4-yl)-*N*-(6-((3S,4s,5R)-4-hydroxy-3,4,5-trimethylpiperidin-1-yl)-2-methylpyrimidin-4-yl)picolinamide | Calc'd 501, found 501 |
| Ex-7.5 | 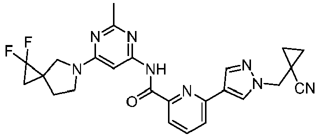 | 6-(1-((1-cyanocy clopropyl)methyl)-1H-pyrazol-4-yl)-*N*-(6-(1,1-difluoro-5-azaspiro[2.4]heptan-5-yl)-2-methylpyrimidin-4-yl)picolinamide | Calc'd 491, found 491 |
| Ex-7.6 | 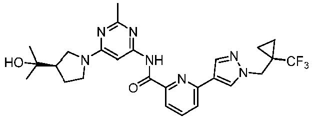 | *(R)*-*N*-(6-(3-(2-hydroxypropan-2-yl)pyrrolidin-1-yl)-2-methylpyrimidin-4-yl)-6-(1-((1-(trifluoromethyl)cyclopropyl)me thyl)-1H-pyrazol-4-yl)picolinamide | Calc'd 530, found 530 |
| Ex-7.7 | 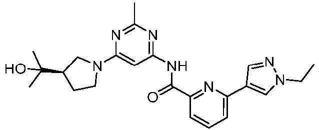 | *(R)*-6-(1-ethyl-1H-pyrazol-4-yl)-*N*-(6-(3-(2-hydroxypropan-2-yl)pyrrolidin-1-yl)-2-methylpyrimidin-4-yl)picolinamide | Calc'd 436, found 436 |
| Ex-7.8 | 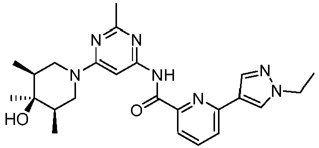 | 6-(1-ethyl-1H-pyrazol-4-yl)-*N-*(6-((3S,4s,5R)-4-hydroxy-3,4,5-trimethylpiperidin-1-yl)-2-methylpyrimidin-4-yl)picolinamide | Calc'd 450, found 450 |
| Ex-7.9 | 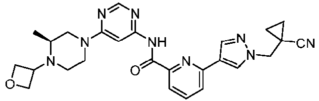 | *(S)*-6-(1-((1-cyanocy clopropyl)methyl)-1H-pyrazol-4-yl)-*N*-(6-(3-methyl-4-(oxetan-3-yl)piperazin-1-yl)pyrimidin-4-yl)picolinamide | Calc'd 500, found 500 |
| Ex 7.10 | 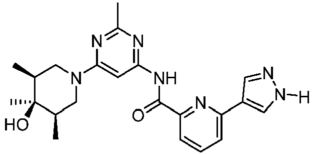 | *N*-(6-((3S,4s,5R)-4-hydroxy-3,4,5-trimethylpiperidin-1-yl)-2-methylpyrimidin-4-yl)-6-(1H-pyrazol-4-yl)picolinamide | Calc'd 422, found 422 |

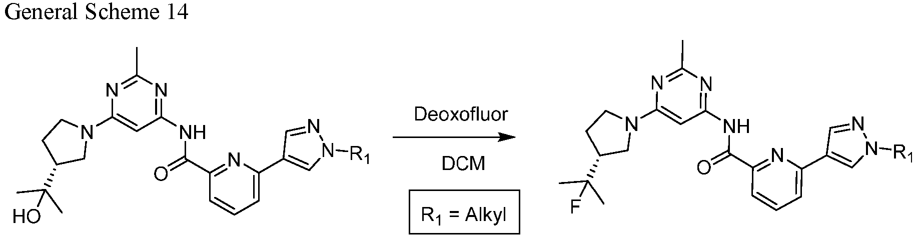

In General Scheme 14, picolinamide compounds 6.1 and 6.2 were transformed to the corresponding tertiary flouride compounds 8.1 and 8.2 using a commercially available flourinating agent. Representative preparative examples are described in more detail below.

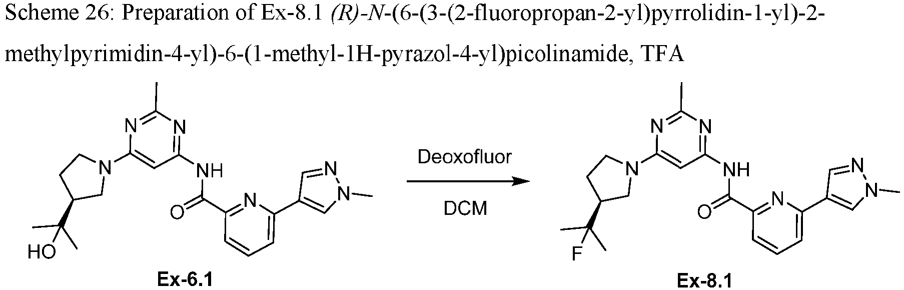

General Procedure: A 30 mL microwave vial was charged with compound 6.1 (15 mg, 0.03 mmol), DCM (276 µl), Deoxofluor (15.3 µl, 0.08 mmol), and the reaction mixture was stirred under inert conditions at RT for 2 hrs. At 2 hrs, the reaction mixture was quenched by addition of methanol (1 mL) and concentrated under reduced pressure. The mixture was diluted in DMSO (4 mL), filtered, and purified by mass-directed reverse phase chromatography eluting with MeCN/H₂O with 0.1% TFA with a linear gradient to afford the title compound Ex-8.1. ¹H NMR (600 MHz, DMSO-d6) δ 10.71 (s, 1H), 8.49 (s, 1H), 8.24 (s, 1H), 8.04 (t, *J =* 7.6 Hz, 1H), 7.94 (dd, *J =* 12.1, 7.9 Hz, 2H), 7.22 (s, 1H), 3.90 (s, 5H), 3.60 (s, 1H), 3.53 - 3.42 (m, 1H), 3.34 (s, 2H), 2.57 (s, 2H), 2.08 (d, *J* = 35.8 Hz, 1H), 1.86 (d, *J* = 55.1 Hz, 1H), 1.48 - 1.23 (m, 6H). MS (ESI) m/z 424 [(M+H)⁺ calc'd for C₂₂H₂₆FN₇O: 424].

Each of the elaborated picolinamides presented in Table 15 below were prepared in accordance with the synthetic routes in General Scheme 14 and Scheme 26, using procedures analogous to those described above.

**Table 15.**

| Ex | Structure | Name | Exact Mass [M+H]⁺ |
|---|---|---|---|
| Ex-8.1 | 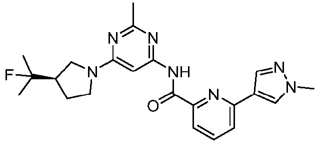 | *(R)*-*N*-(6-(3-(2-fluoropropan-2-yl)pyrrolidin-1-yl)-2-methylpyrimidin-4-yl)-6-(1-methyl-1H-pyrazol-4-yl)picolinamide | Calc'd 424, found 424 |
| Ex-8.2 | 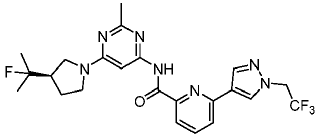 | *(R)*-*N*-(6-(3-(2-fluoropropan-2-yl)pyrrolidin-1-yl)-2-methylpyrimidin-4-yl)-6-(1-(2,2,2-trifluoroethyl)-1H-pyrazol-4-yl)picolinamide | Calc'd 492, found 492 |

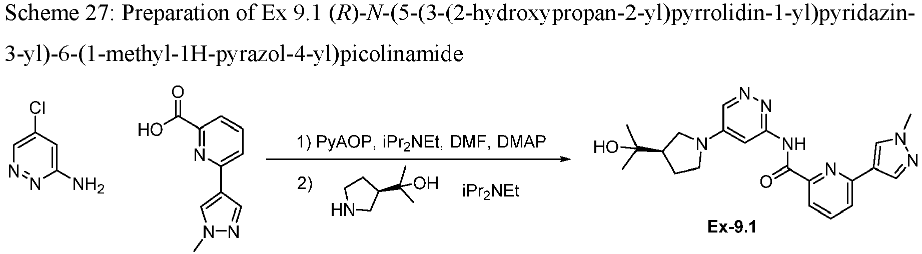

To a mixture of 6-(1-methyl-1H-pyrazol-4-yl)picolinic acid (40.6 mg, 0.2 mmol), PyAOP (104 mg, 0.2 mmol), 5-chloropyridazin-3-amine (25.9 mg, 0.2 mmol), and DMF (1 mL), was added iPr₂NEt (105 µL, 0.6 mmol), followed by DMAP (1 piece). The resulting solution was heated to 50 °C overnight, and then cooled to RT. To the reaction mixture was added (R)-2-(pyrrolidine-3-yl)propan-2-ol hydrochloride (66.3 mg, 0.4 mmol) and additional iPr₂NEt (35 µL, 0.2 mmol). The resulting solution was heated to 110 °C overnight, and then cooled to rt. The crude reaction mixture was diluted with DMSO, filtered, and purified by mass-directed reverse phase chromatography eluting with MeCN/H₂O with 0.1% TFA with a linear gradient to afford the title compound 9.1. ¹H NMR (600 MHz, DMSO-d6) δ 11.29 (s, 1H), 8.62 (s, 1H), 8.59 (s, 1H), 8.38 (s, 1H), 8.10 (t, *J =* 7.7 Hz, 1H), 8.02 (d, *J =* 7.8 Hz, 1H), 7.99 (d, *J* = 7.5 Hz, 1H), 7.52 (d, *J* = 17.4 Hz, 1H), 3.94 (s, 3H), 3.92 - 3.81 (m, 1H), 3.69 - 3.57 (m, 1H), 3.59 - 3.51 (m, 1H), 3.50 - 3.36 (m, 1H), 2.40 (s, 1H), 2.10 - 2.02 (m, 1H), 1.96 (s, 1H), 1.19 (s, 3H), 1.17 (s, 3H). MS (ESI) *m*/*z* C₂₁H₂₆N₇O₂ [M+H]⁺ calc'd 408, found 408

**Table 16.**

| Ex | Structure | Name | Exact Mass [M+H]⁺ |
|---|---|---|---|
| Ex-9.1 | 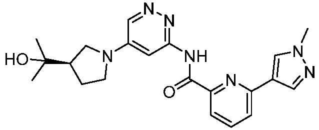 | *(R)*-*N*-(5-(3-(2-hydroxypropan-2-yl)pyrrolidin-1-yl)pyridazin-3-yl)-6-(1-methyl-1H-pyrazol-4-yl)picolinamide | Calc'd 408, found 408 |

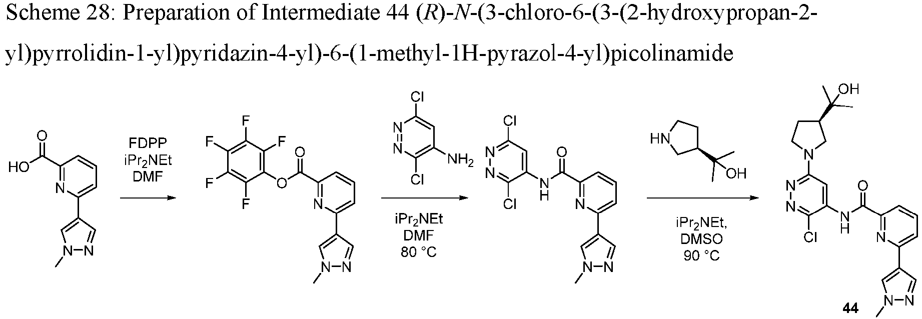

### Step 1: Preparation of perfluorophenyl 6-(1-methyl-1H-pyrazol-4-yl)picolinate

To a mixture of 6-(1-methyl-1H-pyrazol-4-yl)picolinic acid (250 mg, 1.23 mmol), FDPP (567 mg, 1.48 mmol), and DMF (4.1 mL) was added iPr₂NEt (1 mL, 5.7 mmol). The resulting solution was stirred at RT for 3 Hr(s). To the reaction mixture was added water, at which point precipitate was observed. The suspension was filtered, and the filter cake was dried to afford the title compound. MS (ESI) *m*/*z* C₁₆H₉F₅N₃O₂ [M+H]⁺ calc'd 370, found 370.

### Step 2: Preparation of N-(3,6-dichloropyridazin-4-yl)-6-(1-methyl-1H-pyrazol-4-yl)picolinamide

To a mixture of 3,6-dichloropyridazin-4-amine (100 mg, 0.61 mmol), perfluorophenyl 6-(1-methyl-1H-pyrazol-4-yl)picolinate (236 mg, 0.64 mmol), and DMF (3.0 mL) was added iPr₂NEt (0.21 mL, 1.2 mmol). The resulting solution was heated to 80 °C for 8 Hr(s), and then cooled to RT, at which point CH₂Cl₂ and water were added. The phases were separated, and the organic layer was washed twice with brine, dried over MgSO₄, filtered, and concentrated to dryness *in vacuo* to afford the title compound. MS (ESI) *m*/*z* C₁₄H₁₀Cl₂N₆O [M+H]⁺ calc'd 349, found 349.

### Step 3: Preparation of (R)-N-(3-chloro-6-(3-(2-hydroxypropan-2-yl)pyrrolidin-1-yl)pyridazin-4-yl)-6-(1-methyl-1H-pyrazol-4-yl)picolinamide

To a mixture of *N*-(3,6-dichloropyridazin-4-yl)-6-(1-methyl-1H-pyrazol-4-yl)picolinamide (50 mg, 0.14 mmol), (*R*)-2-(pyrrolidine-3-yl)propan-2-ol hydrochloride (28.5 mg, 0.17 mmol), and DMSO (2.0 mL) was added iPr₂NEt (50 µL, 0.29 mmol). The resulting solution was heated to 90 °C for 20 Hr(s), and then cooled to RT, at which point EtOAc and water were added. The phases were separated, and the organic layer was washed twice with water, twice with brine, dried over MgSO₄, filtered, and concentrated *in vacuo.* The crude residue was purified purified by mass-directed reverse phase chromatography eluting with MeCN/H₂O with 0.1% TFA with a linear gradient to afford the title compound 44. MS (ESI) *m*/*z* C₂₁H₂₅ClN₇O₂ [M+H]⁺ calc'd 442, found 442.

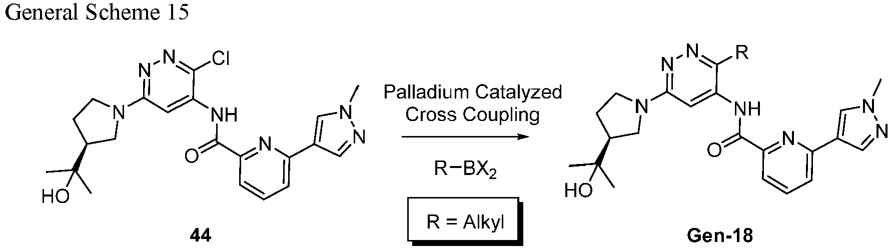

In General Scheme 15, picolinamide 44 was transformed under palladium-catalyzed coupling with either commercially available or synthetically prepared boronates to afford elaborated compounds of the form Gen 18. Representative preparative examples are described in more detail below.

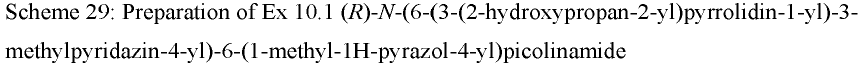

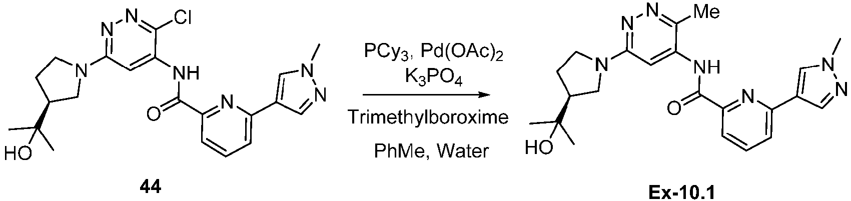

To a mixture of PCy₃ (1.9 mg, 0.068 mmol), Pd(OAc)₂ (0.76 mg, 0.034 mmol), K₃PO₄ (21.6 mg, 0.10 mmol), trimethylboroxine (12.8 mg, 0.10 mmol), 44 (15 mg, 0.034 mmol), and PhMe (2.0 mL) was added water (0.1 mL). The vessel was evacuated and back-filled with an atmosphere of N₂ thrice. The resulting mixture was heated to 100 °C for 2 Hr(s), and then cooled to RT. The crude reaction mixture was concentrated to dryness *in vacuo.* The residue was re-dissolved in DMF, filtered, and purified purified by mass-directed reverse phase chromatography eluting with MeCN/H₂O with 0.1% TFA with a linear gradient to afford the title compound 10.1. ¹H NMR (400 MHz, CDCl₃) δ 10.80 (s, 1H), 8.33 (s, 1H), 8.07 (d, *J =* 7.28 Hz, 1H), 8.02 (s, 1H), 7.96 (t, *J = 7.72* Hz, 1H), 7.91 (s, 1H), 7.74 (d, *J* = 7.72 Hz, 1H), 4.04 (s, 3H), 3.95 - 3.83 (m, 2H), 3.73 - 3.61 (m, 1H), 2.73 (s, 3H), 2.55 - 2.45 (m, 3H), 2.20 - 2.04 (m, 2H), 1.34 (s, 3H), 1.32 (s, 3H). MS (ESI) *m*/*z* C₂₂H₂₈N₇O₂ [M+H]⁺ calc'd 422, found 422

Each of the elaborated picolinamides presented in Table 17 below were prepared in accordance with the synthetic routes in General Scheme 15 and Scheme 29, using procedures analogous to those described above.

**Table 17.**

| Ex | Structure | Name | Exact Mass [M+H]⁺ |
|---|---|---|---|
| Ex-10.1 | 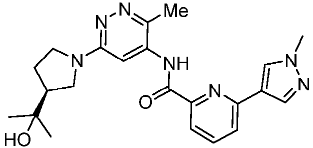 | *N*-[6-[(3*R*)-3-(1-hydroxy-1-methyl-ethyl)pyrrolidin-1-yl]-3-methyl-pyridazin-4-yl]-6-(1-methylpyrazol-4-yl)pyridine-2-carboxamide | Calc'd 422, found 422 |
| Ex-10.2 | 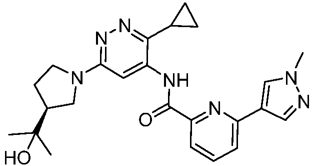 | *N*-[3-cyclopropyl-6-[(3*R*)-3-(1-hydroxy-1-methylethyl)pyrrolidin-1-yl]pyridazin-4-yl]-6-(1-methylpyrazol-4-yl)pyridine-2-carboxamide | Calc'd 448, found 448 |

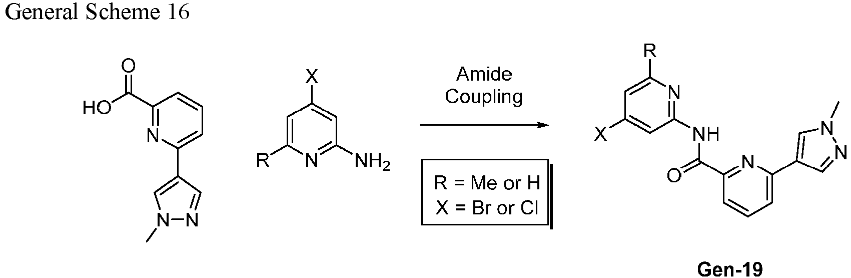

In General Scheme 16, 6-(1-methyl-1H-pyrazol-4-yl)picolinic acid or 6-(1-(cyclopropylmethyl)-1H-pyrazol-4-yl)picolinic acid was condensed with either commercially available or synthetically prepared 4-bromo-pyridin-2-amine, 4-bromo-6-methylpyridin-2-amine, or 4-chloro-6-methylpyridin-2-amine to afford bromo or chloro picolinamides of the form Gen 19. The representative compounds are described in more detail below.

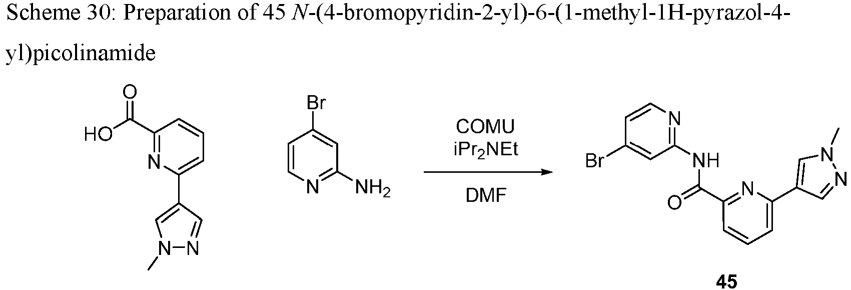

To a mixture of 6-(1-methyl-1H-pyrazol-4-yl)picolinic acid (102 mg, 0.5 mmol), 4-bromopyridin-2-amine (87 mg, 0.5 mmol), COMU (214 mg, 0.5 mmol), and DMF (2.5 mL) was added iPr₂NEt (262 µL, 1.5 mmol). The resulting solution was stirred at RT overnight. The crude reaction mixture was diluted with DMSO, filtered, and purified purified by mass-directed reverse phase chromatography eluting with MeCN/H₂O with 0.1% TFA with a linear gradient to afford the title compound 45. MS (ESI) *m*/*z* C₁₅H₁₂BrN₅O [M+H]⁺ calc'd 358, found 358.

Each of the elaborated picolinamides presented in Table 18 below were prepared in accordance with the synthetic routes in General Scheme 16, using procedures analogous to those described above.

**Table 18.**

| Intermediate | Structure |
|---|---|
| 45 | 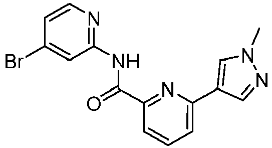 |
| 46 | 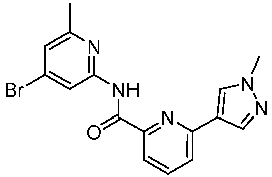 |

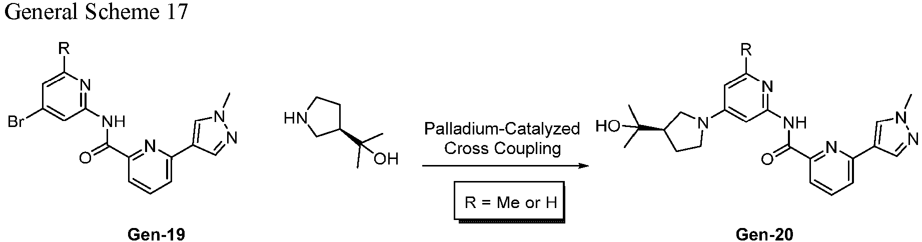

In General Scheme 17 picolinamides Gen 19 were transformed under palladium-catalyzed Buchwald-Hartwig coupling with (*R*)-2-(pyrrolidine-3-yl)propan-2-ol hydrochloride to afford elaborated compounds of the form Gen 20. Representative preparative examples are described in more detail below.

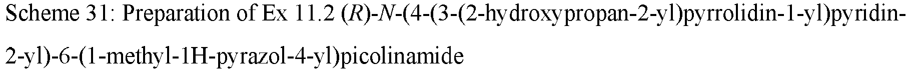

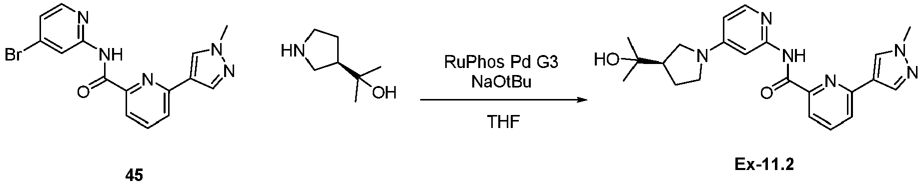

To a mixture of RuPhos Pd G3 (9.2 mg, 0.011 mmol), (*R*)-2-(pyrrolidine-3-yl)propan-2-ol hydrochloride (18.2 mg, 0.11 mmol) under an atmosphere of N₂ was added a suspension of 45 (25.9 mg, 0.055 mmol) in THF (1.2 mL). The resulting suspension was heated to 80 °C overnight, and then cooled to RT. The crude reaction mixture was diluted with 1:1 DMSO/MeOH, filtered, and purified purified by mass-directed reverse phase chromatography eluting with MeCN/H₂O with 0.1% TFA with a linear gradient to afford the title compound 11.2. ¹H NMR (600 MHz, DMSO-d6) δ 13.35 (s, 1H), 11.24 (d, *J =* 6.7 Hz, 1H), 8.54 (s, 1H), 8.34 (s, 1H), 8.07 - 8.01 (m, 2H), 7.98 - 7.92 (m, 2H), 7.26 (d, *J =* 26.5 Hz, 1H), 6.71 (dd, *J =* 33.7, 5.5 Hz, 1H), 3.90 (s, 2H), 3.73 - 3.66 (m, 1H), 3.59 (q, *J =* 10.1 Hz, 1H), 3.49 (t, *J =* 9.0 Hz, 1H), 3.45 - 3.38 (m, 1H), 3.36 (t, *J =* 10.2 Hz, 1H), 2.40 - 2.32 (m, 1H), 2.04 - 1.96 (m, 1H), 1.95 - 1.86 (m, 1H), 1.16 - 1.10 (m, 6H). MS (ESI) *m*/*z* C₂₂H₂₆N₆O₂ [M+H]⁺ calc'd 407, found 407

Each of the elaborated picolinamides presented in Table 19 below were prepared in accordance with the synthetic routes in General Scheme 17 using procedures analogous to those described above.

**Table 19.**

| Ex | Structure | Name | Exact Mass [M+H]⁺ |
|---|---|---|---|
| Ex-11.1 | 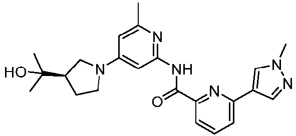 | *(R)*-*N-*(4-(3-(2-hydroxypropan-2-yl)pyrrolidin-1-yl)-6-methylpyridin-2-yl)-6-(1-methyl-1H-pyrazol-4-yl)picolinamide | Calc'd 421, found 421 |
| Ex-11.2 | 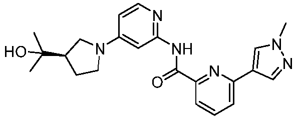 | *(R)*-*N*-(4-(3-(2-hydroxypropan-2-yl)pyrrolidin-1-yl)pyridin-2-yl)-6-(1-methyl-1H-pyrazol-4-yl)picolinamide | Calc'd 407, found 407 |
| **Ex-11.3** | 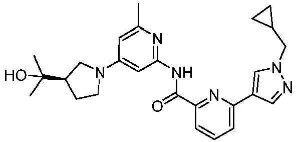 | (*R*)-6-(1-(cyclopropylmethyl)-1H-pyrazol-4-yl)-*N*-(4-(3-(2-hydroxypropan-2-yl)pyrrolidin-1-yl)-6-methylpyridin-2-yl)picolinamide | Calc'd 461, found 461 |
| **Ex-11.4** | 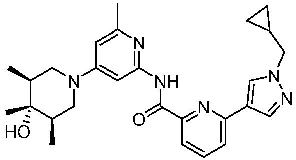 | 6-(1-(cyclopropylmethyl)-1H-pyrazol-4-yl)-*N*-(4-((3*S*,4*r*,5*R*)-4-hydroxy-3,4,5-trimethylpiperidin-1-yl)-6-methylpyridin-2-yl)picolinamide | Calc'd 475, found 475 |

The compounds of the invention, surprisingly and advantageously, exhibit good potency as inhibitors of LRRK2 kinase. The IC50 values reported herein were measured as follows.

### Biological Assay: LRRK2 Kₘ ATP LanthaScreen^{™} Assay

Compound potency against LRRK2 kinase activity was determined using LanthaScreen^{™} technology from Life Technologies Corporation (Carlsbad, CA) using a GST20 tagged truncated human mutant G2019S LRRK2 in the presence of the fluorescein-labeled peptide substrate LRRKtide^{®} (LRRK2 phosphorylated ezrin/radixin/moesin (ERM)), also from Life Technologies. The data presented for the Kₘ ATP LanthaScreen^{™} Assay represents mean IC₅₀ values based on several test results and may have reasonable deviations depending on the specific conditions and reagents used. Kₘ is the Michaelis constant of an enzyme and is defined as the concentration of native substrate (ATP for a kinase) which permits the enzyme to achieve half Vₘₐₓ (Vₘₐₓ = rate of reaction when the enzyme is saturated with substrate). IC₅₀ (half-maximal inhibitory concentration) represents the concentration of inhibitor required to inhibit LRRK2 kinase activity by 50%. Assays were performed in the presence of 134 µM ATP (Kₘ ATP). Upon completion, the assay was stopped, and phosphorylated substrate detected with a terbium (Tb)-labeled anti-pERM antibody (cat. no. PV4898). The compound dose response was prepared by diluting a 10 mM stock of compound to a maximum concentration of 9.99 µM in 100% DMSO, followed by custom fold serial dilution in DMSO nine times. 20 nL of each dilution was spotted via a Labcyte Echo onto a 384-well black-sided plate (Corning 3575) followed by 15 µl of a 1.25 nM enzyme solution in 1× assay buffer (50 mM Tris pH 8.5, 10 mM MgCl₂, 0.01% Brij-35, 1 mM EGTA, 2 mM dithiothreitol, 0.05 mM sodium orthovanadate). Following a 15-min incubation period at RT, the kinase reaction was started with the addition of 5 µL of 400 nM fluorescein-labeled LRRKtide^{®} (LRRK2 phosphorylated ezrin/radixin/moesin (ERM)) peptide substrate and 134 µM ATP solution in 1× assay buffer. The reaction was allowed to progress at ambient temperature for 90 min. The reaction was then stopped by the addition of 20 µL of TR-FRET Dilution Buffer (Life Technologies, Carlsbad, CA) containing 2 nM Tb-labeled anti-phospho LRRKtide^{®} (LRRK2 phosphorylated ezrin/radixin/moesin (ERM)) antibody and 10 mM EDTA (Life Technologies, Carlsbad, CA). After an incubation period of 1 h at RT, the plate was read on an EnVision^{®} multimode plate reader (Perkin Elmer, Waltham, MA) with an excitation wavelength of 337 nm (Laser) and a reading emission at both 520 and 495 nm. Compound IC₅₀ values were interpolated from nonlinear regression best-fits of the log of the final compound concentration, plotted as a function of the 520/495-nm emission ratio using activity base "Abase"). Abase uses a 4 parameter (4P) logistic fit based on the Levenberg-Marquardt algorithm. The pIC₅₀ values set forth in Table 20 below were derived from the IC₅₀ values (in molar concentration) and represent the negative logarithm of these values. "Ex" column in Table 7 corresponds to the example number of the compounds in the examples and tables above.

**Table 20.**

| Ex | LRRK2 IC₅₀ |
|---|---|
| Ex-1.1 | 8.90 |
| Ex-1.2 | 23.8 |
| Ex-1.3 | 192 |
| Ex-1.4 | 10.4 |
| Ex-1.5 | 8.59 |
| Ex-1.6 | 10.0 |
| Ex-1.7 | 41.6 |
| Ex-1.8 | 6.36 |
| Ex-1.9 | 10.7 |
| Ex-1.10 | 1.04 |
| Ex-1.11 | 1.54 |
| Ex-1.12 | 1.93 |
| Ex-1.13 | 1.60 |
| Ex-1.14 | 2.06 |
| Ex-1.15 | 1.78 |
| Ex-1.16 | 0.87 |
| Ex-1.17 | 5.10 |
| Ex-1.18 | 2.06 |
| Ex-1.19 | 11.6 |
| Ex-1.20 | 5.34 |
| Ex-1.21 | 6.89 |
| Ex-1.22 | 1.62 |
| Ex-1.23 | 0.77 |
| Ex-1.24 | 1.20 |
| Ex-1.25 | 1.65 |
| Ex-1.26 | 1.00 |
| Ex-1.27 | 1.02 |
| Ex-1.28 | 1.74 |
| Ex-1.29 | 3.67 |
| Ex-1.30 | 5.61 |
| Ex-1.31 | 17.6 |
| Ex-1.32 | 5.78 |
| Ex-1.33 | 3.04 |
| Ex-1.34 | 52.2 |
| Ex-1.35 | 9.58 |
| Ex-1.36 | 0.08 |
| Ex-1.37 | 1.73 |
| Ex-1.38 | 0.62 |
| Ex-1.39 | 1.08 |
| Ex-1.40 | 1.28 |
| Ex-1.41 | 8.15 |
| Ex-1.42 | 40.5 |
| Ex-1.43 | 2.69 |
| Ex-1.44 | 12.8 |
| Ex-1.45 | 9.90 |
| Ex-1.46 | 5.46 |
| Ex-1.47 | 5.74 |
| Ex-1.48 | 19.3 |
| Ex-1.49 | 15.1 |
| Ex-1.50 | 51.6 |
| Ex-1.51 | 38.1 |
| Ex-1.52 | 68.9 |
| Ex-1.53 | 40.2 |
| Ex-1.54 | 22.9 |
| Ex-1.55 | 41.2 |
| Ex-1.56 | 9.42 |
| Ex-1.57 | 13.8 |
| Ex-1.58 | 11.3 |
| Ex-1.59 | 5.15 |
| Ex-1.60 | 17.3 |
| Ex-1.61 | 3.84 |
| Ex-1.62 | 13.7 |
| Ex-1.63 | 74.8 |
| Ex-1.64 | 2.05 |
| Ex-1.65 | 1.04 |
| Ex-1.66 | 2.66 |
| Ex-1.67 | 10.8 |
| Ex-1.68 | 59.9 |
| Ex-1.69 | 2.68 |
| Ex-1.70 | 124 |
| Ex-1.71 | 96.9 |
| Ex-1.72 | 3.85 |
| Ex-1.73 | 3.31 |
| Ex-1.74 | 1.92 |
| Ex-1.75 | 0.82 |
| Ex-1.76 | 1.09 |
| Ex-1.77 | 1.41 |
| Ex-1.78 | 26.8 |
| Ex-1.79 | 3.69 |
| Ex-1.80 | 13.5 |
| Ex-1.81 | 7.04 |
| Ex-1.82 | 5.91 |
| Ex-1.83 | 17.3 |
| Ex-2.1 | 1.62 |
| Ex-2.2 | 3.68 |
| Ex-2.3 | 5.46 |
| Ex-2.4 | 3.50 |
| Ex-2.5 | 0.91 |
| Ex-2.6 | 13.6 |
| Ex-2.7 | 10.3 |
| Ex-2.8 | 32.1 |
| Ex-2.9 | 3.07 |
| Ex-3.1 | 58.7 |
| Ex-3.2 | 1.43 |
| Ex-4.1 | 4.24 |
| Ex-4.2 | 1.63 |
| Ex-4.3 | 9.11 |
| Ex-4.4 | 6.34 |
| Ex-4.5 | 12.7 |
| Ex-4.6 | 9.69 |
| Ex-4.7 | 2.18 |
| Ex-4.8 | 4.76 |
| Ex-4.9 | 9.96 |
| Ex-4.10 | 5.38 |
| Ex-4.11 | 14.3 |
| Ex-4.12 | 28.1 |
| Ex-4.13 | 9.18 |
| Ex-4.14 | 19.9 |
| Ex-4.15 | 0.80 |
| Ex-4.16 | 2.34 |
| Ex-4.17 | 4.11 |
| Ex-4.18 | 1.52 |
| Ex-4.19 | 2.13 |
| Ex-4.20 | 3.43 |
| Ex-4.21 | 4.04 |
| Ex-4.22 | 1.64 |
| Ex-4.23 | 0.62 |
| Ex 4.24 | 5.19 |
| Ex 4.25 | 9.36 |
| Ex-5.1 | 6.78 |
| Ex-5.2 | 0.70 |
| Ex-5.3 | 1.13 |
| Ex-5.4 | 7.87 |
| Ex-5.5 | 8.7 |
| Ex-5.6 | 1.6 |
| Ex-5.7 | 8.02 |
| Ex-5.8 | 4.11 |
| Ex-5.9 | 2.07 |
| Ex-5.10 | 8.66 |
| Ex-5.11 | 1.64 |
| Ex-5.12 | 2.35 |
| Ex-6.1 | 5.16 |
| Ex-6.2 | 8.45 |
| Ex-6.3 | 28.0 |
| Ex-6.4 | 20.6 |
| Ex-6.5 | 23.6 |
| Ex-6.6 | 13.2 |
| Ex-6.7 | 294 |
| Ex-6.8 | 24.6 |
| Ex-6.9 | 14.4 |
| Ex-6.10 | 179 |
| Ex-6.11 | 2.01 |
| Ex-6.12 | 1.36 |
| Ex-6.13 | 3.63 |
| Ex-6.14 | 3.49 |
| Ex-6.15 | 6.04 |
| Ex-6.16 | 2.58 |
| Ex-6.17 | 11.8 |
| Ex-6.18 | 21.3 |
| Ex-6.19 | 178 |
| Ex-6.20 | 7.49 |
| Ex-6.21 | 30.2 |
| Ex-6.22 | 36.5 |
| Ex-6.23 | 11.2 |
| Ex-7.1 | 4.69 |
| Ex-7.2 | 8.23 |
| Ex-7.3 | 82.4 |
| Ex-7.4 | 0.67 |
| Ex-7.5 | 4.76 |
| Ex-7.6 | 8.59 |
| Ex-7.7 | 8.79 |
| Ex-7.8 | 29.1 |
| Ex-7.9 | 0.65 |
| Ex 7.10 | 20.2 |
| Ex-8.1 | 2.57 |
| Ex-8.2 | 1.83 |
| Ex-9.1 | 46.1 |
| Ex-10.1 | 10.7 |
| Ex-10.2 | 0.774 |
| Ex-11.1 | 7.85 |
| Ex-11.2 | 8.70 |
| Ex-11.3 | 7.27 |
| Ex-11.4 | 25.4 |

While the invention has been described and illustrated with reference to certain particular embodiments thereof, those skilled in the art will appreciate that various adaptations, changes, modifications, substitutions, deletions, or additions of procedures and protocols may be made without departing from the scope of the invention. For example, effective dosages other than the particular dosages as set forth herein above may be applicable as a consequence of variations in the responsiveness of the mammal being treated for any of the indications with the compounds of the invention indicated above. Likewise, the specific pharmacological responses observed may vary according to and depending upon the particular active compounds selected or whether there are present pharmaceutical carriers, as well as the type of formulation and mode of administration employed, and such expected variations or differences in the results are contemplated in accordance with the objects and practices of the present invention. It is intended, therefore, that the invention be defined by the scope of the claims which follow and that such claims be interpreted as broadly as is reasonable.

## Claims

1. A compound having a structural Formula (I): 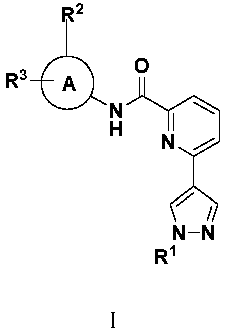 or a pharmaceutically acceptable salt thereof, wherein:
A is a nitrogen containing heteroaryl selected from pyridyl, pyrimidinyl and pyridazinyl;
R¹ is selected from hydrogen, C₁₋₆ alkyl, C₁₋₆ haloalkyl, and -(CH₂)ₙC₃₋₆ cycloalkyl, said alkyl and cycloalkyl optionally substituted with 1 to 3 groups selected from CF₃ and CN;
R² is an N-linked heterocycloalkyl selected from a group consisting of pyrrolidinyl, piperidinyl, piperazinyl, azetidinyl, morpholinyl, tetrahydrofuronyl, azabicylohexanyl, azabicyloheptanyl, azaspiroheptanyl, azaspirononanyl, diazaspirononanyl, and azaspirooctanyl, said pyrrolidinyl, piperidinyl, piperazinyl, azetidinyl, morpholinyl, tetrahydrofuronyl, azabicylohexanyl, azabicyloheptanyl, azaspiroheptanyl, azaspirononanyl, diazaspirononanyl, and azaspirooctanyl, unsubstituted or substituted with 1 to 4 groups independently selected from R⁴;
R³ is selected from hydrogen, CH₃, CF₃, and cyclopropyl;
each R⁴ is independently selected from the group consisting of C₁₋₆ alkyl, C₁₋₆ alkylOH, C₁₋₆ alkylCN, OC₁₋₆ alkyl, C₁₋₆ haloalkyl, C₃₋₆ cycloalkyl, oxetanyl, oxo, OH, halogen, said oxetanyl optionally substituted with 1 to 3 CH₃ groups; and
n is selected from 0 to 3.

2. The compound according to claim 1 or a pharmaceutically acceptable salt thereof, wherein A pyridyl.

3. The compound according to claim 1 or a pharmaceutically acceptable salt thereof, wherein A is pyrimidinyl.

4. The compound according to claim 1 or a pharmaceutically acceptable salt thereof, wherein A is pyridazinyl.

5. The compound according to any one of claims 1 to 3 or a pharmaceutically acceptable salt thereof, wherein R¹ is hydrogen, CH₃, CH₂CH₃, CH₂CF₃, CH(CH₃)CF₃, - (CH₂)ₙcyclopropyl, or -(CH₂)ₙcyclobutyl, said CH₃, CH₂CH₃, CH₂CF₃, CH(CH₃)CF₃, - (CH₂)ₙcyclopropyl, or -(CH₂)ₙcyclobutyl optionally substituted with 1 to 3 groups selected from CN and CF₃.

6. The compound according to any one of claims 1 to 5 or a pharmaceutically acceptable salt thereof, wherein R² is selected from the group consisting of pyrrolidinyl, piperidinyl, piperazinyl, azetidinyl, morpholinyl and tetrahydrofuronyl, said group unsubstituted or substituted with 1 to 4 groups independently selected from R⁴.

7. The compound according to any one of claims 1 to 6 or a pharmaceutically acceptable salt thereof wherein R² is pyrrolidinyl, unsubstituted or substituted with 1 to 4 groups independently selected from R⁴.

8. The compound according to any one of claims 1 to 6 or a pharmaceutically acceptable salt thereof wherein R² is piperidinyl or piperazinyl, unsubstituted or substituted with 1 to 4 groups independently selected from R⁴.

9. The compound according to any one of claims 1 to 5 or a pharmaceutically acceptable salt thereof wherein R² is selected from the group consisting of azabicylohexanyl, azabicyloheptanyl, azaspiroheptanyl, azaspirononanyl, diazaspirononanyl, and azaspirooctanyl, said group unsubstituted or substituted with 1 to 4 groups independently selected from R⁴.

10. The compound according to any one of claims 1 to 5 and 9 or a pharmaceutically acceptable salt thereof wherein R² is selected from the group consisting of azabicylohexanyl, azabicyloheptanyl, and azaspiroheptanyl, said group unsubstituted or substituted with 1 to 4 groups independently selected from R⁴.

11. The compound according to any one of claims 1 to 10 wherein R⁴ is independently selected from the group consisting of hydrogen, CH₃, OCH₃, C(CH₃)₂OH, C(CH₃)₂CN, CF₃, CH₂F, C(CH3)₂F, cyclopropyl, oxetanyl, oxo, OH, and fluoro, said oxetanyl optionally substituted with 1 to 3 groups of CH₃.

12. The compound according to claim 1 represented by structural Formula II: 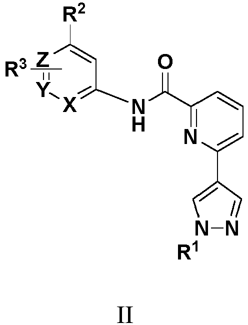 or a pharmaceutically acceptable salt thereof, wherein X, Y, and Z are represented and selected from the group consisting of:
**1)** X=Z=N, and Y=CH or C-CH3;
**2)** Y=N, Z=C, and X=C-CH3, C-CF3, or C-cyclopropyl;
**3)** Z=N, Y=C, and X= C-CH3, C-CF3, or C-cyclopropyl;
**4)** Y=Z=N and X=C-CH3 or C-cyclopropyl;
**5)** X=Y=N= and Z=CH; and
**6)** Z=CH, Y=CH or C-CH3 and X=N.

13. The compound according to claim 12 or a pharmaceutically acceptable salt thereof
wherein when X, Y, and Z combined to form pyridyl selected from the group consisting of 2), 3), 5) and 6), R¹ is selected from the group consisting of CH₃, CH₂CH₃, CH₂CF₃, CH(CH₃)CF₃, - (CH₂)ₙcyclopropyl, and -(CH₂)ₙcyclobutyl, said CH₃, CH₂CH₃, CH₂CF₃, CH(CH₃)CF₃, - (CH₂)ₙcyclopropyl, and -(CH₂)ₙcyclobutyl optionally substituted with 1 to 3 groups selected from CN and CF₃; R² is selected from pyrrolidinyl, piperidinyl, piperazinyl, azetidinyl, morpholinyl and tetrahydrofuronyl, R³ is selected from the group consisting of hydrogen, CH₃, CF₃ and cyclopropyl; and each R⁴ is independently selected from the group consisting of hydrogen, CH₃, OCH₃, C(CH₃)₂OH, C(CH₃)₂CN, CF₃, CH₂F, C(CH3)₂F, cyclopropyl, oxetanyl, oxo, OH, and fluoro, said oxetanyl optionally substituted with 1 to 3 CH₃ groups.

14. The compound according to claim 12 wherein X, Y, and Z combined to form pyrimidinyl represented by group 1).

15. The compound according to claim 12 wherein X, Y, and Z combined to form pyridazinyl represented by group 4).

16. The compound of claim 1, or a pharmaceutically acceptable salt thereof, wherein said compound is selected from:
| |
|---|
| (*R*)-*N*-(5-(3-(2-hydroxypropan-2-yl)pyrrolidin-1-yl)-2-(trifluoromethyl)pyridin-3-yl)-6-(1-methyl-1H-pyrazol-4-yl)picolinamide |
| *N*-(5-(4-(2-hydroxypropan-2-yl)-2-azabicyclo[2.1.1]hexan-2-yl)-2-(trifluoromethyl)pyridin-3-yl)-6-(1-methyl-1H-pyrazol-4-yl)picolinamide |
| *N*-(5-((3S,4s,5R)-4-hydroxy-3,4,5-trimethylpiperidin-1-yl)-2-(trifluoromethyl)pyridin-3-yl)-6-(1-(2,2,2-trifluoroethyl)-1H-pyrazol-4-yl)picolinamide |
| (*R*)-*N*-(5-(3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide |
| (*R*)-6-(1-(cyclopropylmethyl)-1H-pyrazol-4-yl)-N-(5-(3-(2-hydroxypropan-2-yl)pyrrolidin-1-yl)-2-(trifluoromethyl)pyridin-3-yl)picolinamide |
| *N*-(5-(3-methoxyazetidin-1-yl)-2-(trifluoromethyl)pyridin-3-yl)-6-(1-(2,2,2-trifluoroethyl)-1H-pyrazol-4-yl)picolinamide |
| (*R*)-*N*-(5-(3-(fluoromethyl)pyrrolidin-1-yl)-2-(trifluoromethyl)pyridin-3-yl)-6-(1-methyl-1H-pyrazol-4-yl)picolinamide |
| *N*-(5-(3-(2-hydroxypropan-2-yl)-3-methylpyrrolidin-1-yl)-2-(trifluoromethyl)pyridin-3-yl)-6-(1-methyl-1H-pyrazol-4-yl)picolinamide |
| *N*-(5-(3-hydroxypyrrolidin-1-yl)-2-(trifluoromethyl)pyridin-3-yl)-6-(1-methyl-1H-pyrazol-4-yl)picolinamide |
| (*S*)-*N*-(5-(3-methoxypyrrolidin-1-yl)-2-(trifluoromethyl)pyridin-3-yl)-6-(1-(2,2,2-trifluoroethyl)-1H-pyrazol-4-yl)picolinamide |
| *N*-(5-(4-(2-hydroxypropan-2-yl)-2-azabicyclo[2.1.1]hexan-2-yl)-2-(trifluoromethyl)pyridin-3-yl)-6-(1-(2,2,2-trifluoroethyl)-1H-pyrazol-4-yl)picolinamide |
| (*R*) or (*S*)-*N*-(5-(3-hydroxy-3-methylpyrrolidin-1-yl)-2-(trifluoromethyl)pyridin-3-yl)-6-(1-methyl-1H-pyrazol-4-yl)picolinamide |
| (*R*) or (*S*)-*N*-(5-(3-hydroxy-3-methylpyrrolidin-1-yl)-2-(trifluoromethyl)pyridin-3-yl)-6-(1-methyl-1H-pyrazol-4-yl)picolinamide |
| *N*-(5-((2*R*,3*R*)-3-hydroxy-2-methylpyrrolidin-1-yl)-2-(trifluoromethyl)pyridin-3-yl)-6-(1-(2,2,2-trifluoroethyl)-1H-pyrazol-4-yl)picolinamide |
| *N*-(5-((2*R*,3*S*)-3-hydroxy-2-methylpyrrolidin-1-yl)-2-(trifluoromethyl)pyridin-3-yl)-6-(1-(2,2,2-trifluoroethyl)-1H-pyrazol-4-yl)picolinamide |
| (*R*) or *(S) N*-(5-(3-hydroxy-3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide |
| (*R*) or (*S*) *N*-(5-(3-hydroxy-3-(2-hydroxypropan-2-yl)pyrrolidin-1-yl)-2-(trifluoromethyl)pyridin-3-yl)-6-(1-(2,2,2-trifluoroethyl)-1H-pyrazol-4-yl)picolinamide |
| (*R, S*) or (*S, R*) *N*-(5-(3-(2-hydroxypropan-2-yl)-4-methylpyrrolidin-1-yl)-2-(trifluoromethyl)pyridin-3-yl)-6-(1-(2,2,2-trifluoroethyl)-1H-pyrazol-4-yl)picolinamide |
| (*R, S*) or (*S, R*) *N*-(5-(3-(2-hydroxypropan-2-yl)-4-methylpyrrolidin-1-yl)-2 (trifluoromethyl)pyridin-3-yl)-6-(1-(2,2,2-trifluoroethyl)-1H-pyrazol-4-yl)picolinamide |
| (*R*) or (*S*) *N*-(5-(3-(2-hydroxypropan-2-yl)-3-methylpyrrolidin-1-yl)-2-(trifluoromethyl)pyridin-3-yl)-6-(1-(2,2,2-trifluoroethyl)-1H-pyrazol-4-yl)picolinamide |
| (*R*) or (*S*) *N*-(5-(3-(2-hydroxypropan-2-yl)-3-methylpyrrolidin-1-yl)-2-(trifluoromethyl)pyridin-3-yl)-6-(1-(2,2,2-trifluoroethyl)-1H-pyrazol-4-yl)picolinamide |
| *(R)* or *(S) N*-(5-(3-hydroxypyrrolidin-1-yl)-2-(trifluoromethyl)pyridin-3-yl)-6-(1-(2,2,2-trifluoroethyl)-1H-pyrazol-4-yl)picolinamide |
| *(R)* or *(S) N*-(5-(3-hydroxypyrrolidin-1-yl)-2-(trifluoromethyl)pyridin-3-yl)-6-(1-(2,2,2-trifluoroethyl)-1H-pyrazol-4-yl)picolinamide |
| *(R, R)* or *(S, S)* or *(S, R),* or *(R, S) N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide |
| *(R, R)* or *(S, S)* or *(S, R),* or *(R, S) N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide |
| *(R, R)* or *(S, S)* or *(S, R),* or *(R, S) N*-(5-(4-hydroxy-2-methylpyrrolidin-1-yl)-2-(trifluoromethyl)pyridin-3-yl)-6-(1-(2,2,2-trifluoroethyl)-1H-pyrazol-4-yl)picolinamide |
| (*R*) or *(S) N-*(5-(3-hydroxy-3-methylpyrrolidin-1-yl)-2-(trifluoromethyl)pyridin-3-yl)-6-(1-(2,2,2-trifluoroethyl)-1H-pyrazol-4-yl)picolinamide |
| (*R*) or (*S*) *N-*(5-(3-hydroxy-3-methylpyrrolidin-1-yl)-2-(trifluoromethyl)pyridin-3-yl)-6-(1-(2,2,2-trifluoroethyl)-1H-pyrazol-4-yl)picolinamide |
| (*R*) or (*S*) 6-(1-(cyclopropylmethyl)-1H-pyrazol-4-yl)-*N*-(5-(3-hydroxy-3-methylpyrrolidin-1-yl)-2-(trifluoromethyl)pyridin-3-yl)picolinamide |
| (*R*) or (*S*) *N*-(5-(3-(difluoromethyl)-3-hydroxypyrrolidin-1-yl)-2-(trifluoromethyl)pyridin-3-yl)-6-(1-methyl-1H-pyrazol-4-yl)picolinamide |
| (*R*) or (*S*) *N*-(5-(3-hydroxy-3-methylpiperidin-1-yl)-2-(trifluoromethyl)pyridin-3-yl)-6-(1-methyl-1H-pyrazol-4-yl)picolinamide |
| (*S*)-6-(1-methyl-1H-pyrazol-4-yl)-*N*-(5-(3-methyl-4-(oxetan-3-yl)piperazin-1-yl)-2-(trifluoromethyl)pyridin-3-yl)picolinamide |
| (*S*)-6-(1-(cyclopropylmethyl)-1H-pyrazol-4-yl)-*N*-(5-(3-methyl-4-(oxetan-3-yl)piperazin-1-yl)-2-(trifluoromethyl)pyridin-3-yl)picolinamide |
| (*R*) or (*S*) *N*-(5-(3-cyclopropyl-3-hydroxypyrrolidin-1-yl)-2-(trifluoromethyl)pyridin-3-yl)-6-(1-(cyclopropylmethyl)-1H-pyrazol-4-yl)picolinamide |
| (*R*) or (*S*) *N*-(5-(3-cyclopropyl-3-hydroxypyrrolidin-1-yl)-2-(trifluoromethyl)pyridin-3-yl)-6-(1-(cyclopropylmethyl)-1H-pyrazol-4-yl)picolinamide |
| 6-(1-((1-cyanocyclopropyl)methyl)-1H-pyrazol-4-yl)-*N*-(5-(4-(2-hydroxypropan-2-yl)-2-azabicyclo[2.1.1]hexan-2-yl)-2(trifluoromethyl)pyridin-3-yl)picolinamide |
| (*S*)-6-(1-((1-cyanocyclopropyl)methyl)-1H-pyrazol-4-yl)-*N*-(5-(4-methyl-2-oxopyrrolidin-1-yl)-2-(trifluoromethyl)pyridin-3-yl)picolinamide |
| (*S*)-6-(1-((1-cyanocyclopropyl)methyl)-1H-pyrazol-4-yl)-*N*-(5-(3-methyl-4-(oxetan-3-yl)piperazin-1-yl)-2-(trifluoromethyl)pyridin-3-yl)picolinamide |
| *N*-(5-(5-azaspiro[2.4]heptan-5-yl)-2-(trifluoromethyl)pyridin-3-yl)-6-(1-((1-cyanocyclopropyl)methyl)-1H-pyrazol-4-yl)picolinamide |
| 6-(1-((1-cyanocyclopropyl)methyl)-1H-pyrazol-4-yl)-*N*-(5-(3,3-difluoropyrrolidin-1-yl)-2-(trifluoromethyl)pyridin-3-yl)picolinamide |
| (*R*)-*N*-(5-(3-(2-hydroxypropan-2-yl)pyrrolidin-1-yl)-2-(trifluoromethyl)pyridin-3-yl)-6-(1-((1-(trifluoromethyl)cyclopropyl)methyl)-1H-pyrazol-4-yl)picolinamide |
| (*R*)-6-(1-ethyl-1H-pyrazol-4-yl)-*N*-(5-(3-(2-hydroxypropan-2-yl)pyrrolidin-1-yl)-2-(trifluoromethyl)pyridin-3-yl)picolinamide |
| 6-(1-ethyl-1H-pyrazol-4-yl)-*N*-(5-(4-(2-hydroxypropan-2-yl)-2-azabicyclo[2.1.1]hexan-2-yl)-2-(trifluoromethyl)pyridin-3-yl)picolinamide |
| *(R, R)* or *(S, S)* or *(S, R),* or (*R, S*) 6-(1-ethyl-1H-pyrazol-4-yl)-*N*-(5-(4-hydroxy-2-methylpyrrolidin-1-yl)-2-(trifluoromethyl)pyridin-3-yl)picolinamide |
| *(R)* or *(S)* 6-(1-ethyl-1H-pyrazol-4-yl)-*N*-(5-(3-hydroxy-3-methylpyrrolidin-1-yl)-2-(trifluoromethyl)pyridin-3-yl)picolinamide |
| *(S)*-6-(1-ethyl-1H-pyrazol-4-yl)-*N*-(5-(3-methyl-4-(oxetan-3-yl)piperazin-1-yl)-2-(trifluoromethyl)pyridin-3-yl)picolinamide |
| 6-(1-cyclobutyl-1H-pyrazol-4-yl)-*N*-(5-(3-methoxyazetidin-1-yl)-2-(trifluoromethyl)pyridin-3-yl)picolinamide |
| *(R)*-6-(1-cyclobutyl-1H-pyrazol-4-yl)-*N*-(5-(3-(2-hydroxypropan-2-yl)pyrrolidin-1-yl)-2-(trifluoromethyl)pyridin-3-yl)picolinamide |
| *(S)*-6-(1-cyclobutyl-1H-pyrazol-4-yl)-*N*-(5-(3-methyl-4-(oxetan-3-yl)piperazin-1-yl)-2-(trifluoromethyl)pyridin-3-yl)picolinamide |
| 6-(1-methyl-1H-pyrazol-4-yl)-*N*-(2-methyl-5-(3-(trifluoromethyl)pyrrolidin-1-yl)pyridin-3-yl)picolinamide |
| *N-*(5-(3,3-difluoropyrrolidin-1-yl)-2-methylpyridin-3-yl)-6-(1-methyl-1H-pyrazol-4-yl)picolinamide |
| *N*-(5-((2*S*,6*R*)-2,6-dimethylmorpholino)-2-methylpyridin-3-yl)-6-(1-methyl-1H-pyrazol-4-yl)picolinamide |
| *N*-(5-(3-(fluoromethyl)pyrrolidin-1-yl)-2-methylpyridin-3-yl)-6-(1-methyl-1*H-*pyrazol-4-yl)picolinamide |
| *(S)*-*N*-(2-methyl-5-(4-methyl-2-oxopyrrolidin-1-yl)pyridin-3-yl)-6-(1-(2,2,2-trifluoroethyl)-1H-pyrazol-4-yl)picolinamide |
| *(S)*-6-(1-methyl-1H-pyrazol-4-yl)-*N*-(2-methyl-5-(3-methyl-4-(oxetan-3-yl)piperazin-1-yl)pyridin-3-yl)picolinamide |
| *(R)*-*N*-(5-(3-(2-hydroxypropan-2-yl)pyrrolidin-1-yl)-2-(trifluoromethyl)pyridin-3-yl)-6-(1H-pyrazol-4-yl)picolinamide |
| *N*-(5-(4-(2-hydroxypropan-2-yl)-2-azabicyclo[2.1.1]hexan-2-yl)-2-(trifluoromethyl)pyridin-3-yl)-6-(1H-pyrazol-4-yl)picolinamide |
| *N*-(5-((3*S*,4*s*,5*R*)-4-hydroxy-3,4,5-trimethylpiperidin-1-yl)-2-(trifluoromethyl)pyridin-3-yl)-6-(1H-pyrazol-4-yl)picolinamide |
| *(S)-N*-(5-(3-methyl-4-(oxetan-3-yl)piperazin-1-yl)-2-(trifluoromethyl)pyridin-3-yl)-6-(1H-pyrazol-4-yl)picolinamide |
| *(S)*-*N*-(5-(4-methyl-2-oxopyrrolidin-1-yl)-2-(trifluoromethyl)pyridin-3-yl)-6-(1H-pyrazol-4-yl)picolinamide |
| *(R)*-*N*-(2-cyclopropyl-5-(3-(2-hydroxypropan-2-yl)pyrrolidin-1-yl)pyridin-3-yl)-6-(1-methyl-1H-pyrazol-4-yl)picolinamide |
| *N*-(2-cyclopropyl-5-(4-(2-hydroxypropan-2-yl)-2-azabicyclo[2.1.1]hexan-2-yl)pyridin-3-yl)-6-(1-methyl-1H-pyrazol-4-yl)picolinamide |
| *N*-(2-cyclopropyl-5-(2-oxopyrrolidin-1-yl)pyridin-3-yl)-6-(1-methyl-1H-pyrazol-4-yl)picolinamide |
| *N*-(2-cyclopropyl-5-(2-oxopyrrolidin-1-yl)pyridin-3-yl)-6-(1-(2,2,2-trifluoroethyl)-1H-pyrazol-4-yl)picolinamide |
| *(S)*-*N*-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide |
| *(R)*-*N*-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide |
| *N*-(2-cyclopropyl-5-(2-oxopiperidin-1-yl)pyridin-3-yl)-6-(1-(2,2,2-trifluoroethyl)-1H-pyrazol-4-yl)picolinamide |
| *N*-(2-cyclopropyl-5-(3,3-dimethyl-2-oxopiperidin-1-yl)pyridin-3-yl)-6-(1-(2,2,2-trifluoroethyl)-1H-pyrazol-4-yl)picolinamide |
| *N*-(2-cyclopropyl-5-(4,4-dimethyl-2-oxopyrrolidin-1-yl)pyridin-3-yl)-6-(1-(2,2,2-trifluoroethyl)-1H-pyrazol-4-yl)picolinamide |
| *N*-(2-cyclopropyl-5-(4-methyl-2-oxopiperazin-1-yl)pyridin-3-yl)-6-(1-(2,2,2-trifluoroethyl)-1H-pyrazol-4-yl)picolinamide |
| *N*-(2-cyclopropyl-5-(3,3,4-trimethyl-2-oxopiperazin-1-yl)pyridin-3-yl)-6-(1-(2,2,2-trifluoroethyl)-1H-pyrazol-4-yl)picolinamide |
| *N*-(2-cyclopropyl-5-(3,3-dimethyl-2-oxopyrrolidin-1-yl)pyridin-3-yl)-6-(1-(2,2,2-trifluoroethyl)-1H-pyrazol-4-yl)picolinamide |
| *N*-(2-cyclopropyl-5-(6-oxo-5-azaspiro[2.4]heptan-5-yl)pyridin-3-yl)-6-(1-(2,2,2-trifluoroethyl)-1H-pyrazol-4-yl)picolinamide |
| *(R)*-*N*-(2-cyclopropyl-5-(3-methyl-4-(oxetan-3-yl)piperazin-1-yl)pyridin-3-yl)-6-(1-(2,2,2-trifluoroethyl)-1H-pyrazol-4-yl)picolinamide |
| *(S)*-*N*-(2-cyclopropyl-5-(3-methyl-4-(oxetan-3-yl)piperazin-1-yl)pyridin-3-yl)-6-(1-(2,2,2-trifluoroethyl)-1H-pyrazol-4-yl)picolinamide |
| *N*-(2-cyclopropyl-5-((3S,5R)-3,5-dimethyl-4-(oxetan-3-yl)piperazin-1-yl)pyridin-3-yl)-6-(1-(2,2,2-trifluoroethyl)-1H-pyrazol-4-yl)picolinamide |
| *(S)*-*N*-(2-cyclopropyl-5-(3-methoxypyrrolidin-1-yl)pyridin-3-yl)-6-(1-(2,2,2-trifluoroethyl)-1H-pyrazol-4-yl)picolinamide |
| *N*-(2-cyclopropyl-5-((1S,4R)-3-oxo-2-azabicyclo[2.2.1]heptan-2-yl)pyridin-3-yl)-6-(1-(2,2,2-trifluoroethyl)-1H-pyrazol-4-yl)picolinamide |
| *N*-(2-cyclopropyl-5-(8-methyl-5-oxa-2,8-diazaspiro[3.5]nonan-2-yl)pyridin-3-yl)-6-(1-(2,2,2-trifluoroethyl)-1H-pyrazol-4-yl)picolinamide |
| *N*-(2-cyclopropyl-5-(5-oxo-6-azaspiro[3.4]octan-6-yl)pyridin-3-yl)-6-(1-(2,2,2-trifluoroethyl)-1H-pyrazol-4-yl)picolinamide |
| *N*-(2-cyclopropyl-5-(1-oxo-2-azaspiro[4.4]nonan-2-yl)pyridin-3-yl)-6-(1-(2,2,2-trifluoroethyl)-1H-pyrazol-4-yl)picolinamide |
| *(S)*-*N*-(2-cyclopropyl-5-(3-methyl-4-(oxetan-3-yl)piperazin-1-yl)pyridin-3-yl)-6-(1-methyl-1H-pyrazol-4-yl)picolinamide |
| *N*-(2-cyclopropyl-5-(2-oxa-7-azaspiro[3.5]nonan-7-yl)pyridin-3-yl)-6-(1-methyl-1H-pyrazol-4-yl)picolinamide |
| *N*-(5-((3*R*,4*S*)-3-(2-hydroxypropan-2-yl)-4-methoxypyrrolidin-1-yl)-2-(trifluoromethyl)pyridin-3-yl)-6-(1-(2,2,2-trifluoroethyl)-1H-pyrazol-4-yl)picolinamide |
| *(S)*-*N*-(5-(3-(2-hydroxypropan-2-yl)-3-methoxypyrrolidin-1-yl)-2-(trifluoromethyl)pyridin-3-yl)-6-(1-(2,2,2-trifluoroethyl)-1H-pyrazol-4-yl)picolinamide |
| *(R)*-*N*-(5-(3-(2-hydroxypropan-2-yl)-3-methoxypyrrolidin-1-yl)-2-(trifluoromethyl)pyridin-3-yl)-6-(1-(2,2,2-trifluoroethyl)-1H-pyrazol-4-yl)picolinamide |
| *(S)*-6-(1-(cyclopropylmethyl)-1H-pyrazol-4-yl)-*N*-(5-(3-(2-hydroxypropan-2-yl)-3-methoxypyrrolidin-1-yl)-2-(trifluoromethyl)pyridin-3-yl)picolinamide |
| 6-(1-(cyclopropylmethyl)-1H-pyrazol-4-yl)-*N*-(5-((3R,4S)-3-(2-hydroxypropan-2-yl)-4-methoxypyrrolidin-1-yl)-2-(trifluoromethyl)pyridin-3-yl)picolinamide |
| *(R)*-6-(1-ethyl-1H-pyrazol-4-yl)-*N*-(5-(3-(2-hydroxypropan-2-yl)-3-methoxypyrrolidin-1-yl)-2-(trifluoromethyl)pyridin-3-yl)picolinamide |
| *(S)*-6-(1-ethyl-1H-pyrazol-4-yl)-*N*-(5-(3-(2-hydroxypropan-2-yl)-3-methoxypyrrolidin-1-yl)-2-(trifluoromethyl)pyridin-3-yl)picolinamide |
| 6-(1-ethyl-1H-pyrazol-4-yl)-*N*-(5-((3S,4R)-3-(2-hydroxypropan-2-yl)-4-methoxypyrrolidin-1-yl)-2-(trifluoromethyl)pyridin-3-yl)picolinamide |
| 6-(1-ethyl-1H-pyrazol-4-yl)-*N*-(5-((3R,4S)-3-(2-hydroxypropan-2-yl)-4-methoxypyrrolidin-1-yl)-2-(trifluoromethyl)pyridin-3-yl)picolinamide |
| (R) or (S) N-(5-((R)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4 -yl)picolinamide |
| (R) or (S) N-(5-((R)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4 -yl)picolinamide |
| N-(2-((2R,6S)-2,6-dimethylmorpholino)-5-(trifluoromethyl)pyridin-4-yl)-6-(1-methyl-1H-pyrazol-4-yl)picolinamide |
| (R)-N-(2-(3-(2-hydroxypropan-2-yl)pyrrolidin-1-yl)-5-(trifluoromethyl)pyridin-4-yl)-6-(1-methyl-1H-pyrazol-4-yl)picolinamide |
| N-(2-(4-(2-hydroxypropan-2-yl)-2-azabicyclo[2.1.1]hexan-2-yl)-5-(trifluoromethyl)pyridin-4-yl)-6-(1-methyl-1H-pyrazol-4-yl)picolinamide |
| Meso 6-(1-methyl-1H-pyrazol-4-yl)-N-(2-(tetrahydro-1H-furo[3,4-c]pyrrol-5(3H)-yl)-5-(trifluoromethyl)pyridin-4-yl)picolinamide |
| N-(2-((2R,4R)-4-hydroxy-2-methylpyrrolidin-1-yl)-5-(trifluoromethyl)pyridin-4-yl)-6-(1-methyl-1H-pyrazol-4-yl)picolinamide |
| N-(2-((2S,4R)-4-hydroxy-2-methylpyrrolidin-1-yl)-5-(trifluoromethyl)pyridin-4-yl)-6-(1-methyl-1H-pyrazol-4-yl)picolinatnide |
| N-(2-((2R,3S)-3-hydroxy-2-methylazetidin-1-yl)-5-(trifluoromethyl)pyridin-4-yl)-6-(1-methyl-1H-pyrazol-4-yl)picolinamide |
| N-(2-(3-hydroxyazetidin-1-yl)-5-(trifluoromethyl)pyridin-4-yl)-6-(1-methyl-1H-pyrazol-4-yl)picolinamide |
| N-(2-((3R,5S)-4-hydroxy-2-methylpyrrolidin-1-yl)-5-(trifluoromethyl)pyridin-4-yl)-6-(1-methyl-1H-pyrazol-4-yl)picolinamide |
| (R, R) or (S, S) N-(2-(3-hydroxy-2-methylpyrrolidin-1-yl)-5-(trifluoromethyl)pyridin-4-yl)-6-(1-methyl-1H-pyrazol-4-yl)picolinamide |
| (R, R) or (S, S) N-(2-(3-hydroxy-2-methylpyrrolidin-1-yl)-5-(trifluoromethyl)pyridin-4-yl)-6-(1-methyl-1H-pyrazol-4-yl)picolinamide |
| N-(2-(3-methoxyazetidin-1-yl)-5-(trifluoromethyl)pyridin-4-yl)-6-(1-methyl-1H-pyrazol-4-yl)picolinamide |
| N-(2-(3-(2-hydroxypropan-2-yl)azetidin-1-yl)-5-(trifluoromethyl)pyridin-4-yl)-6-(1-methyl-1H-pyrazol-4-yl)picolinamide |
| N-(2-(3-(difluoromethyl)pyrrolidin-1-yl)-5-(trifluoromethyl)pyridin-4-yl)-6-(1-methyl-1H-pyrazol-4-yl)picolinamide |
| (R)-N-(2-(3-(2-hydroxypropan-2-yl)pyrrolidin-1-yl)-5-(trifluoromethyl)pyridin-4-yl)-6-(1-(2,2,2-trifluoroethyl)-1H-pyrazol-4-yl)picolinamide |
| (R) or (S) N-(2-(3-hydroxy-3-methylpyrrolidin-1-yl)-5-(trifluoromethyl)pyridin-4-yl)-6-(1-methyl-1H-pyrazol-4-yl)picolinatnide |
| (R) or (S) N-(2-(3-hydroxy-3-methylpyrrolidin-1-yl)-5-(trifluoromethyl)pyridin-4-yl)-6-(1-methyl-1H-pyrazol-4-yl)picolinamide |
| (S)-6-(1-methyl-1H-pyrazol-4-yl)-N-(2-(3-methyl-4-(oxetan-3-yl)piperazin-1-yl)-5-(trifluoromethyl)pyridin-4-yl)picolinamide |
| 6-(1-methyl-1H-pyrazol-4-yl)-N-(2-(4-(oxetan-3-yl)piperidin-1-yl)-5-(trifluoromethyl)pyridin-4-yl)picolinamide |
| (R)-N-(2-(3-(2-hydroxypropan-2-yl)pyrrolidin-1-yl)-5-methylpyridin-4-yl)-6-(1-methyl-1H-pyrazol-4-yl)picolinamide |
| (R)-N-(2-(3-(2-hydroxypropan-2-yl)pyrrolidin-1-yl)-5-(trifluoromethyl)pyridin-4-yl)-6-(1H-pyrazol-4-yl)picolinamide |
| N-(2-((2R,3S)-3-hydroxy-2-methylazetidin-1-yl)-5-(trifluoromethyl)pyridin-4-yl)-6-(1H-pyrazol-4-yl)picolinamide |
| N-(2-((2R,4R)-4-hydroxy-2-methylpyrrolidin-1-yl)-5-(trifluoromethyl)pyridin-4-yl)-6-(1H-pyrazol-4-yl)picolinamide |
| N-(2-((2S,4R)-4-hydroxy-2-methylpyrrolidin-1-yl)-5-(trifluoromethyl)pyridin-4-yl)-6-(1H-pyrazol-4-yl)picolinamide |
| 6-(1-methyl-1H-pyrazol-4-yl)-N-(5-methyl-2-morpholinopyridin-4-yl)picolinamide |
| 6-(1-(cyclopropylmethyl)-1H-pyrazol-4-yl)-N-(5-methyl-2-morpholinopyridin-4-yl)picolinamide |
| 6-(1-(cyclopropylmethyl)-1H-pyrazol-4-yl)-N-(2-((2S,6R)-2,6-dimethylmorpholino)-5-methylpyridin-4-yl)picolinamide |
| N-(2-((2S,6R)-2,6-dimethylmorpholino)-5-methylpyridin-4-yl)-6-(1-(2,2,2-trifluoroethyl)-1H-pyrazol-4-yl)picolinamide |
| N-(2-((2S,6R)-2,6-dimethylmorpholino)-5-(trifluoromethyl)pyridin-4-yl)-6-(1-ethyl-1H-pyrazol-4-yl)picolinamide |
| N-(2-((2S,6R)-2,6-dimethylmorpholino)-5-methylpyridin-4-yl)-6-(1-((1-(trifluoromethyl)cyclopropyl)methyl)-1H-pyrazol-4-yl)picolinamide |
| N-(2-((2S,6R)-2,6-dimethylmorpholino)-5-methylpyridin-4-yl)-6-(1-ethyl-1H-pyrazol-4-yl)picolinamide |
| 6-(1-ethyl-1H-pyrazol-4-yl)-N-(5-methyl-2-morpholinopyridin-4-yl)picolinamide |
| 6-(1-cyclobutyl-1H-pyrazol-4-yl)-N-(5-methyl-2-morpholinopyridin-4-yl)picolinamide |
| 6-(1-cyclobutyl-1H-pyrazol-4-yl)-N-(2-((2S,6R)-2,6-dimethylmorpholino)-5-methylpyridin-4-yl)picolinamide |
| N-(2-((2S,6R)-2,6-dimethylmorpholino)-5-methylpyridin-4-yl)-6-(1H-pyrazol-4-yl)picolinamide |
| N-(5-methyl-2-morpholinopyridin-4-yl)-6-(1H-pyrazol-4-yl)picolinamide |
| (R)-N-(6-(3-(2-hydroxypropan-2-yl)pyrrolidin-1-yl)-2-methylpyrimidin-4-yl)-6-(1-methyl-1H-pyrazol-4-yl)picolinamide |
| (R)-N-(6-(3-(2-hydroxypropan-2-yl)pyrrolidin-1-yl)pyrimidin-4-yl)-6-(1-methyl-1H-pyrazol-4-yl)picolinamide |
| N-(6-((2S,6R)-2,6-dimethylmorpholino)pyrimidin-4-yl)-6-(1-methyl-1H-pyrazol-4-yl)picolinamide |
| N-(6-((3S,4r,5R)-4-hydroxy-3,5-dimethylpiperidin-1-yl)pyrimidin-4-yl)-6-(1-methyl-1H-pyrazol-4-yl)picolinamide |
| N-(6-((3S,4r,5R)-4-hydroxy-3,4,5-trimethylpiperidin-1-yl)pyrimidin-4-yl)-6-(1-methyl-1H-pyrazol-4-yl)picolinamide |
| N-(6-(4-(2-hydroxypropan-2-yl)-2-azabicyclo[2.1.1]hexan-2-yl)pyrimidin-4-yl)-6-(1-methyl-1H-pyrazol-4-yl)picolinamide |
| N-(6-(2-azaspiro[4.4]nonan-2-yl)pyrimidin-4-yl)-6-(1-methyl-1H-pyrazol-4-yl)picolinamide |
| (R) or (S) N-(6-(1,1-difluoro-5-azaspiro[2.4]heptan-5-yl)pyrimidin-4-yl)-6-(1-methyl-1H-pyrazol-4-yl)picolinamide |
| (R) or (S) N-(6-(1,1-difluoro-5-azaspiro[2.4]heptan-5-yl)pyrimidin-4-yl)-6-(1-methyl-1H-pyrazol-4-yl)picolinamide |
| (R) or (S) N-(6-(1,1-difluoro-5-azaspiro[2.4]heptan-5-yl)-2-methylpyrimidin-4-yl)-6-(1-methyl-1H-pyrazol-4-yl)picolinamide |
| N-(6-((3S,4r,5R)-4-hydroxy-3,4,5-trimethylpiperidin-1-yl)-2-methylpyrimidin-4-yl)-6-(1-methyl-1H-pyrazol-4-yl)picolinatnide |
| (R)-N-(6-(3-(2-hydroxypropan-2-yl)pyrrolidin-1-yl)pyrimidin-4-yl)-6-(1-(2,2,2-trifluoroethyl)-1H-pyrazol-4-yl)picolinamide |
| N-(6-(4-(oxetan-3-yl)piperazin-1-yl)pyrimidin-4-yl)-6-(1-(2,2,2-trifluoroethyl)-1H-pyrazol-4-yl)picolinamide |
| N-(6-((3S,4r,5R)-4-hydroxy-3,4,5-trimethylpiperidin-1-yl)pyrimidin-4-yl)-6-(1-(2,2,2-trifluoroethyl)-1H-pyrazol-4-yl)picolinatnide |
| N-(6-(4-(3-methyloxetan-3-yl)piperazin-1-yl)pyrimidin-4-yl)-6-(1-(2,2,2-trifluoroethyl)-1H-pyrazol-4-yl)picolinamide |
| N-(6-(3-(2-cyanopropan-2-yl)pyrrolidin-1-yl)-2-methylpyrimidin-4-yl)-6-(1-(cyclopropylmethyl)-1H-pyrazol-4-yl)picolinamide |
| (R)-N-(2-cyclopropyl-6-(3-(2-hydroxypropan-2-yl)pyrrolidin-1-yl)pyrimidin-4-yl)-6-(1-methyl-1H-pyrazol-4-yl)picolinamide |
| (R)-N-(2-cyclopropyl-6-(3-(2-hydroxypropan-2-yl)pyrrolidin-1-yl)pyrimidin-4-yl)-6-(1-(2,2,2-trifluoroethyl)-1H-pyrazol-4-yl)picolinamide |
| N-(2-cyclopropyl-6-((3S,4s,5R)-4-hydroxy-3,4,5-trimethylpiperidin-1-yl)pyrimidin-4-yl)-6-(1-(2,2,2-trifluoroethyl)-1H-pyrazol-4-yl)picolinamide |
| N-(2-cyclopropyl-6-(3-hydroxy-3-methylpyrrolidin-1-yl)pyrimidin-4-yl)-6-(1-(2,2,2-trifluoroethyl)-1H-pyrazol-4-yl)picolinatnide |
| N-(2-cyclopropyl-6-(3-fluoroazetidin-1-yl)pyrimidin-4-yl)-6-(1-(cyclopropylmethyl)-1H-pyrazol-4-yl)picolinamide |
| N-(2-cyclopropyl-6-(3,3-difluoroazetidin-1-yl)pyrimidin-4-yl)-6-(1-(cyclopropylmethyl)-1H-pyrazol-4-yl)picolinamide |
| N-(2-cyclopropyl-6-((3S,4s,5R)-4-hydroxy-3,4,5-trimethylpiperidin-1-yl)pyrimidin-4-yl)-6-(1-(cyclopropylmethyl)-1H-pyrazol-4-yl)picolinamide |
| (R)-N-(6-(3-(2-hydroxypropan-2-yl)pyrrolidin-1-yl)-2-methylpyrimidin-4-yl)-6-(1-(2,2,2-trifluoroethyl)-1H-pyrazol-4-yl)picolinamide |
| (R)-6-(1-(cyclopropylmethyl)-1H-pyrazol-4-yl)-N-(6-(3-(2-hydroxypropan-2-yl)pyrrolidin-1-yl)-2-methylpyrimidin-4-yl)picolinamide |
| N-(6-((3S,4s,5R)-4-hydroxy-3,4,5-trimethylpiperidin-1-yl)-2-methylpyrimidin-4-yl)-6-(1-(2,2,2-trifluoroethyl)-1H-pyrazol-4-yl)picolinamide |
| 6-(1-((1-cyanocyclopropyl)methyl)-1H-pyrazol-4-yl)-N-(6-((3S,4s,5R)-4-hydroxy-3,4,5-trimethylpiperidin-1-yl)-2-methylpyrimidin-4-yl)picolinamide |
| 6-(1-((1-cyanocyclopropyl)methyl)-1H-pyrazol-4-yl)-N-(6-(1,1-difluoro-5-azaspiro[2.4]heptan-5-yl)-2-methylpyrimidin-4-yl)picolinamide |
| (R)-N-(6-(3-(2-hydroxypropan-2-yl)pyrrolidin-1-yl)-2-methylpyrimidin-4-yl)-6-(1-((1-(trifluoromethyl)cyclopropyl)methyl)-1H-pyrazol-4-yl)picolinamide |
| (R)-6-(1-ethyl-1H-pyrazol-4-yl)-N-(6-(3-(2-hydroxypropan-2-yl)pyrrolidin-1-yl)-2-methylpyrimidin-4-yl)picolinamide |
| 6-(1-ethyl-1H-pyrazol-4-yl)-N-(6-((3S,4s,5R)-4-hydroxy-3,4,5-trimethylpiperidin-1-yl)-2-methylpyrimidin-4-yl)picolinamide |
| (S)-6-(1-((1-cyanocyclopropyl)methyl)-1H-pyrazol-4-yl)-N-(6-(3-methyl-4-(oxetan-3-yl)piperazin-1-yl)pyrimidin-4-yl)picolinamide |
| N-(6-((3S,4s,5R)-4-hydroxy-3,4,5-trimethylpiperidin-1-yl)-2-methylpyrimidin-4-yl)-6-(1H-pyrazol-4-yl)picolinamide |
| (R)-N-(6-(3-(2-fluoropropan-2-yl)pyrrolidin-1-yl)-2-methylpyrimidin-4-yl)-6-(1-methyl-1H-pyrazol-4-yl)picolinamide |
| (R)-N-(6-(3-(2-fluoropropan-2-yl)pyrrolidin-1-yl)-2-methylpyrimidin-4-yl)-6-(1-(2,2,2-trifluoroethyl)-1H-pyrazol-4-yl)picolinatnide |
| (R)-N-(5-(3-(2-hydroxypropan-2-yl)pyrrolidin-1-yl)pyridazin-3-yl)-6-(1-methyl-1H-pyrazol-4-yl)picolinamide |
| N-[6-[(3R)-3-(1-hydroxy-1-methyl-ethyl)pyrrolidin-1-yl]-3-methyl-pyridazin-4-yl]-6-(1-methylpyrazol-4-yl)pyridine-2-carboxamide |
| N-[3-cyclopropyl-6-[(3R)-3-(1-hydroxy-1-methyl-ethyl)pyrrolidin-1-yl]pyridazin-4-yl]-6-(1-methylpyrazol-4-yl)pyridine-2-carboxamide |
| (R)-N-(4-(3-(2-hydroxypropan-2-yl)pyrrolidin-1-yl)-6-methylpyridin-2-yl)-6-(1-methyl-1H-pyrazol-4-yl)picolinamide, and |
| (R)-N-(4-(3-(2-hydroxypropan-2-yl)pyrrolidin-1-yl)pyridin-2-yl)-6-(1-methyl-1H-pyrazol-4-yl)picolinamide. |
| (6-(1-(cyclopropylmethyl)-1H-pyrazol-4-yl)-*N*-(4-((3*S*,4*r*,5*R*)-4-hydroxy-3,4,5-trimethylpiperidin-1-yl)-6-methylpyridin-2-yl)picolinamide |
| *(R)*-6-(1-(cyclopropylmethyl)-1H-pyrazol-4-yl)-*N*-(4-(3-(2-hydroxypropan-2-yl)pyrrolidin-1-yl)-6-methylpyridin-2-yl)picolinamide, and |
| *N*-(2-((2*S*,6*R*)-2,6-dimethylmorpholino)-5-methylpyridin-4-yl)-6-(1-methyl-1H-pyrazol-4-yl)picolinamide. |

17. A pharmaceutical composition comprising a compound of any of claims 1 to 16 or a pharmaceutically acceptable salt thereof, and a pharmaceutically acceptable carrier.

18. A compound of any of Claims 1 to 16 or a pharmaceutically acceptable salt thereof, for use in the treatment of Parkinson's Disease.

19. A compound according to any of claims 1 to 16 or a pharmaceutically acceptable salt thereof for use in therapy.

20. A compound according to any of claims 1 to 16, or a pharmaceutically acceptable salt thereof, for use in the treatment of an indication selected from:
abnormal motor symptoms associated with Parkinson's disease, non-motor symptoms associated with Parkinson's disease, Lewy body dementia, L-Dopa induced dyskinesias,
Alzheimer's disease, mild cognitive impairment, the transition from mild cognitive impairment to Alzheimer's disease, tauopathy disorders **characterized by** hyperphosphorylation of tau such as argyrophilic grain disease, Picks disease, corticobasal degeneration, progressive supranuclear palsy, inherited frontotemporal dementia, and Parkinson's disease linked to chromosome 17,
neuroinflammation associated with of microglial inflammatory responses associated with multiple sclerosis, HIV-induced dementia, ALS, ischemic stroke, traumatic brain injury and spinal cord injury,
lymphomas, leukemias, multiple sclerosis, rheumatoid arthritis, systemic lupus erythematosus, autoimmune hemolytic anemia, pure red cell aplasia, idiopathic thrombocytopenic pupura (ITP), Evans Syndrome, vasculitis, bullous skin disorder, type I diabetes mellitus, Sjorgen's syndrome, Delvic's disease, inflammatory myopathies, and ankylosing spondylitis,
renal cancer, breast cancer, lung cancer, prostate cancer, and acute myelogenous leukemia (AML) in subjects expressing the LRRK2 G2019S mutation,
papillary renal and thyroid carcinomas in a subject in whom LRRK2 is amplified or overexpressed, Crohn's disease and leprosy.

21. A combination comprising a compound according to any of claims 1 to 16, or a pharmaceutically acceptable salt thereof and one, two or more other active compounds.

## Patentansprüche

1. Eine Verbindung der Strukturformel (I): 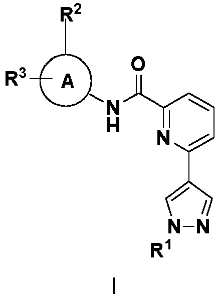 oder ein pharmazeutisch annehmbares Salz davon, wobei:
A ein stickstoffhaltiges Heteroaryl ist, ausgewählt aus Pyridyl, Pyrimidinyl und Pyridazinyl,
R¹ ausgewählt ist aus Wasserstoff, C₁₋₆-Alkyl, C₁₋₆-Halogenalkyl und -(CH₂)ₙC₃₋₆-Cycloalkyl,
wobei das Alkyl und Cycloalkyl gegebenenfalls substituiert sind mit 1 bis 3 Gruppen,
ausgewählt aus CF₃ und CN,
R² ein N-verknüpftes Heterocycloalkyl ist, ausgewählt aus einer Gruppe bestehend aus Pyrrolidinyl, Piperidinyl, Piperazinyl, Azetidinyl, Morpholinyl, Tetrahydrofuronyl, Azabicyclohexanyl, Azabicycloheptanyl, Azaspiroheptanyl, Azaspirononanyl, Diazaspirononanyl, und Azaspirooctanyl, wobei das Pyrrolidinyl, Piperidinyl, Piperazinyl, Azetidinyl, Morpholinyl, Tetrahydrofuronyl, Azabicyclohexanyl, Azabicycloheptanyl, Azaspiroheptanyl, Azaspirononanyl, Diazaspirononanyl und Azaspirooctanyl unsubstituiert oder substituiert sind mit 1 bis 4 Gruppen, unabhängig ausgewählt aus R⁴,
R³ ausgewählt ist aus Wasserstoff, CH₃, CF₃ und Cyclopropyl,
jedes R⁴ unabhängig ausgewählt ist aus der Gruppe bestehend aus C₁₋₆-Alkyl, C₁₋₆-Alkyl-OH, C₁₋₆-Alkyl-CN, OC₁₋₆-Alkyl, C₁₋₆-Halogenalkyl, C₃₋₆-Cycloalkyl, Oxetanyl, Oxo, OH, Halogen, wobei das Oxetanyl gegebenenfalls mit 1 bis 3 CH₃-Gruppen substituiert ist, und
n ausgewählt ist aus 0 bis 3.

2. Die Verbindung gemäß Anspruch 1 oder ein pharmazeutisch annehmbares Salz davon, wobei A Pyridyl ist.

3. Die Verbindung gemäß Anspruch 1 oder ein pharmazeutisch annehmbares Salz davon, wobei A Pyrimidinyl ist.

4. Die Verbindung gemäß Anspruch 1 oder ein pharmazeutisch annehmbares Salz davon, wobei A Pyridazinyl ist.

5. Die Verbindung gemäß einem der Ansprüche 1 bis 3 oder ein pharmazeutisch annehmbares Salz davon, wobei R¹ Wasserstoff, CH₃, CH₂CH₃, CH₂CF₃, CH(CH₃)CF₃, - (CH₂)ₙCyclopropyl oder -(CH₂)ₙCyclobutyl ist, wobei das CH₃, CH₂CH₃, CH₂CF₃, CH(CH₃)CF₃, - (CH₂)ₙCyclopropyl oder -(CH₂)ₙCyclobutyl gegebenenfalls substituiert ist mit 1 bis 3 Gruppen, ausgewählt aus CN und CF₃.

6. Die Verbindung gemäß einem der Ansprüche 1 bis 5 oder ein pharmazeutisch annehmbares Salz davon, wobei R² ausgewählt ist aus der Gruppe bestehend aus Pyrrolidinyl, Piperidinyl, Piperazinyl, Azetidinyl, Morpholinyl und Tetrahydrofuronyl, wobei die Gruppe unsubstituiert oder substituiert ist mit 1 bis 4 Gruppen, unabhängig ausgewählt aus R⁴.

7. Die Verbindung gemäß einem der Ansprüche 1 bis 6 oder ein pharmazeutisch annehmbares Salz davon, wobei R² Pyrrolidinyl ist, unsubstituiert oder substituiert mit 1 bis 4 Gruppen, unabhängig ausgewählt aus R⁴.

8. Die Verbindung gemäß einem der Ansprüche 1 bis 6 oder ein pharmazeutisch annehmbares Salz davon, wobei R² Piperidinyl oder Piperazinyl ist, unsubstituiert oder substituiert mit 1 bis 4 Gruppen, unabhängig ausgewählt aus R⁴.

9. Die Verbindung gemäß einem der Ansprüche 1 bis 5 oder ein pharmazeutisch annehmbares Salz davon, wobei R² ausgewählt ist aus der Gruppe bestehend aus Azabicyclohexanyl, Azabicycloheptanyl, Azaspiroheptanyl, Azaspirononanyl, Diazaspirononanyl und Azaspirooctanyl, wobei die Gruppe unsubstituiert oder substituiert ist mit 1 bis 4 Gruppen, unabhängig ausgewählt aus R⁴.

10. Die Verbindung gemäß einem der Ansprüche 1 bis 5 und 9 oder ein pharmazeutisch annehmbares Salz davon, wobei R² ausgewählt ist aus der Gruppe bestehend aus Azabicyclohexanyl, Azabicycloheptanyl und Azaspiroheptanyl, wobei die Gruppe unsubstituiert oder substituiert ist mit 1 bis 4 Gruppen, unabhängig ausgewählt aus R⁴.

11. Die Verbindung gemäß einem der Ansprüche 1 bis 10, wobei R⁴ unabhängig ausgewählt ist aus der Gruppe bestehend aus Wasserstoff, CH₃, OCH₃, C(CH₃)₂OH, C(CH₃)₂CN, CF₃, CH₂F, C(CH₃)₂F, Cyclopropyl, Oxetanyl, Oxo, OH und Fluor, wobei das Oxetanyl gegebenenfalls substituiert ist mit 1 bis 3 CH₃-Gruppen.

12. Die Verbindung gemäß Anspruch 1, dargestellt durch Strukturformel II: 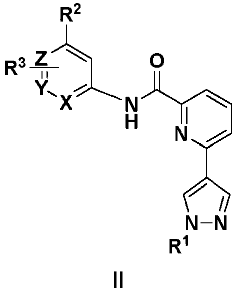 oder ein pharmazeutisch annehmbares Salz davon, wobei X, Y und Z dargestellt und ausgewählt sind aus der Gruppe bestehend aus:
**1)** X=Z=N, und Y=CH oder C-CH₃,
**2)** Y=N, Z=C, und X=C-CH₃, C-CF₃ oder C-Cyclopropyl,
**3)** Z=N, Y=C, und X= C-CH₃, C-CF₃ oder C-Cyclopropyl,
**4)** Y=Z=N, und X=C-CH₃ oder C-Cyclopropyl,
**5)** X=Y=N=, und Z=CH, und
**6)** Z=CH, Y=CH oder C-CH₃, und X=N.

13. Die Verbindung gemäß Anspruch 12 oder ein pharmazeutisch annehmbares Salz davon,
wobei wenn X, Y und Z verbunden sind unter Bildung von Pyridyl, ausgewählt aus der Gruppe bestehend aus 2), 3), 5) und 6), R¹ ausgewählt ist aus der Gruppe bestehend aus CH₃, CH₂CH₃, CH₂CF₃, CH(CH₃)CF₃, -(CH₂)ₙCyclopropyl und -(CH₂)ₙCyclobutyl, wobei das CH₃, CH₂CH₃, CH₂CF₃, CH(CH₃)CF₃, -(CH₂)ₙCyclopropyl und -(CH₂)ₙCyclobutyl gegebenenfalls substituiert sind mit 1 bis 3 Gruppen, ausgewählt aus CN und CF₃, R² ausgewählt ist aus Pyrrolidinyl, Piperidinyl, Piperazinyl, Azetidinyl, Morpholinyl und Tetrahydrofuranyl, R³ ausgewählt ist aus der Gruppe bestehend aus Wasserstoff, CH₃, CF₃ und Cyclopropyl, und jedes R⁴ unabhängig ausgewählt ist aus der Gruppe bestehend aus Wasserstoff, CH₃, OCH₃, C(CH₃)₂OH, C(CH₃)₂CN, CF₃, CH₂F, C(CH₃)₂F, Cyclopropyl, Oxetanyl, Oxo, OH und Fluor, wobei das Oxetanyl gegebenenfalls substituiert ist mit 1 bis 3 CH₃-Gruppen.

14. Die Verbindung gemäß Anspruch 12, wobei X, Y und Z verbunden sind unter Bildung von Pyrimidinyl, dargestellt durch Gruppe 1).

15. Die Verbindung gemäß Anspruch 12, wobei X, Y und Z verbunden sind unter Bildung von Pyridazinyl, dargestellt durch Gruppe 4).

16. Die Verbindung nach Anspruch 1 oder ein pharmazeutisch annehmbares Salz davon, wobei die Verbindung ausgewählt ist aus:
| |
|---|
| *(R)*-*N*-(5-(3-(2-Hydroxypropan-2-yl)pyrrolidin-1-yl)-2-(trifluormethyl)pyridin-3-yl)-6-(1-methyl-1H-pyrazol-4-yl)picolinamid |
| *N*-(5-(4-(2-Hydroxypropan-2-yl)-2-azabicyclo[2.1.1]hexan-2-yl)-2-(trifluormethyl)pyridin-3-yl)-6-(1-methyl-1H-pyrazol-4-yl)picolinamid |
| *N*-(5-((3*S*,4*s*,5*R*)-4-Hydroxy-3,4,5-trimethylpiperidin-1-yl)-2-(trifluormethyl)pyridin-3-yl)-6-(1-(2,2,2-trifluorethyl)-1H-pyrazol-4-yl)picolinamid |
| *(R)*-*N*-(5-(3-(2-Hydroxypropan-2-yl)pyrrolidin-1-yl)-2-(trifluormethyl)pyridin-3-yl)-6-(1-(2,2,2-trifluorethyl)-1H-pyrazol-4-yl)picolinamid |
| *(R)*-6-(1-(Cyclopropylmethyl)-1H-pyrazol-4-yl)-*N*-(5-(3-(2-hydroxypropan-2-yl)pyrrolidin-1-yl)-2-(trifluormethyl)pyridin-3-yl)picolinamid |
| *N*-(5-(3-Methoxyazetidin-1-yl)-2-(trifluormethyl)pyridin-3-yl)-6-(1-(2,2,2-trifluorethyl)-1H-pyrazol-4-yl)picolinamid |
| *(R)*-*N*-(5-(3-(Fluormethyl)pyrrolidin-1-yl)-2-(trifluormethyl)pyridin-3-yl)-6-(1-methyl-1H-pyrazol-4-yl)picolinamid |
| *N*-(5-(3-(2-Hydroxypropan-2-yl)-3-methylpyrrolidin-1-yl)-2-(trifluormethyl)pyridin-3-yl)-6-(1-methyl-1H-pyrazol-4-yl)picolinamid |
| *N*-(5-(3-Hydroxypyrrolidin-1-yl)-2-(trifluormethyl)pyridin-3-yl)-6-(1-methyl-1H-pyrazol-4-yl)picolinamid |
| *(S)*-*N*-(5-(3-Methoxypyrrolidin-1-yl)-2-(trifluormethyl)pyridin-3-yl)-6-(1-(2,2,2-trifluorethyl)-1H-pyrazol-4-yl)picolinamid |
| *N*-(5-(4-(2-Hydroxypropan-2-yl)-2-azabicyclo[2.1.1]hexan-2-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxy-3-methylpyrrolidin-1-yl)-2-(trifluormethyl)pyridin-3-yl)-6-(1-methyl-1H-pyrazol-4-yl)picolinamid |
| *(R)* oder *(S)*-*N*-(5-(3-Hydroxy-3-methylpyrrolidin-1-yl)-2-(trifluormethyl)pyridin-3-yl)-6-(1-methyl-1H-pyrazol-4-yl)picolinamid |
| *N*-(5-((2*R*,3*R*)-3-Hydroxy-2-methylpyrrolidin-1-yl)-2-(trifluormethyl)pyridin-3-yl)-6-(1-(2,2,2-trifluorethyl)-1H-pyrazol-4-yl)picolinamid |
| *N*-(5-((2*R*,3*S*)-3-Hydroxy-2-methylpyrrolidin-1-yl)-2-(trifluormethyl)pyridin-3-yl)-6-(1-(2,2,2-trifluorethyl)-1H-pyrazol-4-yl)picolinamid |
| (*R*) oder (*S*)-*N*-(5-(3-Hydroxy-3-(2-hydroxypropan-2-yl)pyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxy-3-(2-hydroxypropan-2-yl)pyrrolidin-1-yl)-2-(trifluormethyl)pyridin-3-yl)-6-(1-(2,2,2-trifluorethyl)-1H-pyrazol-4-yl)picolinamid |
| *(R,S*) oder *(S,R)*-*N*-(5-(3-(2-Hydroxypropan-2-yl)-4-methylpyrrolidin-1-yl)-2-(trifluormethyl)pyridin-3-yl)-6-(1-(2,2,2-trifluorethyl)-1H-pyrazol-4-yl)picolinamid |
| *(R,S)* oder *(S,R)*-*N*-(5-(3-(2-Hydroxypropan-2-yl)-4-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-(2-Hydroxypropan-2-yl)-3-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-(2-Hydroxypropan-2-yl)-3-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxypyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxypyrrolidin-1-yl)-2-(trifluormethyl)pyridin-3-yl)-6-(1-(2,2,2-trifluorethyl)-1H-pyrazol-4-yl)picolinamid |
| *(R,R)* oder *(S,S)* oder *(S,R)* oder *(R,S)*-*N*-(5-(4-Hydroxy-2-methylpyrrolidin-1-yl)-2-(trifluormethyl)pyridin-3-yl)-6-(1-(2,2,2-trifluorethyl)-1H-pyrazol-4-yl)picolinamid |
| *(R,R)* oder *(S,S)* oder *(S,R)* oder *(R,S)*-*N*-(5-(4-Hydroxy-2-methylpyrrolidin-1-yl)-2-(trifluormethyl)pyridin-3-yl)-6-(1-(2,2,2-trifluorethyl)-1H-pyrazol-4-yl)picolinamid |
| *(R,R)* oder *(S,S)* oder *(S,R)* oder *(R,S)*-*N*-(5-(4-Hydroxy-2-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxy-3-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-*N*-(5-(3-Hydroxy-3-methylpyrrolidin-1-yl)-2-(trifluormethyl)pyridin-3-yl)-6-(1-(2,2,2-trifluorethyl)-1H-pyrazol-4-yl)picolinamid |
| *(R)* oder *(S)*-6-(1-(Cyclopropylmethyl)-1H-pyrazol-4-yl)-*N*-(5-(3-hydroxy-3-methylpyrrolidin-1-yl)-2-(trifluormethyl)pyridin-3-yl)picolinamid |
| *(R)* oder *(S)*-*N*-(5-(3-(Difluormethyl)-3-hydroxypyrrolidin-1-yl)-2-(trifluormethyl)pyridin-3-yl)-6-(1-methyl-1H-pyrazol-4-yl)picolinamid |
| *(R)* oder *(S)*-*N*-(5-(3-Hydroxy-3-methylpiperidin-1-yl)-2-(trifluormethyl)pyridin-3-yl)-6-(1-methyl-1H-pyrazol-4-yl)picolinamid |
| *(S)*-6-(1-Methyl-1H-pyrazol-4-yl)-*N*-(5-(3-methyl-4-(oxetan-3-yl)piperazin-1-yl)-2-(trifluormethyl)pyridin-3-yl)picolinamid |
| *(S)*-6-(1-(Cyclopropylmethyl)-1H-pyrazol-4-yl)-*N*-(5-(3-methyl-4-(oxetan-3-yl)piperazin-1-yl)-2-(trifluormethyl)pyridin-3-yl)picolinamid |
| *(R)* oder *(S)*-*N*-(5-(3-Cyclopropyl-3-hydroxypyrrolidin-1-yl)-2-(trifluormethyl)pyridin-3-yl)-6-(1-(cyclopropylmethyl)-1H-pyrazol-4-yl)picolinamid |
| *(R)* oder *(S)*-*N*-(5-(3-Cyclopropyl-3-hydroxypyrrolidin-1-yl)-2-(trifluormethyl)pyridin-3-yl)-6-(1-(cyclopropylmethyl)-1H-pyrazol-4-yl)picolinamid |
| 6-(1-((1-Cyanocyclopropyl)methyl)-1H-pyrazol-4-yl)-*N*-(5-(4-(2-hydroxypropan-2-yl)-2-azabicyclo[2.1.1]hexan-2-yl)-2(trifluormethyl)pyridin-3-yl)picolinamid |
| *(S)*-6-(1-((1-Cyanocyclopropyl)methyl)-1H-pyrazol-4-yl)-*N*-(5-(4-methyl-2-oxopyrrolidin-1-yl)-2-(trifluormethyl)pyridin-3-yl)picolinamid |
| *(S)*-6-(1-((1-Cyanocyclopropyl)methyl)-1H-pyrazol-4-yl)-*N*-(5-(3-methyl-4-(oxetan-3-yl)piperazin-1-yl)-2-(trifluormethyl)pyridin-3-yl)picolinamid |
| *N*-(5-(5-Azaspiro[2.4]heptan-5-yl)-2-(trifluormethyl)pyridin-3-yl)-6-(1-((1-cyanocyclopropyl)methyl)-1H-pyrazol-4-yl)picolinamid |
| 6-(1-((1-Cyanocyclopropyl)methyl)-1H-pyrazol-4-yl)-*N*-(5-(3,3-difluorpyrrolidin-1-yl)-2-(trifluormethyl)pyridin-3-yl)picolinamid |
| *(R)*-*N*-(5-(3-(2-Hydroxypropan-2-yl)pyrrolidin-1-yl)-2-(trifluormethyl)pyridin-3-yl)-6-(1-((1-(trifluormethyl)cyclopropyl)methyl)-1H-pyrazol-4-yl)picolinamid |
| *(R)*-6-(1-Ethyl-1H-pyrazol-4-yl)-*N*-(5-(3-(2-hydroxypropan-2-yl)pyrrolidin-1-yl)-2-(trifluormethyl)pyridin-3-yl)picolinamid |
| 6-(1-Ethyl-1H-pyrazol-4-yl)-*N*-(5-(4-(2-hydroxypropan-2-yl)-2-azabicyclo[2.1.1]hexan-2-yl)-2-(trifluormethyl)pyridin-3-yl)picolinamid |
| *(R,R)* oder *(S,S)* oder *(S,R)* oder *(R,S)*-6-(1-Ethyl-1H-pyrazol-4-yl)-*N*-(5-(4-hydroxy-2-methylpyrrolidin-1-yl)-2-(trifluormethyl)pyridin-3-yl)picolinamid |
| *(R)* oder *(S)*-6-(1-Ethyl-1H-pyrazol-4-yl)-*N*-(5-(3-hydroxy-3-methylpyrrolidin-1-yl)-2-(trifluormethyl)pyridin-3-yl)picolinamid |
| *(S)*-6-(1-Ethyl-1H-pyrazol-4-yl)-*N*-(5-(3-methyl-4-(oxetan-3-yl)piperazin-1-yl)-2-(trifluormethyl)pyridin-3-yl)picolinamid |
| 6-(1-Cyclobutyl-1H-pyrazol-4-yl)-*N*-(5-(3-methoxyazetidin-1-yl)-2-(trifluormethyl)pyridin-3-yl)picolinamid |
| *(R)*-6-(1-Cyclobutyl-1H-pyrazol-4-yl)-*N*-(5-(3-(2-hydroxypropan-2-yl)pyrrolidin-1-yl)-2-(trifluormethyl)pyridin-3-yl)picolinamid |
| *(S)*-6-(1-Cyclobutyl-1H-pyrazol-4-yl)-*N*-(5-(3-methyl-4-(oxetan-3-yl)piperazin-1-yl)-2-(trifluormethyl)pyridin-3-yl)picolinamid |
| 6-(1-Methyl-1H-pyrazol-4-yl)-*N*-(2-methyl-5-(3-(trifluormethyl)pyrrolidin-1-yl)pyridin-3-yl)picolinamid |
| *N*-(5-(3,3-Difluorpyrrolidin-1-yl)-2-methylpyridin-3-yl)-6-(1-methyl-1H-pyrazol-4-yl)picolinamid |
| *N*-(5-((2S,6R)-2,6-Dimethylmorpholino)-2-methylpyridin-3-yl)-6-(1-methyl-1H-pyrazol-4-yl)picolinamid |
| *N*-(5-(3-(Fluormethyl)pyrrolidin-1-yl)-2-methylpyridin-3-yl)-6-(1-methyl-1H-pyrazol-4-yl)picolinamid |
| *(S)*-*N*-(2-Methyl-5-(4-methyl-2-oxopyrrolidin-1-yl)pyridin-3-yl)-6-(1-(2,2,2-trifluorethyl)-1H-pyrazol-4-yl)picolinamid |
| *(S)*-6-(1-Methyl-1H-pyrazol-4-yl)-*N*-(2-methyl-5-(3-methyl-4-(oxetan-3-yl)piperazin-1-yl)pyridin-3-yl)picolinamid |
| *(R)*-*N*-(5-(3-(2-Hydroxypropan-2-yl)pyrrolidin-1-yl)-2-(trifluormethyl)pyridin-3-yl)-6-(1H-pyrazol-4-yl)picolinamid |
| *N*-(5-(4-(2-Hydroxypropan-2-yl)-2-azabicyclo[2.1.1]hexan-2-yl)-2-(trifluormethyl)pyridin-3-yl)-6-(1H-pyrazol-4-yl)picolinamid |
| *N*-(5-((3*S*,4*s*,5*R*)-4-Hydroxy-3,4,5-trimethylpiperidin-1-yl)-2-(trifluormethyl)pyridin-3-yl)-6-(1H-pyrazol-4-yl)picolinamid |
| *(S)*-*N*-(5-(3-Methyl-4-(oxetan-3-yl)piperazin-1-yl)-2-(trifluormethyl)pyridin-3-yl)-6-(1H-pyrazol-4-yl)picolinamid |
| *(S)*-*N*-(5-(4-Methyl-2-oxopyrrolidin-1-yl)-2-(trifluormethyl)pyridin-3-yl)-6-(1H-pyrazol-4-yl)picolinamid |
| *(R)*-*N*-(2-Cyclopropyl-5-(3-(2-hydroxypropan-2-yl)pyrrolidin-1-yl)pyridin-3-yl)-6-(1-methyl-1H-pyrazol-4-yl)picolinamid |
| *N*-(2-Cyclopropyl-5-(4-(2-hydroxypropan-2-yl)-2-azabicyclo[2.1.1]hexan-2-yl)pyridin-3-yl)-6-(1-methyl-1H-pyrazol-4-yl)picolinamid |
| *N*-(2-Cyclopropyl-5-(2-oxopyrrolidin-1-yl)pyridin-3-yl)-6-(1-methyl-1H-pyrazol-4-yl)picolinamid |
| *N*-(2-Cyclopropyl-5-(2-oxopyrrolidin-1-yl)pyridin-3-yl)-6-(1-(2,2,2-trifluorethyl)-1H-pyrazol-4-yl)picolinamid |
| *(S)*-*N*-(2-Cyclopropyl-5-(4-methyl-2-oxopyrrolidin-1-yl)pyridin-3-yl)-6-(1-(2,2,2-trifluorethyl)-1H-pyrazol-4-yl)picolinamid |
| *(R)*-*N*-(2-Cyclopropyl-5-(4-methyl-2-oxopyrrolidin-1-yl)pyridin-3-yl)-6-(1-(2,2,2-trifluorethyl)-1H-pyrazol-4-yl)picolinamid |
| *N*-(2-Cyclopropyl-5-(2-oxopiperidin-1-yl)pyridin-3-yl)-6-(1-(2,2,2-trifluorethyl)-1H-pyrazol-4-yl)picolinamid |
| *N*-(2-Cyclopropyl-5-(3,3-dimethyl-2-oxopiperidin-1-yl)pyridin-3-yl)-6-(1-(2,2,2-trifluorethyl)-1H-pyrazol-4-yl)picolinamid |
| *N*-(2-Cyclopropyl-5-(4,4-dimethyl-2-oxopyrrolidin-1-yl)pyridin-3-yl)-6-(1-(2,2,2-trifluorethyl)-1H-pyrazol-4-yl)picolinamid |
| *N*-(2-Cyclopropyl-5-(4-methyl-2-oxopiperazin-1-yl)pyridin-3-yl)-6-(1-(2,2,2-trifluorethyl)-1H-pyrazol-4-yl)picolinamid |
| *N*-(2-Cyclopropyl-5-(3,3,4-trimethyl-2-oxopiperazin-1-yl)pyridin-3-yl)-6-(1-(2,2,2-trifluorethyl)-1H-pyrazol-4-yl)picolinamid |
| *N*-(2-Cyclopropyl-5-(3,3-dimethyl-2-oxopyrrolidin-1-yl)pyridin-3-yl)-6-(1-(2,2,2-trifluorethyl)-1H-pyrazol-4-yl)picolinamid |
| *N*-(2-Cyclopropyl-5-(6-oxo-5-azaspiro[2.4]heptan-5-yl)pyridin-3-yl)-6-(1-(2,2,2-trifluorethyl)-1H-pyrazol-4-yl)picolinamid |
| *(R)*-*N*-(2-Cyclopropyl-5-(3-methyl-4-(oxetan-3-yl)piperazin-1-yl)pyridin-3-yl)-6-(1-(2,2,2-trifluorethyl)-1H-pyrazol-4-yl)picolinamid |
| *(S)*-*N*-(2-Cyclopropyl-5-(3-methyl-4-(oxetan-3-yl)piperazin-1-yl)pyridin-3-yl)-6-(1-(2,2,2-trifluorethyl)-1H-pyrazol-4-yl)picolinamid |
| *N*-(2-Cyclopropyl-5-((3*S*,5*R*)-3,5-dimethyl-4-(oxetan-3-yl)piperazin-1-yl)pyridin-3-yl)-6-(1-(2,2,2-trifluorethyl)-1H-pyrazol-4-yl)picolinamid |
| *(S)*-*N*-(2-Cyclopropyl-5-(3-methoxypyrrolidin-1-yl)pyridin-3-yl)-6-(1-(2,2,2-trifluorethyl)-1H-pyrazol-4-yl)picolinamid |
| *N*-(2-Cyclopropyl-5-((1*S*,4*R*)-3-oxo-2-azabicyclo[2.2.1]heptan-2-yl)pyridin-3-yl)-6-(1-(2,2,2-trifluorethyl)-1H-pyrazol-4-yl)picolinamid |
| *N*-(2-Cyclopropyl-5-(8-methyl-5-oxa-2,8-diazaspiro[3.5]nonan-2-yl)pyridin-3-yl)-6-(1-(2,2,2-trifluorethyl)-1H-pyrazol-4-yl)picolinamid |
| *N*-(2-Cyclopropyl-5-(5-oxo-6-azaspiro[3.4]octan-6-yl)pyridin-3-yl)-6-(1-(2,2,2-trifluorethyl)-1H-pyrazol-4-yl)picolinamid |
| *N*-(2-Cyclopropyl-5-(1-oxo-2-azaspiro[4.4]nonan-2-yl)pyridin-3-yl)-6-(1-(2,2,2-trifluorethyl)-1H-pyrazol-4-yl)picolinamid |
| *(S)*-*N*-(2-Cyclopropyl-5-(3-methyl-4-(oxetan-3-yl)piperazin-1-yl)pyridin-3-yl)-6-(1-methyl-1H-pyrazol-4-yl)picolinamid |
| *N*-(2-Cyclopropyl-5-(2-oxa-7-azaspiro[3.5]nonan-7-yl)pyridin-3-yl)-6-(1-methyl-1H-pyrazol-4-yl)picolinamid |
| *N*-(5-((3R,4S)-3-(2-hydroxypropan-2-yl)-4-methoxypyrrolidin-1-yl)-2-(trifluormethyl)pyridin-3-yl)-6-(1-(2,2,2-trifluorethyl)-1H-pyrazol-4-yl)picolinamid |
| *(S)*-*N*-(5-(3-(2-Hydroxypropan-2-yl)-3-methoxypyrrolidin-1-yl)-2-(trifluormethyl)pyridin-3-yl)-6-(1-(2,2,2-trifluorethyl)-1H-pyrazol-4-yl)picolinamid |
| *(R)*-*N*-(5-(3-(2-Hydroxypropan-2-yl)-3-methoxypyrrolidin-1-yl)-2-(trifluormethyl)pyridin-3-yl)-6-(1-(2,2,2-trifluorethyl)-1H-pyrazol-4-yl)picolinamid |
| *(S)*-6-(1-(Cyclopropylmethyl)-1H-pyrazol-4-yl)-*N*-(5-(3-(2-hydroxypropan-2-yl)-3-methoxypyrrolidin-1-yl)-2-(trifluormethyl)pyridin-3-yl)picolinamid |
| 6-(1-(Cyclopropylmethyl)-1H-pyrazol-4-yl)-*N*-(5-((3*R*,4*S*)-3-(2-hydroxypropan-2-yl)-4-methoxypyrrolidin-1-yl)-2-(trifluormethyl)pyridin-3-yl)picolinamid |
| *(R)*-6-(1-Ethyl-1H-pyrazol-4-yl)-*N*-(5-(3-(2-hydroxypropan-2-yl)-3-methoxypyrrolidin-1-yl)-2-(trifluormethyl)pyridin-3-yl)picolinamid |
| *(S)*-6-(1-Ethyl-1H-pyrazol-4-yl)-*N*-(5-(3-(2-hydroxypropan-2-yl)-3-methoxypyrrolidin-1-yl)-2-(trifluormethyl)pyridin-3-yl)picolinamid |
| 6-(1-Ethyl-1H-pyrazol-4-yl)-*N*-(5-((3*S*,4*R*)-3-(2-hydroxypropan-2-yl)-4-methoxypyrrolidin-1-yl)-2-(trifluormethyl)pyridin-3-yl)picolinamid |
| 6-(1-Ethyl-1H-pyrazol-4-yl)-*N*-(5-((3*R*,4*S*)-3-(2-hydroxypropan-2-yl)-4-methoxypyrrolidin-1-yl)-2-(trifluormethyl)pyridin-3-yl)picolinamid |
| *(R)* oder *(S)*-*N*-(5-(*(R)*-3-(2-Hydroxypropan-2-yl)pyrrolidin-1-yl)-2-(trifluormethyl)pyridin-3-yl)-6-(1-(1,1,1-trifluorpropan-2-yl)-1H-pyrazol-4-yl)picolinamid |
| *(R)* oder *(S)*-*N*-(5-((R)-3-(2-Hydroxypropan-2-yl)pyrrolidin-1-yl)-2-(trifluormethyl)pyridin-3-yl)-6-(1-(1,1,1-trifluorpropan-2-yl)-1H-pyrazol-4-yl)picolinamid |
| *N*-(2-((2R,6S)-2,6-Dimethylmorpholino)-5-(trifluormethyl)pyridin-4-yl)-6-(1-methyl-1H-pyrazol-4-yl)picolinamid |
| *(R)*-*N*-(2-(3-(2-Hydroxypropan-2-yl)pyrrolidin-1-yl)-5-(trifluormethyl)pyridin-4-yl)-6-(1-methyl-1H-pyrazol-4-yl)picolinamid |
| *N*-(2-(4-(2-Hydroxypropan-2-yl)-2-azabicyclo[2.1.1]hexan-2-yl)-5-(trifluormethyl)pyridin-4-yl)-6-(1-methyl-1H-pyrazol-4-yl)picolinamid |
| meso-6-(1-Methyl-1H-pyrazol-4-yl)-*N*-(2-(tetrahydro-1H-furo[3,4-c]pyrrol-5(3H)-yl)-5-(trifluormethyl)pyridin-4-yl)picolinamid |
| *N*-(2-((2*R*,4*R*)-4-Hydroxy-2-methylpyrrolidin-1-yl)-5-(trifluormethyl)pyridin-4-yl)-6-(1-methyl-1H-pyrazol-4-yl)picolinamid |
| *N*-(2-((2*S*,4*R*)-4-Hydroxy-2-methylpyrrolidin-1-yl)-5-(trifluormethyl)pyridin-4-yl)-6-(1-methyl-1H-pyrazol-4-yl)picolinamid |
| *N*-(2-((2*R*,3*S*)-3-Hydroxy-2-methylazetidin-1-yl)-5-(trifluormethyl)pyridin-4-yl)-6-(1-methyl-1H-pyrazol-4-yl)picolinamid |
| *N*-(2-(3-Hydroxyazetidin-1-yl)-5-(trifluormethyl)pyridin-4-yl)-6-(1-methyl-1H-pyrazol-4-yl)picolinamid |
| *N*-(2-((3*R*,5*S*)-4-Hydroxy-2-methylpyrrolidin-1-yl)-5-(trifluormethyl)pyridin-4-yl)-6-(1-methyl-1H-pyrazol-4-yl)picolinamid |
| *(R,R)* oder *(S,S)*-*N*-(2-(3-Hydroxy-2-methylpyrrolidin-1-yl)-5-(trifluormethyl)pyridin-4-yl)-6-(1-methyl-1H-pyrazol-4-yl)picolinamid |
| *(R,R)* oder *(S,S)*-*N*-(2-(3-Hydroxy-2-methylpyrrolidin-1-yl)-5-(trifluormethyl)pyridin-4-yl)-6-(1-methyl-1H-pyrazol-4-yl)picolinamid |
| *N*-(2-(3-Methoxyazetidin-1-yl)-5-(trifluormethyl)pyridin-4-yl)-6-(1-methyl-1H-pyrazol-4-yl)picolinamid |
| *N*-(2-(3-(2-Hydroxypropan-2-yl)azetidin-1-yl)-5-(trifluormethyl)pyridin-4-yl)-6-(1-methyl-1H-pyrazol-4-yl)picolinamid |
| *N*-(2-(3-(Difluormethyl)pyrrolidin-1-yl)-5-(trifluormethyl)pyridin-4-yl)-6-(1-methyl-1H-pyrazol-4-yl)picolinamid |
| *(R)*-*N*-(2-(3-(2-Hydroxypropan-2-yl)pyrrolidin-1-yl)-5-(trifluormethyl)pyridin-4-yl)-6-(1-(2,2,2-trifluorethyl)-1H-pyrazol-4-yl)picolinamid |
| *(R)* oder *(S)*-*N*-(2-(3-Hydroxy-3-methylpyrrolidin-1-yl)-5-(trifluormethyl)pyridin-4-yl)-6-(1-methyl-1H-pyrazol-4-yl)picolinamid |
| *(R)* oder *(S)*-*N*-(2-(3-Hydroxy-3-methylpyrrolidin-1-yl)-5-(trifluormethyl)pyridin-4-yl)-6-(1-methyl-1H-pyrazol-4-yl)picolinamid |
| *(S)*-6-(1-Methyl-1H-pyrazol-4-yl)-*N*-(2-(3-methyl-4-(oxetan-3-yl)piperazin-1-yl)-5-(trifluormethyl)pyridin-4-yl)picolinamid |
| 6-(1-Methyl-1H-pyrazol-4-yl)-*N*-(2-(4-(oxetan-3-yl)piperidin-1-yl)-5-(trifluormethyl)pyridin-4-yl)picolinamid |
| *(R)*-*N*-(2-(3-(2-Hydroxypropan-2-yl)pyrrolidin-1-yl)-5-methylpyridin-4-yl)-6-(1-methyl-1H-pyrazol-4-yl)picolinamid |
| *(R)*-*N*-(2-(3-(2-Hydroxypropan-2-yl)pyrrolidin-1-yl)-5-(trifluormethyl)pyridin-4-yl)-6-(1H-pyrazol-4-yl)picolinamid |
| *N*-(2-((2*R*,3*S*)-3-Hydroxy-2-methylazetidin-1-yl)-5-(trifluormethyl)pyridin-4-yl)-6-(1H-pyrazol-4-yl)picolinamid |
| *N*-(2-((2*R*,4*R*)-4-Hydroxy-2-methylpyrrolidin-1-yl)-5-(trifluormethyl)pyridin-4-yl)-6-(1H-pyrazol-4-yl)picolinamid |
| *N*-(2-((2*S*,4*R*)-4-Hydroxy-2-methylpyrrolidin-1-yl)-5-(trifluormethyl)pyridin-4-yl)-6-(1H-pyrazol-4-yl)picolinamid |
| 6-(1-Methyl-1H-pyrazol-4-yl)-*N*-(5-methyl-2-morpholinopyridin-4-yl)picolinamid |
| 6-(1-(Cyclopropylmethyl)-1H-pyrazol-4-yl)-*N*-(5-methyl-2-morpholinopyridin-4-yl)picolinamid |
| 6-(1-(Cyclopropylmethyl)-1H-pyrazol-4-yl)-*N*-(2-((2S,6R)-2,6-dimethylmorpholino)-5-methylpyridin-4-yl)picolinamid |
| *N*-(2-((2*S*,6*R*)-2,6-Dimethylmorpholino)-5-methylpyridin-4-yl)-6-(1-(2,2,2-trifluorethyl)-1H-pyrazol-4-yl)picolinamid |
| *N*-(2-((2*S*,6*R*)-2,6-Dimethylmorpholino)-5-(trifluormethyl)pyridin-4-yl)-6-(1-ethyl-1H-pyrazol-4-yl)picolinamid |
| *N*-(2-((2*S*,6*R*)-2,6-Dimethylmorpholino)-5-methylpyridin-4-yl)-6-(1-((1-(trifluormethyl)cyclopropyl)methyl)-1H-pyrazol-4-yl)picolinamid |
| *N*-(2-((2*S*,6*R*)-2,6-Dimethylmorpholino)-5-methylpyridin-4-yl)-6-(1-ethyl-1H-pyrazol-4-yl)picolinamid |
| 6-(1-Ethyl-1H-pyrazol-4-yl)-*N*-(5-methyl-2-morpholinopyridin-4-yl)picolinamid |
| 6-(1-Cyclobutyl-1H-pyrazol-4-yl)-*N*-(5-methyl-2-morpholinopyridin-4-yl)picolinamid |
| 6-(1-Cyclobutyl-1H-pyrazol-4-yl)-*N-*(2-((2S,6R)-2,6-dimethylmorpholino)-5-methylpyridin-4-yl)picolinamid |
| *N*-(2-((2*S*,6*R*)-2,6-Dimethylmorpholino)-5-methylpyridin-4-yl)-6-(1H-pyrazol-4-yl)picolinamid |
| *N*-(5-Methyl-2-morpholinopyridin-4-yl)-6-(1H-pyrazol-4-yl)picolinamid |
| *(R)*-*N*-(6-(3-(2-Hydroxypropan-2-yl)pyrrolidin-1-yl)-2-methylpyrimidin-4-yl)-6-(1-methyl-1H-pyrazol-4-yl)picolinamid |
| *(R)*-*N*-(6-(3-(2-Hydroxypropan-2-yl)pyrrolidin-1-yl)pyrimidin-4-yl)-6-(1-methyl-1H-pyrazol-4-yl)picolinamid |
| *N*-(6-((2*S*,6*R*)-2,6-Dimethylmorpholino)pyrimidin-4-yl)-6-(1-methyl-1H-pyrazol-4-yl)picolinamid |
| *N*-(6-((3*S*,4*r*,5*R*)-4-Hydroxy-3,5-dimethylpiperidin-1-yl)pyrimidin-4-yl)-6-(1-methyl-1H-pyrazol-4-yl)picolinamid |
| *N*-(6-((3*S*,4*r*,5*R*)-4-Hydroxy-3,4,5-trimethylpiperidin-1-yl)pyrimidin-4-yl)-6-(1-methyl-1H-pyrazol-4-yl)picolinamid |
| *N*-(6-(4-(2-Hydroxypropan-2-yl)-2-azabicyclo[2.1.1]hexan-2-yl)pyrimidin-4-yl)-6-(1-methyl-1H-pyrazol-4-yl)picolinamid |
| *N*-(6-(2-Azaspiro[4.4]nonan-2-yl)pyrimidin-4-yl)-6-(1-methyl-1H-pyrazol-4-yl)picolinamid |
| *(R)* oder *(S)*-*N*-(6-(1,1-Difluor-5-azaspiro[2.4]heptan-5-yl)pyrimidin-4-yl)-6-(1-methyl-1H-pyrazol-4-yl)picolinamid |
| *(R)* oder *(S)*-*N*-(6-(1,1-Difluor-5-azaspiro[2.4]heptan-5-yl)pyrimidin-4-yl)-6-(1-methyl-1H-pyrazol-4-yl)picolinamid |
| *(R)* oder *(S)*-*N*-(6-(1,1-Difluor-5-azaspiro[2.4]heptan-5-yl)-2-methylpyrimidin-4-yl)-6-(1-methyl-1H-pyrazol-4-yl)picolinamid |
| *N*-(6-((3*S*,4*r*,5*R*)-4-Hydroxy-3,4,5-trimethylpiperidin-1-yl)-2-methylpyrimidin-4-yl)-6-(1-methyl-1H-pyrazol-4-yl)picolinamid |
| *(R)*-*N*-(6-(3-(2-Hydroxypropan-2-yl)pyrrolidin-1-yl)pyrimidin-4-yl)-6-(1-(2,2,2-trifluorethyl)-1H-pyrazol-4-yl)picolinamid |
| *N*-(6-(4-(Oxetan-3-yl)piperazin-1-yl)pyrimidin-4-yl)-6-(1-(2,2,2-trifluorethyl)-1H-pyrazol-4-yl)picolinamid |
| *N*-(6-((3*S*,4*r*,5*R*)-4-Hydroxy-3,4,5-trimethylpiperidin-1-yl)pyrimidin-4-yl)-6-(1-(2,2,2-trifluorethyl)-1H-pyrazol-4-yl)picolinamid |
| *N*-(6-(4-(3-Methyloxetan-3-yl)piperazin-1-yl)pyrimidin-4-yl)-6-(1-(2,2,2-trifluorethyl)-1H-pyrazol-4-yl)picolinamid |
| *N*-(6-(3-(2-Cyanopropan-2-yl)pyrrolidin-1-yl)-2-methylpyrimidin-4-yl)-6-(1-(cyclopropylmethyl)-1H-pyrazol-4-yl)picolinamid |
| *(R)*-*N*-(2-Cyclopropyl-6-(3-(2-hydroxypropan-2-yl)pyrrolidin-1-yl)pyrimidin-4-yl)-6-(1-methyl-1H-pyrazol-4-yl)picolinamid |
| *(R)*-*N*-(2-Cyclopropyl-6-(3-(2-hydroxypropan-2-yl)pyrrolidin-1-yl)pyrimidin-4-yl)-6-(1-(2,2,2-trifluorethyl)-1H-pyrazol-4-yl)picolinamid |
| *N*-(2-Cyclopropyl-6-((3*S*,4*s*,5*R*)-4-hydroxy-3,4,5-trimethylpiperidin-1-yl)pyrimidin-4-yl)-6-(1-(2,2,2-trifluorethyl)-1H-pyrazol-4-yl)picolinamid |
| *N*-(2-Cyclopropyl-6-(3-hydroxy-3-methylpyrrolidin-1-yl)pyrimidin-4-yl)-6-(1-(2,2,2-trifluorethyl)-1H-pyrazol-4-yl)picolinamid |
| *N*-(2-Cyclopropyl-6-(3-fluorazetidin-1-yl)pyrimidin-4-yl)-6-(1-(cyclopropylmethyl)-1H-pyrazol-4-yl)picolinamid |
| *N*-(2-Cyclopropyl-6-(3,3-difluorazetidin-1-yl)pyrimidin-4-yl)-6-(1-(cyclopropylmethyl)-1H-pyrazol-4-yl)picolinamid |
| *N*-(2-Cyclopropyl-6-((3*S*,4*s*,5*R*)-4-hydroxy-3,4,5-trimethylpiperidin-1-yl)pyrimidin-4-yl)-6-(1-(cyclopropylmethyl)-1H-pyrazol-4-yl)picolinamid |
| *(R)*-*N*-(6-(3-(2-Hydroxypropan-2-yl)pyrrolidin-1-yl)-2-methylpyrimidin-4-yl)-6-(1-(2,2,2-trifluorethyl)-1H-pyrazol-4-yl)picolinamid |
| *(R)*-6-(1-(Cyclopropylmethyl)-1H-pyrazol-4-yl)-*N*-(6-(3-(2-hydroxypropan-2-yl)pyrrolidin-1-yl)-2-methylpyrimidin-4-yl)picolinamid |
| *N-*(6-((3*S*,4*s*,5*R*)-4-Hydroxy-3,4,5-trimethylpiperidin-1-yl)-2-methylpyrimidin-4-yl)-6-(1-(2,2,2-trifluorethyl)-1H-pyrazol-4-yl)picolinamid |
| 6-(1-((1-Cyanocyclopropyl)methyl)-1H-pyrazol-4-yl)-*N*-(6-((3*S*,4*s*,5*R*)-4-hydroxy-3,4,5-trimethylpiperidin-1-yl)-2-methylpyrimidin-4-yl)picolinamid |
| 6-(1-((1-Cyanocyclopropyl)methyl)-1H-pyrazol-4-yl)-*N*-(6-(1,1-difluor-5-azaspiro[2.4]heptan-5-yl)-2-methylpyrimidin-4-yl)picolinamid |
| *(R)*-*N*-(6-(3-(2-Hydroxypropan-2-yl)pyrrolidin-1-yl)-2-methylpyrimidin-4-yl)-6-(1-((1-(trifluormethyl)cyclopropyl)methyl)-1H-pyrazol-4-yl)picolinamid |
| *(R)*-6-(1-Ethyl-1H-pyrazol-4-yl)-*N*-(6-(3-(2-hydroxypropan-2-yl)pyrrolidin-1-yl)-2-methylpyrimidin-4-yl)picolinamid |
| 6-(1-Ethyl-1H-pyrazol-4-yl)-*N*-(6-((3*S*,4*s*,5*R*)-4-hydroxy-3,4,5-trimethylpiperidin-1-yl)-2-methylpyrimidin-4-yl)picolinamid |
| *(S)*-6-(1-((1-Cyanocyclopropyl)methyl)-1H-pyrazol-4-yl)-*N*-(6-(3-methyl-4-(oxetan-3-yl)piperazin-1-yl)pyrimidin-4-yl)picolinamid |
| *N*-(6-((3*S*,4*s*,5*R*)-4-Hydroxy-3,4,5-trimethylpiperidin-1-yl)-2-methylpyrimidin-4-yl)-6-(1H-pyrazol-4-yl)picolinamid |
| *(R)*-*N*-(6-(3-(2-Fluorpropan-2-yl)pyrrolidin-1-yl)-2-methylpyrimidin-4-yl)-6-(1-methyl-1H-pyrazol-4-yl)picolinamid |
| *(R)*-*N*-(6-(3-(2-Fluorpropan-2-yl)pyrrolidin-1-yl)-2-methylpyrimidin-4-yl)-6-(1-(2,2,2-trifluorethyl)-1H-pyrazol-4-yl)picolinamid |
| *(R)*-*N*-(5-(3-(2-Hydroxypropan-2-yl)pyrrolidin-1-yl)pyridazin-3-yl)-6-(1-methyl-1H-pyrazol-4-yl)picolinamid |
| *N*-[6-[(3*R*)-3-(1-Hydroxy-1-methyl-ethyl)pyrrolidin-1-yl]-3-methyl-pyridazin-4-yl]-6-(1-methylpyrazol-4-yl)pyridin-2-carboxamide |
| *N*-[3-Cyclopropyl-6-[(3*R*)-3-(1-hydroxy-1-methyl-ethyl)pyrrolidin-1-yl]pyridazin-4-yl]-6-(1-methylpyrazol-4-yl)pyridin-2-carboxamide |
| *(R)*-*N*-(4-(3-(2-Hydroxypropan-2-yl)pyrrolidin-1-yl)-6-methylpyridin-2-yl)-6-(1-methyl-1H-pyrazol-4-yl)picolinamid und |
| *(R)*-*N*-(4-(3-(2-Hydroxypropan-2-yl)pyrrolidin-1-yl)pyridin-2-yl)-6-(1-methyl-1H-pyrazol-4-yl)picolinamid |
| (6-(1-(Cyclopropylmethyl)-1H-pyrazol-4-yl)-*N*-(4-((3*S*,4*r*,5*R*)-4-hydroxy-3,4,5-trimethylpiperidin-1-yl)-6-methylpyridin-2-yl)picolinamid |
| *(R)*-6-(1-(Cyclopropylmethyl)-1H-pyrazol-4-yl)-*N*-(4-(3-(2-hydroxypropan-2-yl)pyrrolidin-1-yl)-6-methylpyridin-2-yl)picolinamid und |
| *N*-(2-((2*S*,6*R*)-2,6-Dimethylmorpholino)-5-methylpyridin-4-yl)-6-(1-methyl-1H-pyrazol-4-yl)picolinamid |

17. Eine pharmazeutische Zusammensetzung, umfassend eine Verbindung nach einem der Ansprüche 1 bis 16 oder ein pharmazeutisch annehmbares Salz davon und einen pharmazeutisch annehmbaren Träger.

18. Eine Verbindung nach einem der Ansprüche 1 bis 16 oder ein pharmazeutisch annehmbares Salz davon zur Verwendung bei der Behandlung von Parkinson-Krankheit.

19. Eine Verbindung gemäß einem der Ansprüche 1 bis 16 oder ein pharmazeutisch annehmbares Salz davon zur Verwendung in der Therapie.

20. Eine Verbindung gemäß einem der Ansprüche 1 bis 16 oder ein pharmazeutisch annehmbares Salz davon zur Verwendung bei der Behandlung einer Indikation, ausgewählt aus:
abnormalen motorischen Symptomen in Verbindung mit Parkinson-Krankheit, nichtmotorischen Symptomen in Verbindung mit Parkinson-Krankheit, Lewy-Körperchen-Demenz, L-Dopa-induzierten Dyskinesien,
Alzheimer-Krankheit, leichter kognitiver Beeinträchtigung, dem Übergang von leichter kognitiver Beeinträchtigung zu Alzheimer-Krankheit, Tauopathie-Störungen, die durch Hyperphosphorylierung von Tau gekennzeichnet sind, wie beispielsweise Silberkornkrankheit, Morbus Pick, kortikobasale Degeneration, progressive supranukleäre Blickparese, hereditäre frontotemporale Demenz und Parkinson-Krankheit verbunden mit Chromosom 17,
Neuroinflammation in Verbindung mit Mikroglia-Entzündungsreaktionen in Verbindung mit multipler Sklerose, HIV-induzierter Demenz, ALS, ischämischem Schlaganfall, traumatischer Hirnverletzung und Rückenmarkverletzung,
Lymphomen, Leukämien, multipler Sklerose, rheumatoider Arthritis, systemischem Lupus erythematodes, autoimmunhämolytischer Anämie, erworbener isolierter aplastischer Anämie, idiopathischer thrombozytopenischer Purpura (ITP), Evans-Syndrom, Vaskulitis, bullösen Hauterkrankungen, Typ-I-Diabetes mellitus, Sjögren-Syndrom, Devic-Syndrom, inflammatorischen Myopathien und Spondylitis ankylosans,
Nierenkarzinom, Brustkrebs, Lungenkrebs, Prostatakrebs und akuter myeloischer Leukämie (AML) bei Subjekten, die die LRRK2-G2019S-Mutation exprimieren,
papillären Nieren- und Schilddrüsenkarzinomen bei einem Subjekt, bei dem LRRK2 verstärkt oder überexprimiert wird, Morbus Crohn oder Lepra.

21. Eine Kombination, umfassend eine Verbindung gemäß einem der Ansprüche 1 bis 16 oder ein pharmazeutisch annehmbares Salz davon und einen, zwei oder mehrere andere Wirkstoffe.

## Revendications

1. Composé ayant une Formule structurale (I) : 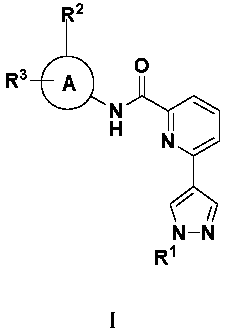 ou sel pharmaceutiquement acceptable correspondant :
A étant un hétéroaryle contenant azote choisi parmi pyridinyle, pyrimidinyle et pyridazinyle ;
R¹ étant choisi parmi hydrogène, C₁₋₆ alkyle, C₁₋₆ halogénoalkyle, et -(CH₂)ₙC₃₋₆ cycloalkyle, lesdits alkyle et cycloalkyle étant éventuellement substitués par 1 à 3 groupes choisis parmi CF₃ et CN ;
R² étant un hétérocycloalkyle N-lié choisi dans le groupe constitué par pyrrolidinyle, pipéridinyle, pipérazinyle, azétidinyle, morpholinyle, tétrahydrofuronyle, azabicylohexanyle, azabicyloheptanyle, azaspiroheptanyle, azaspirononanyle, diazaspirononanyle, et azaspirooctanyle, lesdits pyrrolidinyle, pipéridinyle, pipérazinyle, azétidinyle, morpholinyle, tétrahydrofuronyle, azabicylohexanyle, azabicyloheptanyle, azaspiroheptanyle, azaspirononanyle, diazaspirononanyle, et azaspirooctanyle, étant non substitués ou substitués par 1 à 4 groupes indépendamment choisis parmi R⁴ ;
R³ étant choisi parmi hydrogène, CH₃, CF₃, et cyclopropyle ;
chaque R⁴ étant indépendamment choisi dans le groupe constitué par C₁₋₆ alkyle, C₁₋₆ alkylOH, C₁₋₆ alkylCN, OC₁₋₆ alkyle, C₁₋₆ halogénoalkyle, C₃₋₆ cycloalkyle, oxétanyle, oxo, OH, halogène, ledit oxétanyle étant éventuellement substitué par 1 à 3 groupes CH₃ ; et
n étant choisi parmi 0 à 3.

2. Composé selon la revendication 1 ou sel pharmaceutiquement acceptable correspondant, A étant pyridinyle.

3. Composé selon la revendication 1 ou sel pharmaceutiquement acceptable correspondant, A étant pyrimidinyle.

4. Composé selon la revendication 1 ou sel pharmaceutiquement acceptable correspondant, A étant pyridazinyle.

5. Composé selon l'une quelconque des revendications 1 à 3 ou sel pharmaceutiquement acceptable correspondant, R¹ étant hydrogène, CH₃, CH₂CH₃, CH₂CF₃, CH(CH₃)CF₃, -(CH₂)ₙcyclopropyle, ou -(CH₂)ₙcyclobutyle, ledit CH₃, CH₂CH₃, CH₂CF₃, CH (CH₃)CF₃, - (CH₂)ₙcyclopropyle, ou -(CH₂)ₙcyclobutyle étant éventuellement substitué par 1 à 3 groupes choisis parmi CN et CF₃.

6. Composé selon l'une quelconque des revendications 1 à 5 ou sel pharmaceutiquement acceptable correspondant, R² étant choisi dans le groupe constitué par pyrrolidinyle, pipéridinyle, pipérazinyle, azétidinyle, morpholinyle et tétrahydrofuronyle, ledit groupe étant non substitué ou substitué par 1 à 4 groupes indépendamment choisis parmi R⁴.

7. Composé selon l'une quelconque des revendications 1 à 6 ou sel pharmaceutiquement acceptable correspondant, R² étant pyrrolidinyle, non substitué ou substitué par 1 à 4 groupes indépendamment choisis parmi R⁴.

8. Composé selon l'une quelconque des revendications 1 à 6 ou sel pharmaceutiquement acceptable correspondant, R² étant pipéridinyle ou pipérazinyle, non substitué ou substitué par 1 à 4 groupes indépendamment choisis parmi R⁴.

9. Composé selon l'une quelconque des revendications 1 à 5 ou sel pharmaceutiquement acceptable correspondant, R² étant choisi dans le groupe constitué par azabicylohexanyle, azabicyloheptanyle, azaspiroheptanyle, azaspirononanyle, diazaspirononanyle, et azaspirooctanyle, ledit groupe étant non substitué ou substitué par 1 à 4 groupes indépendamment choisis parmi R⁴.

10. Composé selon l'une quelconque des revendications 1 à 5 et 9 ou sel pharmaceutiquement acceptable correspondant, R² étant choisi dans le groupe constitué par azabicylohexanyle, azabicyloheptanyle, et azaspiroheptanyle, ledit groupe étant non substitué ou substitué par 1 à 4 groupes indépendamment choisis parmi R⁴.

11. Composé selon l'une quelconque des revendications 1 à 10, R⁴ étant indépendamment choisi dans le groupe constitué par hydrogène, CH₃, OCH₃, C (CH₃)₂OH, C (CH₃)₂CN, CF₃, CH₂F, C(CH3)₂F, cyclopropyle, oxétanyle, oxo, OH, et fluoro, ledit oxétanyle étant éventuellement substitué par 1 à 3 groupes de CH₃.

12. Composé selon la revendication 1 représenté par la Formule structurale II : 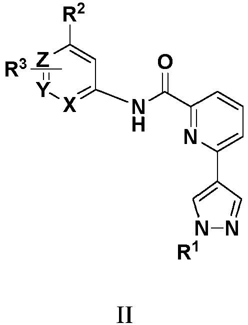 ou sel pharmaceutiquement acceptable correspondant, X, Y, et Z étant représentés et choisis dans le groupe constitué par :
1) X=Z=N, et Y=CH ou C-CH3 ;
2) Y=N, Z=C, et X=C-CH3, C-CF3, ou C-cyclopropyle ;
3) Z=N, Y=C, et X= C-CH3, C-CF3, ou C-cyclopropyle ;
4) Y=Z=N et X=C-CH3 ou C-cyclopropyle ;
5) X=Y=N= et Z=CH ; et
6) Z=CH, Y=CH ou C-CH3 et X=N.

13. Composé selon la revendication 12 ou sel pharmaceutiquement acceptable correspondant
lorsque X, Y, et Z sont combinés pour former pyridinyle choisi dans le groupe constitué par 2), 3), 5) et 6), R¹ étant choisi dans le groupe constitué par CH₃, CH₂CH₃, CH₂CF₃, CH(CH₃)CF₃, - (CH₂)ₙcyclopropyle, et -(CH₂)ₙcyclobutyle, ledit CH₃, CH₂CH₃, CH₂CF₃, CH (CH₃)CF₃, - (CH₂)ₙcyclopropyle, et -(CH₂)ₙcyclobutyle étant éventuellement substitué par 1 à 3 groupes choisis parmi CN et CF₃ ; R² étant choisi parmi pyrrolidinyle, pipéridinyle, pipérazinyle, azétidinyle, morpholinyle et tétrahydrofuronyle, R³ étant choisi dans le groupe constitué par hydrogène, CH₃, CF₃ et cyclopropyle ; et chaque R⁴ étant indépendamment choisi dans le groupe constitué par hydrogène, CH₃, OCH₃, C (CH₃)₂OH, C(CH₃)₂CN, CF₃, CH₂F, C(CH₃)₂F, cyclopropyle, oxétanyle, oxo, OH, et fluoro, ledit oxétanyle étant éventuellement substitué par 1 à 3 groupes CH₃.

14. Composé selon la revendication 12, X, Y, et Z étant combinés pour former pyrimidinyle représenté par le groupe 1).

15. Composé selon la revendication 12, X, Y, et Z étant combinés pour former pyridazinyle représenté par le groupe 4).

16. Composé selon la revendication 1, ou sel pharmaceutiquement acceptable correspondant, ledit composé étant choisi parmi :
| |
|---|
| (R)-N-(5-(3-(2-hydroxypropan-2-yl)pyrrolidin-1-yl)-2-(trifluorométhyl)pyridin-3-yl)-6-(1-méthyl-1H-pyrazol-4-yl)picolinamide |
| N-(5-(4-(2-hydroxypropan-2-yl)-2-azabicyclo[2.1.1]hexan-2-yl)-2-(trifluoromethyl)pyridin-3-yl)-6-(1-methyl-1H-pyrazol-4-yl)picolinamide |
| N-(5-((3S,4s,5R)-4-hydroxy-3,4,5-trimethylpiperidin-1-yl)-2-(trifluorométhyl)pyridin-3-yl)-6-(1-(2,2,2-trifluoroéthyl)-1H-pyrazol-4-yl)picolinamide |
| (R)-N-(5-(3-(2-hydroxypropan-2-yl)pyrrolidin-1-yl)-2-(trifluorométhyl)pyridin-3-yl)-6-(1-(2,2,2-trifluoroéthyl)-1H-pyrazol-4-yl)picolinamide |
| (R)-6-(1-(cyclopropylméthyl)-1H-pyrazol-4-yl)-N-(5-(3-(2-hydroxypropan-2-yl)pyrrolidin-1-yl)-2-(trifluorométhyl)pyridin-3-yl)picolinamide |
| N-(5-(3-méthoxyazétidin-1-yl)-2-(trifluorométhyl)pyridin-3-yl)-6-(1-(2,2,2-trifluoroéthyl)-1H-pyrazol-4-yl)picolinamide |
| (R)-N-(5-(3-(fluorométhyl)pyrrolidin-1-yl)-2-(trifluoromethyl)pyridin-3-yl)-6-(1-methyl-1H-pyrazol-4-yl)picolinamide |
| N-(5-(3-(2-hydroxypropan-2-yl)-3-methylpyrrolidin-1-yl)-2-(trifluorométhyl)pyridin-3-yl)-6-(1-méthyl-1H-pyrazol-4-yl)picolinamide |
| N-(5-(3-hydroxypyrrolidin-1-yl)-2-(trifluorométhyl)pyridin-3-yl)-6-(1-méthyl-1H-pyrazol-4-yl)picolinamide |
| (S)-N-(5-(3-méthoxypyrrolidin-1-yl)-2-(trifluorométhyl)pyridin-3-yl)-6-(1-(2,2,2-trifluoroethyl)-1H-pyrazol-4-yl)picolinamide |
| N-(5-(4-(2-hydroxypropan-2-yl)-2-azabicyclo[2.1.1]hexan-2-yl)-2-(trifluorométhyl)pyridin-3-yl)-6-(1-(2,2,2-trifluoroethyl)-1H-pyrazol-4-yl)picolinamide |
| (R) ou (S)-N-(5-(3-hydroxy-3-méthylpyrrolidin-1-yl)-2-(trifluorométhyl)pyridin-3-yl)-6-(1-méthyl-1H-pyrazol-4-yl)picolinamide |
| (R) ou (S)-N-(5-(3-hydroxy-3-méthylpyrrolidin-1-yl)-2-(trifluorométhyl)pyridin-3-yl)-6-(1-méthyl-1H-pyrazol-4-yl)picolinamide |
| N-(5-((2R,3R)-3-hydroxy-2-méthylpyrrolidin-1-yl)-2-(trifluorométhyl)pyridin-3-yl)-6-(1-(2,2,2-trifluoroethyl)-1H-pyrazol-4-yl)picolinamide |
| N-(5-((2R,3S)-3-hydroxy-2-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-hydroxy-3-(2-hydroxypropan-2-yl)pyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-hydroxy-3-(2-hydroxypropan-2-yl)pyrrolidin-1-yl)-2-(trifluorométhyl)pyridin-3-yl)-6-(1-(2,2,2-trifluoroéthyl)-1H-pyrazol-4-yl)picolinamide |
| (R, S) ou (S, R) N-(5-(3-(2-hydroxypropan-2-yl)-4-méthylpyrrolidin-1-yl)-2-(trifluorométhyl)pyridin-3-yl)-6-(1-(2,2,2-trifluoroéthyl)-1H-pyrazol-4-yl)picolinamide |
| (R, S) ou (S, R) N-(5-(3-(2-hydroxypropan-2-yl)-4-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-(2-hydroxypropan-2-yl)-3-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-(2-hydroxypropan-2-yl)-3-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-hydroxypyrrolidin-1-yl)-2-(trifluorométhyl)pyridin-3-yl)-6-(1-(2,2,2-trifluoroethyl)-1H-pyrazol-4-yl)picolinamide |
| (R) ou (S) N-(5-(3-hydroxypyrrolidin-1-yl)-2-(trifluorométhyl)pyridin-3-yl)-6-(1-(2,2,2-trifluoroethyl)-1H-pyrazol-4-yl)picolinamide |
| (R, R) ou (S, S) ou (S, R), ou (R S) N-(5-(4-hydroxy-2-méthylpyrrolidin-1-yl)-2-(trifluorométhyl)pyridin-3-yl)-6-(1-(2,2,2-trifluoroéthyl)-1H-pyrazol-4-yl)picolinamide |
| (R, R) ou (S, S) ou (S, R), ou (R S) N-(5-(4-hydroxy-2-méthylpyrrolidin-1-yl)-2-(trifluorométhyl)pyridin-3-yl)-6-(1-(2,2,2-trifluoroéthyl)-1H-pyrazol-4-yl)picolinamide |
| (R, R) ou (S, S) ou (S, R), ou (R, S) N-(5-(4-hydroxy-2-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-hydroxy-3-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) N-(5-(3-hydroxy-3-méthylpyrrolidin-1-yl)-2-(trifluorométhyl)pyridin-3-yl)-6-(1-(2,2,2-trifluoroéthyl)-1H-pyrazol-4-yl)picolinamide |
| (R) ou (S) 6-(1-(cyclopropylméthyl)-1H-pyrazol-4-yl)-N-(5-(3-hydroxy-3-méthylpyrrolidin-1-yl)-2-(trifluorométhyl)pyridin-3-yl)picolinamide |
| (R) ou (S) N-(5-(3-(difluorométhyl)-3-hydroxypyrrolidin-1-yl)-2-(trifluorométhyl)pyridin-3-yl)-6-(1-methyl-1H-pyrazol-4-yl)picolinamide |
| (R) ou (S) N-(5-(3-hydroxy-3-méthylpipéridin-1-yl)-2-(trifluorométhyl)pyridin-3-yl)-6-(1-méthyl-1H-pyrazol-4-yl)picolinamide |
| (S)-6-(1-méthyl-1H-pyrazol-4-yl)-N-(5-(3-méthyl-4-(oxétan-3-yl)pipérazin-1-yl)-2-(trifluorométhyl)pyridin-3-yl)picolinamide |
| (S)-6-(1-(cyclopropylméthyl)-1H-pyrazol-4-yl)-N-(5-(3-méthyl-4-(oxétan-3-yl)pipérazin-1-yl)-2-(trifluorométhyl)pyridin-3-yl)picolinamide |
| (R) ou (S) N-(5-(3-cyclopropyl-3-hydroxypyrrolidin-1-yl)-2-(trifluorométhyl)pyridin-3-yl)-6-(1-(cyclopropylméthyl)-1H-pyrazol-4-yl)picolinamide |
| (R) ou (S) N-(5-(3-cyclopropyl-3-hydroxypyrrolidin-1-yl)-2-(trifluorométhyl)pyridin-3-yl)-6-(1-(cyclopropylméthyl)-1H-pyrazol-4-yl)picolinamide |
| 6-(1-((1-cyanocyclopropyl)méthyl)-1H-pyrazol-4-yl)-N-(5-(4-(2-hydroxypropan-2-yl)-2-azabicyclo[2.1.1]hexan-2-yl)-2(trifluorométhyl)pyridin-3-yl)picolinamide |
| (S)-6-(1-((1-cyanocyclopropyl)méthyl)-1H-pyrazol-4-yl)-N-(5-(4-méthyl-2-oxopyrrolidin-1-yl)-2-(trifluorométhyl)pyridin-3-yl)picolinamide |
| (S)-6-(1-((1-cyanocyclopropyl)méthyl)-1H-pyrazol-4-yl)-N-(5-(3-méthyl-4-(oxétan-3-yl)pipérazin-1-yl)-2-(trifluorométhyl)pyridin-3-yl)picolinamide |
| N-(5-(5-azaspiro[2.4]heptan-5-yl)-2-(trifluorométhyl)pyridin-3-yl)-6-(1-((1-cyanocyclopropyl)méthyl)-1H-pyrazol-4-yl)picolinamide |
| 6-(1-((1-cyanocyclopropyl)méthyl)-1H-pyrazol-4-yl)-N-(5-(3,3-difluoropyrrolidin-1-yl)-2-(trifluorométhyl)pyridin-3-yl)picolinamide |
| (R)-N-(5-(3-(2-hydroxypropan-2-yl)pyrrolidin-1-yl)-2-(trifluorométhyl)pyridin-3-yl)-6-(1-((1-(trifluorométhyl)cyclopropyl)méthyl)-1H-pyrazol-4-yl)picolinamide |
| (R)-6-(1-éthyl-1H-pyrazol-4-yl)-N-(5-(3-(2-hydroxypropan-2-yl)pyrrolidin-1-yl)-2-(trifluorométhyl)pyridin-3-yl)picolinamide |
| 6-(1-éthyl-1H-pyrazol-4-yl)-N-(5-(4-(2-hydroxypropan-2-yl)-2-azabicyclo[2.1.1]hexan-2-yl)-2-(trifluorométhyl)pyridin-3-yl)picolinamide |
| (R, R) ou (S, S) ou (S, R), ou (R, S) 6-(1-éthyl-1H-pyrazol-4-yl)-N-(5-(4-hydroxy-2-méthylpyrrolidin-1-yl)-2-(trifluoromethyl)pyridin-3-yl)picolinamide |
| (R) ou (S) 6-(1-éthyl-1H-pyrazol-4-yl)-N-(5-(3-hydroxy-3-méthylpyrrolidin-1-yl)-2-(trifluorométhyl)pyridin-3-yl)picolinamide |
| (S)-6-(1-éthyl-1H-pyrazol-4-yl)-N-(5-(3-méthyl-4-(oxétan-3-yl)pipérazin-1-yl)-2-(trifluorométhyl)pyridin-3-yl)picolinamide |
| 6-(1-cyclobutyl-1H-pyrazol-4-yl)-N-(5-(3-méthoxyazétidin-1-yl)-2-(trifluorométhyl)pyridin-3-yl)picolinamide |
| (R)-6-(1-cyclobutyl-1H-pyrazol-4-yl)-N-(5-(3-(2-hydroxypropan-2-yl)pyrrolidin-1-yl)-2-(trifluorométhyl)pyridin-3-yl)picolinamide |
| (S)-6-(1-cyclobutyl-1H-pyrazol-4-yl)-N-(5-(3-méthyl-4-(oxétan-3-yl)pipérazin-1-yl)-2-(trifluorométhyl)pyridin-3-yl)picolinamide |
| 6-(1-méthyl-1H-pyrazol-4-yl)-N-(2-méthyl-5-(3-(trifluorométhyl)pyrrolidin-1-yl)pyridin-3-yl)picolinamide |
| N-(5-(3,3-difluoropyrrolidin-1-yl)-2-méthylpyridin-3-yl)-6-(1-methyl-1H-pyrazol-4-yl)picolinamide |
| N-(5-((2S,6R)-2,6-diméthylmorpholino)-2-méthylpyridin-3-yl)-6-(1-méthyl-1H-pyrazol-4-yl)picolinamide |
| N-(5-(3-(fluorométhyl)pyrrolidin-1-yl)-2-méthylpyridin-3-yl)-6-(1-méthyl-1H-pyrazol-4-yl)picolinamide |
| (S)-N-(2-méthyl-5-(4-méthyl-2-oxopyrrolidin-1-yl)pyridin-3-yl)-6-(1-(2,2,2-trifluoroéthyl)-1H-pyrazol-4-yl)picolinamide |
| (S)-6-(1-méthyl-1H-pyrazol-4-yl)-N-(2-méthyl-5-(3-méthyl-4-(oxétan-3-yl)pipérazin-1-yl)pyridin-3-yl)picolinamide |
| (R)-N-(5-(3-(2-hydroxypropan-2-yl)pyrrolidin-1-yl)-2-(trifluorométhyl)pyridin-3-yl)-6-(1H-pyrazol-4-yl)picolinamide |
| N-(5-(4-(2-hydroxypropan-2-yl)-2-azabicyclo[2.1.1]hexan-2-yl)-2-(trifluoromethyl)pyridin-3-yl)-6-(1H-pyrazol-4-yl)picolinamide |
| N-(5-((3S,4s,5R)-4-hydroxy-3,4,5-triméthylpipéridin-1-yl)-2-(trifluorométhyl)pyridin-3-yl)-6-(1H-pyrazol-4-yl)picolinamide |
| (S)-N-(5-(3-méthyl-4-(oxétan-3-yl)pipérazin-1-yl)-2-(trifluorométhyl)pyridin-3-yl)-6-(1H-pyrazol-4-yl)picolinamide |
| (S)-N-(5-(4-méthyl-2-oxopyrrolidin-1-yl)-2-(trifluorométhyl)pyridin-3-yl)-6-(1H-pyrazol-4-yl)picolinamide |
| (R)-N-(2-cyclopropyl-5-(3-(2-hydroxypropan-2-yl)pyrrolidin-1-yl)pyridin-3-yl)-6-(1-méthyl-1H-pyrazol-4-yl)picolinamide |
| N-(2-cyclopropyl-5-(4-(2-hydroxypropan-2-yl)-2-azabicyclo[2.1.1]hexan-2-yl)pyridin-3-yl)-6-(1-méthyl-1H-pyrazol-4-yl)picolinamide |
| N-(2-cyclopropyl-5-(2-oxopyrrolidin-1-yl)pyridin-3-yl)-6-(1-methyl-1H-pyrazol-4-yl)picolinamide |
| N-(2-cyclopropyl-5-(2-oxopyrrolidin-1-yl)pyridin-3-yl)-6-(1-(2,2,2-trifluoroethyl)-1H-pyrazol-4-yl)picolinamide |
| (S)-N-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide |
| (R)-N-(2-cyclopropyl-5-(4-methyl-2-oxopyrrolidin-1-yl)pyridin-3-yl)-6-(1-(2,2,2-trifluoroethyl)-1H-pyrazol-4-yl)picolinamide |
| N-(2-cyclopropyl-5-(2-oxopiperidin-1-yl)pyridin-3-yl)-6-(1-(2,2,2-trifluoroethyl)-1H-pyrazol-4-yl)picolinamide |
| N-(2-cyclopropyl-5-(3,3-diméthyl-2-oxopipéridin-l-yl)pyridin-3-yl)-6-(1-(2,2,2-trifluoroethyl)-1H-pyrazol-4-yl)picolinamide |
| N-(2-cyclopropyl-5-(4,4-dimethyl-2-oxopyrrolidin-1-yl)pyridin-3-yl)-6-(1-(2,2,2-trifluoroethyl)-1H-pyrazol-4-yl)picolinamide |
| N-(2-cyclopropyl-5-(4-méthyl-2-oxopipérazin-1-yl)pyridin-3-yl)-6-(1-(2,2,2-trifluoroethyl)-1H-pyrazol-4-yl)picolinamide |
| N-(2-cyclopropyl-5-(3,3,4-trimethyl-2-oxopiperazin-1-yl)pyridin-3-yl)-6-(1-(2,2,2-trifluoroethyl)-1H-pyrazol-4-yl)picolinamide |
| N-(2-cyclopropyl-5-(3,3-diméthyl-2-oxopyrrolidin-1-yl)pyridin-3-yl)-6-(1-(2,2,2-trifluoroethyl)-1H-pyrazol-4-yl)picolinamide |
| N-(2-cyclopropyl-5-(6-oxo-5-azaspiro[2.4]heptan-5-yl)pyridin-3-yl)-6-(1-(2,2,2-trifluoroethyl)-1H-pyrazol-4-yl)picolinamide |
| (R)-N-(2-cyclopropyl-5-(3-methyl-4-(oxetan-3-yl)piperazin-1-yl)pyridin-3-yl)-6-(1-(2,2,2-trifluoroéthyl)-1H-pyrazol-4-yl)picolinamide |
| (S)-N-(2-cyclopropyl-5-(3-methyl-4-(oxetan-3-yl)piperazin-1-yl)pyridin-3-yl)-6-(1-(2,2,2-trifluoroéthyl)-1H-pyrazol-4-yl)picolinamide |
| N-(2-cyclopropyl-5-((3S,5R)-3,5-dimethyl-4-(oxetan-3-yl)piperazin-1-yl)pyridin-3-yl)-6-(1-(2,2,2-trifluoroéthyl)-1H-pyrazol-4-yl)picolinamide |
| (S)-N-(2-cyclopropyl-5-(3-methoxypyrrolidin-1-yl)pyridin-3-yl)-6-(1-(2,2,2-trifluoroethyl)-1H-pyrazol-4-yl)picolinamide |
| N-(2-cyclopropyl-5-((1S,4R)-3-oxo-2-azabicyclo[2.2.1]heptan-2-yl)pyridin-3-yl)-6-(1-(2,2,2-trifluoroethyl)-1H-pyrazol-4-yl)picolinamide |
| N-(2-cyclopropyl-5-(8-methyl-5-oxa-2,8-diazaspiro[3,5]nonan-2-yl)pyridin-3-yl)-6-(1-(2,2,2-trifluoroethyl)-1H-pyrazol-4-yl)picolinamide |
| N-(2-cyclopropyl-5-(5-oxo-6-azaspiro[3.4]octan-6-yl)pyridin-3-yl)-6-(1-(2,2,2-trifluoroethyl)-1H-pyrazol-4-yl)picolinamide |
| N-(2-cyclopropyl-5-(1-oxo-2-azaspiro[4.4]nonan-2-yl)pyridin-3-yl)-6-(1-(2,2,2-trifluoroethyl)-1H-pyrazol-4-yl)picolinamide |
| (S)-N-(2-cyclopropyl-5-(3-méthyl-4-(oxétan-3-yl)piperazin-1-yl)pyridin-3-yl)-6-(1-methyl-1H-pyrazol-4-yl)picolinamide |
| N-(2-cyclopropyl-5-(2-oxa-7-azaspiro[3,5]nonan-7-yl)pyridin-3-yl)-6-(1-méthyl-1H-pyrazol-4-yl)picolinamide |
| N-(5-((3R,4S)-3-(2-hydroxypropan-2-yl)-4-méthoxypyrrolidin-1-yl)-2-(trifluorométhyl)pyridin-3-yl)-6-(1-(2,2,2-trifluoroéthyl)-1H-pyrazol-4-yl)picolinamide |
| (S)-N-(5-(3-(2-hydroxypropan-2-yl)-3-méthoxypyrrolidin-1-yl)-2-(trifluorométhyl)pyridin-3-yl)-6-(1-(2,2,2-trifluoroéthyl)-1H-pyrazol-4-yl)picolinamide |
| (R)-N-(5-(3-(2-hydroxypropan-2-yl)-3-méthoxypyrrolidin-1-yl)-2-(trifluorométhyl)pyridin-3-yl)-6-(1-(2,2,2-trifluoroethyl)-1H-pyrazol-4-yl)picolinamide |
| (S)-6-(1-(cyclopropylmethyl)-1H-pyrazol-4-yl)-N-(5-(3-(2-hydroxypropan-2-yl)-3-méthoxypyrrolidin-1-yl)-2-(trifluorométhyl)pyridin-3-yl)picolinamide |
| 6-(1-(cyclopropylmethyl)-1H-pyrazol-4-yl)-N-(5-((3R,4S)-3-(2-hydroxypropan-2-yl)-4-méthoxypyrrolidin-1-yl)-2-(trifluorométhyl)pyridin-3-yl)picolinamide |
| (R)-6-(1-éthyl-1H-pyrazol-4-yl)-N-(5-(3-(2-hydroxypropan-2-yl)-3-methoxypyrrolidin-1-yl)-2-(trifluorométhyl)pyridin-3-yl)picolinamide |
| (S)-6-(1-éthyl-1H-pyrazol-4-yl)-N-(5-(3-(2-hydroxypropan-2-yl)-3-methoxypyrrolidin-1-yl)-2-(trifluorométhyl)pyridin-3-yl)picolinamide |
| 6-(1-éthyl-1H-pyrazol-4-yl)-N-(5-((3S,4R)-3-(2-hydroxypropan-2-yl)-4-methoxypyrrolidin-1-yl)-2-(trifluorométhyl)pyridin-3-yl)picolinamide |
| 6-(1-éthyl-1H-pyrazol-4-yl)-N-(5-((3R,4S8)-3-(2-hydroxypropan-2-yl)-4-methoxypyrrolidin-1-yl)-2-(trifluorométhyl)pyridin-3-yl)picolinamide |
| (R) ou (S) N-(5-((R)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4-yl)picolinamide |
| (R) ou (S) N-(5-((R)-3-(2-hydroxypropan-2-yl)pyrrolidin-1-yl)-2-(trifluoromethyl)pyridin-3-yl)-6-(1-(1,1,1-trifluoropropan-2-yl)-1H-pyrazol-4-yl)picolinamide |
| N-(2-((2R,6S)-2,6-diméthylmorpholino)-5-(trifluorométhyl)pyridin-4-yl)-6-(1-méthyl-1H-pyrazol-4-yl)picolinamide |
| (R)-N-(2-(3-(2-hydroxypropan-2-yl)pyrrolidin-1-yl)-5-(trifluorométhyl)pyridin-4-yl)-6-(1-méthyl-1H-pyrazol-4-yl)picolinamide |
| N-(2-(4-(2-hydroxypropan-2-yl)-2-azabicyclo[2.1.1]hexan-2-yl)-5-(trifluoromethyl)pyridin-4-yl)-6-(1-methyl-1H-pyrazol-4-yl)picolinamide |
| Méso 6-(1-methyl-1H-pyrazol-4-yl)-N-(2-(tetrahydro-1H-furo[3,4-c]pyrrol-5(3H)-yl)-5-(trifluorométhyl)pyridin-4-yl)picolinamide |
| N-(2-((2R,4R)-4-hydroxy-2-methylpyrrolidin-1-yl)-5-(trifluorométhyl)pyridin-4-yl)-6-(1-méthyl-1H-pyrazol-4-yl)picolinamide |
| N-(2-((2S,4R)-4-hydroxy-2-methylpyrrolidin-1-yl)-5-(trifluorométhyl)pyridin-4-yl)-6-(1-méthyl-1H-pyrazol-4-yl)picolinamide |
| N-(2-((2R,3S)-3-hydroxy-2-methylazetidin-1-yl)-5-(trifluorométhyl)pyridin-4-yl)-6-(1-méthyl-1H-pyrazol-4-yl)picolinamide |
| N-(2-(3-hydroxyazetidin-1-yl)-5-(trifluorométhyl)pyridin-4-yl)-6-(1-méthyl-1H-pyrazol-4-yl)picolinamide |
| N-(2-((3R,5S)-4-hydroxy-2-methylpyrrolidin-1-yl)-5-(trifluorométhyl)pyridin-4-yl)-6-(1-méthyl-1H-pyrazol-4-yl)picolinamide |
| (R, R) ou (S, S) N-(2-(3-hydroxy-2-méthylpyrrolidin-1-yl)-5-(trifluorométhyl)pyridin-4-yl)-6-(1-méthyl-1H-pyrazol-4-yl)picolinamide |
| (R, R) ou (S, S) N-(2-(3-hydroxy-2-méthylpyrrolidin-1-yl)-5-(trifluorométhyl)pyridin-4-yl)-6-(1-méthyl-1H-pyrazol-4-yl)picolinamide |
| N-(2-(3-methoxyazetidin-1-yl)-5-(trifluorométhyl)pyridin-4-yl)-6-(1-méthyl-1H-pyrazol-4-yl)picolinamide |
| N-(2-(3-(2-hydroxypropan-2-yl)azetidin-1-yl)-5-(trifluorométhyl)pyridin-4-yl)-6-(1-méthyl-1H-pyrazol-4-yl)picolinamide |
| N-(2-(3-(difluoromethyl)pyrrolidin-1-yl)-5-(trifluoromethyl)pyridin-4-yl)-6-(1-methyl-1H-pyrazol-4-yl)picolinamide |
| (R)-N-(2-(3-(2-hydroxypropan-2-yl)pyrrolidin-1-yl)-5-(trifluorométhyl)pyridin-4-yl)-6-(1-(2,2,2-trifluoroethyl)-1H-pyrazol-4-yl)picolinamide |
| (R) ou (S) N-(2-(3-hydroxy-3-méthylpyrrolidin-1-yl)-5-(trifluoromethyl)pyridin-4-yl)-6-(1-methyl-1H-pyrazol-4-yl)picolinamide |
| (R) ou (S) N-(2-(3-hydroxy-3-méthylpyrrolidin-1-yl)-5-(trifluoromethyl)pyridin-4-yl)-6-(1-methyl-1H-pyrazol-4-yl)picolinamide |
| (S)-6-(1-methyl-1H-pyrazol-4-yl)-N-(2-(3-methyl-4-(oxétan-3-yl)pipérazin-1-yl)-5-(trifluorométhyl)pyridin-4-yl)picolinamide |
| 6-(1-méthyl-1H-pyrazol-4-yl)-N-(2-(4-(oxétan-3-yl)pipéridin-1-yl)-5-(trifluorométhyl)pyridin-4-yl)picolinamide |
| (R)-N-(2-(3-(2-hydroxypropan-2-yl)pyrrolidin-1-yl)-5-méthylpyridin-4-yl)-6-(1-méthyl-1H-pyrazol-4-yl)picolinamide |
| (R)-N-(2-(3-(2-hydroxypropan-2-yl)pyrrolidin-1-yl)-5-(trifluoromethyl)pyridin-4-yl)-6-(1H-pyrazol-4-yl)picolinamide |
| N-(2-((2R,3S)-3-hydroxy-2-methylazetidin-1-yl)-5-(trifluoromethyl)pyridin-4-yl)-6-(1H-pyrazol-4-yl)picolinamide |
| N-(2-((2R,4R)-4-hydroxy-2-methylpyrrolidin-1-yl)-5-(trifluoromethyl)pyridin-4-yl)-6-(1H-pyrazol-4-yl)picolinamide |
| N-(2-((2S,4R)-4-hydroxy-2-methylpyrrolidin-1-yl)-5-(trifluoromethyl)pyridin-4-yl)-6-(1H-pyrazol-4-yl)picolinamide |
| 6-(1-méthyl-1H-pyrazol-4-yl)-N-(5-méthyl-2-morpholinopyridin-4-yl)picolinamide |
| 6-(1-(cyclopropylmethyl)-1H-pyrazol-4-yl)-N-(5-methyl-2-morpholinopyridin-4-yl)picolinamide |
| 6-(1-(cyclopropylmethyl)-1H-pyrazol-4-yl)-N-(2-((2S,6R)-2,6-diméthylmorpholino)-5-méthylpyridin-4-yl)picolinamide |
| N-(2-((2S,6R)-2,6-diméthylmorpholino)-5-méthylpyridin-4-yl)-6-(1-(2,2,2-trifluoroéthyl)-1H-pyrazol-4-yl)picolinamide |
| N-(2-((2S,6R)-2,6-diméthylmorpholino)-5-(trifluoromethyl)pyridin-4-yl)-6-(1-ethyl-1H-pyrazol-4-yl)picolinamide |
| N-(2-((2S,6R)-2,6-diméthylmorpholino)-5-méthylpyridin-4-yl)-6-(1-((1-(trifluorométhyl)cyclopropyl)méthyl)-1H-pyrazol-4-yl)picolinamide |
| N-(2-((2S,6R)-2,6-diméthylmorpholino)-5-méthylpyridin-4-yl)-6-(1-éthyl-1H-pyrazol-4-yl)picolinamide |
| 6-(1-ethyl-1H-pyrazol-4-yl)-N-(5-methyl-2-morpholinopyridin-4-yl)picolinamide |
| 6-(1-cyclobutyl-1H-pyrazol-4-yl)-N-(5-méthyl-2-morpholinopyridin-4-yl)picolinamide |
| 6-(1-cyclobutyl-1H-pyrazol-4-yl)-N-(2-((2S,6R)-2,6-diméthylmorpholino)-5-méthylpyridin-4-yl)picolinamide |
| N-(2-((2S,6R)-2,6-diméthylmorpholino)-5-méthylpyridin-4-yl)-6-(1H-pyrazol-4-yl)picolinamide |
| N-(5-methyl-2-morpholinopyridin-4-yl)-6-(1H-pyrazol-4-yl)picolinamide |
| (R)-N-(6-(3-(2-hydroxypropan-2-yl)pyrrolidin-1-yl)-2-méthylpyrimidin-4-yl)-6-(1-méthyl-1H-pyrazol-4-yl)picolinamide |
| (R)-N-(6-(3-(2-hydroxypropan-2-yl)pyrrolidin-1-yl)pyrimidin-4-yl)-6-(1-méthyl-1H-pyrazol-4-yl)picolinamide |
| N-(6-((2S,6R)-2,6-diméthylmorpholino)pyrimidin-4-yl)-6-(1-methyl-1H-pyrazol-4-yl)picolinamide |
| N-(6-((3S,4r,5R)-4-hydroxy-3,5-dimethylpiperidin-1-yl)pyrimidin-4-yl)-6-(1-methyl-1H-pyrazol-4-yl)picolinamide |
| N-(6-((3S,4r,5R)-4-hydroxy-3,4,5-trimethylpiperidin-1-yl)pyrimidin-4-yl)-6-(1-methyl-1H-pyrazol-4-yl)picolinamide |
| N-(6-(4-(2-hydroxypropan-2-yl)-2-azabicyclo[2.1.1]hexan-2-yl)pyrimidin-4-yl)-6-(1-méthyl-1H-pyrazol-4-yl)picolinamide |
| N-(6-(2-azaspiro[4.4]nonan-2-yl)pyrimidin-4-yl)-6-(1-methyl-1H-pyrazol-4-yl)picolinamide |
| (R) ou (S) N-(6-(1,1-difluoro-5-azaspiro[2.4]heptan-5-yl)pyrimidin-4-yl)-6-(1-methyl-1H-pyrazol-4-yl)picolinamide |
| (R) ou (S) N-(6-(1,1-difluoro-5-azaspiro[2.4]heptan-5-yl)pyrimidin-4-yl)-6-(1-methyl-1H-pyrazol-4-yl)picolinamide |
| (R) ou (S) N-(6-(1,1-difluoro-5-azaspiro[2.4]heptan-5-yl)-2-méthylpyrimidin-4-yl)-6-(1-méthyl-1H-pyrazol-4-yl)picolinamide |
| N-(6-((3S,4r,5R)-4-hydroxy-3,4,5-triméthylpipéridin-1-yl)-2-méthylpyrimidin-4-yl)-6-(1-méthyl-1H-pyrazol-4-yl)picolinamide |
| (R)-N-(6-(3-(2-hydroxypropan-2-yl)pyrrolidin-1-yl)pyrimidin-4-yl)-6-(1-(2,2,2-trifluoroethyl)-1H-pyrazol-4-yl)picolinamide |
| N-(6-(4-(oxetan-3-yl)piperazin-1-yl)pyrimidin-4-yl)-6-(1-(2,2,2-trifluoroethyl)-1H-pyrazol-4-yl)picolinamide |
| N-(6-((3S,4r,5R)-4-hydroxy-3,4,5-triméthylpipéridin-1-yl)pyrimidin-4-yl)-6-(1-(2,2,2-trifluoroethyl)-1H-pyrazol-4-yl)picolinamide |
| N-(6-(4-(3-méthyloxétan-3-yl)pipérazin-1-yl)pyrimidin-4-yl)-6-(1-(2,2,2-trifluoroéthyl)-1H-pyrazol-4-yl)picolinamide |
| N-(6-(3-(2-cyanopropan-2-yl)pyrrolidin-1-yl)-2-méthylpyrimidin-4-yl)-6-(1-(cyclopropylméthyl)-1H-pyrazol-4-yl)picolinamide |
| (R)-N-(2-cyclopropyl-6-(3-(2-hydroxypropan-2-yl)pyrrolidin-1-yl)pyrimidin-4-yl)-6-(1-methyl-1H-pyrazol-4-yl)picolinamide |
| (R)-N-(2-cyclopropyl-6-(3-(2-hydroxypropan-2-yl)pyrrolidin-1-yl)pyrimidin-4-yl)-6-(1-(2,2,2-trifluoroéthyl)-1H-pyrazol-4-yl)picolinamide |
| N-(2-cyclopropyl-6-((3S,4s,5R)-4-hydroxy-3,4,5-triméthylpipéridin-1-yl)pyrimidin-4-yl)-6-(1-(2,2,2-trifluoroéthyl)-1H-pyrazol-4-yl)picolinamide |
| N-(2-cyclopropyl-6-(3-hydroxy-3-methylpyrrolidin-1-yl)pyrimidin-4-yl)-6-(1-(2,2,2-trifluoroethyl)-1H-pyrazol-4-yl)picolinamide |
| N-(2-cyclopropyl-6-(3-fluoroazetidin-1-yl)pyrimidin-4-yl)-6-(1-(cyclopropylmethyl)-1H-pyrazol-4-yl)picolinamide |
| N-(2-cyclopropyl-6-(3,3-difluoroazetidin-1-yl)pyrimidin-4-yl)-6-(1-(cyclopropylméthyl)-1H-pyrazol-4-yl)picolinamide |
| N-(2-cyclopropyl-6-((3S,4s,5R)-4-hydroxy-3,4,5-triméthylpipéridin-1-yl)pyrimidin-4-yl)-6-(1-(cyclopropylmethyl)-1H-pyrazol-4-yl)picolinamide |
| (R)-N-(6-(3-(2-hydroxypropan-2-yl)pyrrolidin-1-yl)-2-méthylpyrimidin-4-yl)-6-(1-(2,2,2-trifluoroéthyl)-1H-pyrazol-4-yl)picolinamide |
| (R)-6-(1-(cyclopropylméthyl)-1H-pyrazol-4-yl)-N-(6-(3-(2-hydroxypropan-2-yl)pyrrolidin-1-yl)-2-méthylpyrimidin-4-yl)picolinamide |
| N-(6-((3S,4s,5R)-4-hydroxy-3,4,5-triméthylpipéridin-1-yl)-2-méthylpyrimidin-4-yl)-6-(1-(2,2,2-trifluoroethyl)-1H-pyrazol-4-yl)picolinamide |
| 6-(1-((1-cyanocyclopropyl)methyl)-1H-pyrazol-4-yl)-N-(6-((3S,4s,5R)-4-hydroxy-3,4,5-triméthylpipéridin-1-yl)-2-méthylpyrimidin-4-yl)picolinamide |
| 6-(1-((1-cyanocyclopropyl)methyl)-1H-pyrazol-4-yl)-N-(6-(1,1-difluoro-5-azaspiro[2.4]heptan-5-yl)-2-méthylpyrimidin-4-yl)picolinamide |
| (R)-N-(6-(3-(2-hydroxypropan-2-yl)pyrrolidin-1-yl)-2-méthylpyrimidin-4-yl)-6-(1-((1-(trifluorométhyl)cyclopropyl)méthyl)-1H-pyrazol-4-yl)picolinamide |
| (R)-6-(1-éthyl-1H-pyrazol-4-yl)-N-(6-(3-(2-hydroxypropan-2-yl)pyrrolidin-1-yl)-2-méthylpyrimidin-4-yl)picolinamide |
| 6-(1-ethyl-1H-pyrazol-4-yl)-N-(6-((3S,4s,5R)-4-hydroxy-3,4,5-triméthylpipéridin-1-yl)-2-méthylpyrimidin-4-yl)picolinamide |
| (S)-6-(1-((1-cyanocyclopropyl)methyl)-1H-pyrazol-4-yl)-N-(6-(3-méthyl-4-(oxétan-3-yl)pipérazin-1-yl)pyrimidin-4-yl)picolinamide |
| N-(6-((33,4s,5R)-4-hydroxy-3,4,5-triméthylpipéridin-1-yl)-2-methylpyrimidin-4-yl)-6-(1H-pyrazol-4-yl)picolinamide |
| (R)-N-(6-(3-(2-fluoropropan-2-yl)pyrrolidin-1-yl)-2-méthylpyrimidin-4-yl)-6-(1-méthyl-1H-pyrazol-4-yl)picolinamide |
| (R)-N-(6-(3-(2-fluoropropan-2-yl)pyrrolidin-1-yl)-2-méthylpyrimidin-4-yl)-6-(1-(2,2,2-trifluoroéthyl)-1H-pyrazol-4-yl)picolinamide |
| (R)-N-(5-(3-(2-hydroxypropan-2-yl)pyrrolidin-1-yl)pyridazin-3-yl)-6-(1-methyl-1H-pyrazol-4-yl)picolinamide |
| N-[6-[(3R)-3-(1-hydroxy-1-méthyl-éthyl)pyrrolidin-1-yl]-3-méthyl-pyridazin-4-yl]-6-(1-méthylpyrazol-4-yl)pyridine-2-carboxamide |
| N-[3-cyclopropyl-6-[(3R)-3-(1-hydroxy-1-methylethyl)pyrrolidin-1-yl]pyridazin-4-yl]-6-(1-méthylpyrazol-4-yl)pyridine-2-carboxamide |
| (R)-N-(4-(3-(2-hydroxypropan-2-yl)pyrrolidin-1-yl)-6-méthylpyridin-2-yl)-6-(1-méthyl-1H-pyrazol-4-yl)picolinamide, et |
| (R)-N-(4-(3-(2-hydroxypropan-2-yl)pyrrolidin-1-yl)pyridin-2-yl)-6-(1-méthyl-1H-pyrazol-4-yl)picolinamide, et |
| (6-(1-(cyclopropylmethyl)-1H-pyrazol-4-yl)-N-(4-((3S,4r,5R)-4-hydroxy-3,4,5-triméthylpipéridin-1-yl)-6-méthylpyridin-2-yl)picolinamide |
| (R)-6-(1-(cyclopropylméthyl)-1H-pyrazol-4-yl)-N-(4-(3-(2-hydroxypropan-2-yl)pyrrolidin-1-yl)-6-méthylpyridin-2-yl)picolinamide, et |
| N-(2-((2S,6R)-2,6-diméthylmorpholino)-5-méthylpyridin-4-yl)-6-(1-méthyl-1H-pyrazol-4-yl)picolinamide. |

17. Composition pharmaceutique comprenant un composé selon l'une quelconque des revendications 1 à 16 ou un sel pharmaceutiquement acceptable correspondant, et un support pharmaceutiquement acceptable.

18. Composé selon l'une quelconque des Revendications 1 à 16 ou sel pharmaceutiquement acceptable correspondant, pour une utilisation dans le traitement de la maladie de Parkinson.

19. Composé selon l'une quelconque des revendications 1 à 16 ou sel pharmaceutiquement acceptable correspondant, pour une utilisation en thérapie.

20. Composé selon l'une quelconque des revendications 1 à 16 ou sel pharmaceutiquement acceptable correspondant, pour une utilisation dans le traitement d'une indication choisie parmi :
symptômes moteurs anormaux associés à la maladie de Parkinson, symptômes non moteurs associés à la maladie de Parkinson, démence à corps de Lewy, dyskinésies induites par la L-Dopa,
maladie d'Alzheimer, troubles cognitifs légers, transition des troubles cognitifs légers à la maladie d'Alzheimer, tauopathies **caractérisées par** une hyperphosphorylation de la protéine tau telles que la maladie à grains argyrophiles, la maladie de Pick, la dégénérescence corticobasale, la paralysie supranucléaire progressive, la démence frontotemporale héréditaire et la maladie de Parkinson liée au chromosome 17,
neuroinflammation associée aux réponses inflammatoires microgliales associées à la sclérose en plaques, à la démence induite par le VIH, à la SLA, à l'accident vasculaire cérébral ischémique, aux traumatismes crâniens et aux lésions de la moelle épinière,
lymphomes, leucémies, sclérose en plaques, polyarthrite rhumatoïde, lupus érythémateux systémique, anémie hémolytique auto-immune, aplasie érythrocytaire pure, purpura thrombopénique idiopathique (PTI), syndrome d'Evans, vascularite, dermatose bulleuse, diabète de type 1, syndrome de Sjögren, maladie de Delvic, myopathies inflammatoires et spondylarthrite ankylosante, cancer du rein, cancer du sein, cancer du poumon, cancer de la prostate et leucémie myéloïde aiguë (LMA) chez les sujets exprimant la mutation LRRK2 G2019S,
carcinomes papillaires rénaux et thyroïdiens chez un sujet chez lequel LRRK2 est amplifié ou surexprimé, maladie de Crohn et lèpre.

21. Combinaison comprenant un composé selon l'une quelconque des revendications 1 à 16, ou un sel pharmaceutiquement acceptable correspondant et un, deux autres composés actifs ou plus.
